# EUROPEAN PATENT APPLICATION

(11) **EP 4 086 252 A1**
(43) Date of publication of application: **09.11.2022**
(21) Application number: 20908860.8
(22) Date of filing: 16.12.2020
(51) Int. Cl.: C07D 471/04, A61K 31/437, A61P 35/00

(54) **HETEROCYCLIC SULFOXIMINE COMPOUND AND INTERMEDIATE THEREOF, PREPARATION METHOD THEREFOR, AND APPLICATION THEREOF**

(30) Priority: 31.12.2019 CN 201911419093
(71) Applicant: NANGENE BIOMEDICAL CO., LTD., Jiangsu 210038 (CN)
(72) Inventor: LI, Anhu, Nanjing, Jiangsu 210038 (CN)
(74) Representative: Raffay & Fleck
(86) International application number: PCT/CN2020/136676
(87) International publication number: WO 2021/135938

(57) **Abstract**

The present invention relates to a heterocyclic sulfoximine compound represented by formula (I), and racemates, enantiomers, pharmaceutically acceptable salts or solvates thereof. Also disclosed are an intermediate compound for synthesizing the compound, a preparation method therefor, and a pharmaceutical composition comprising the compound and use thereof. The compound is a RET, a RET mutant, or a RET fusion protein inhibitor, capable of treating diseases caused by abnormal activity of a RET, a RET mutant, or a RET fusion protein inhibitor, for example, tumors.

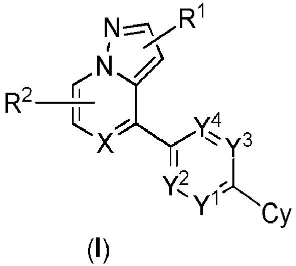

## Description

### Cross-reference to related application

The present application claims priority to Chinese Patent Application No.201911419093.0, entitled "Heterocyclic sulfoximine compound and intermediate thereof, preparation method therefor, and application thereof", and filed on December 31, 2019, the entire disclosure of which is incorporated herein by reference in its entirety.

### Technical Field

The present invention relates to the field of pharmaceutical chemistry, specifically, to a heterocyclic sulfoximine compound, or a racemate, an enantiomer, a pharmaceutically acceptable salt or a solvate thereof, an intermediate compound for synthesizing the compound, a preparation method therefor, and a pharmaceutical composition comprising the compound and use thereof.

### Background Art

RET is a tyrosine receptor kinase that belongs to the glial cell-derived neurotrophic factor (GDNF) family and is an essential kinase for the development of the nervous system and several other tissues. Its loss of function mutations causes Hirschsprung disease, and its gain of function mutations is associated with a variety of human tumors.

RET kinases are prone to mutation and formation of fusion proteins with other partners. RET point mutations mainly occur in multiple endocrine tumors (MEN2A and MEN2B) and medullary thyroid carcinoma (MTC), and RET fusion proteins occur mainly in papillary thyroid cancer and non-small cell lung cancer. Currently, in papillary thyroid cancer, there are more than a dozen partners that form fusion proteins with RET, mainly including RET-CCDC6 and RET-NCOA4. The fusion protein in non-small cell lung cancer is mainly RET-KIF5B.

In a study of 4,871 patient samples including multiple tumors (S. Kato et al. Clin. Cancer Res. 2017, 23, 1988-1997), it was found that the total mutation rate of RET was about 1.8%, and the main mutations comprises three types of mutation (38.6%), fusion (30.7%) and amplification (25%). The major mutant is RET (M918T), which occurs in medullary thyroid carcinoma (MTC). Among them, the RET mutation in sporadic MTC is >60%, and the RET mutation in hereditary MTC is >90%. Incidence of RET fusion proteins (RET-KIF5B, RET-CCDC6, and RET-NCOA4) is as follows: 2% for non-small cell lung cancer; and 10-20% for papillary thyroid cancer and other thyroid cancers. Incidence of RET amplification is as follows: 9.3% for fallopian tube adenocarcinoma; 5.3% for uterine carcinosarcoma; and 5% forduodenal adenocarcinoma.

In addition to the RET mutants and fusion proteins mentioned above, there are many such mutants and fusion proteins including, but not limited to, RET(A883F), RET(E762Q), RET(G691S), RET(L790F), RET(R749T), RET(R813Q), RET(R912P), RET(S891A), RET(S904A), RET(S904F), RET(V804M), RET(V778I), RET(V804E), RET(V804L), RET(G810R)-KIF5B, RET(V804L)-KIF5B, RET(V804M)-KIF5B, RET(Y791F), RET(Y806H), RET-BCR, RET-KIF5B(Kex15Rex14), RET-PRKAR1A(PTC2) and the like.

Since RET mutants and fusion proteins play important roles in a variety of human tumors, the development of inhibitors against these biological targets is of clinical interest. In fact, in recent years, the FDA of US has approved several multikinase inhibitors containing RET targets for the treatment of tumors (R. Roskoski, Jr. and A. Sadeghi-Nejad, Pharmacol. Res. 2018, 128, 1-17), for example, Cabozantinib and Vandetanib for the treatment of medullary thyroid carcinoma (MTC); and Lenvatinib and Sorafenib for the treatment of differentiated thyroid cancer. Although multikinase inhibitors containing RET targets have been approved for clinical use, highly selective RET inhibitors have not been approved for clinical use, and selective RET inhibitors such as BLU-667 and LOXO-292 are currently undergoing clinical trials.

S. W. Andrews et al reported a class of substituted pyrazolo[1,5-a]pyridine compounds as RET inhibitors (WO2017/011776, WO2018/071447, WO2018/071454).

S. W. Andrews et al reported a class of substituted pyrazolo[1,5-a]pyrazine compounds as RET inhibitors (WO2018/136661).

J. D. Brubaker and colleagues reported a class of cyclohexyl-substituted pyrimidine compounds as RET inhibitors (WO2017/079140).

H. Inagaki and colleagues reported a class of substituted pyridine compounds as RET inhibitors (WO2018/060714).

M. P. Demartino et al reported a class of substituted pyridyl pyridone compounds as RET inhibitors (WO2017/145050).

Despite good progress has been made in the development of RET, RET mutants or RET fusion protein inhibitors, there are currently no highly selective RET, RET mutant and/or RET fusion protein inhibitors approved for clinical treatment of tumors. One of the objectives of the present invention is to disclose a class of highly selective inhibitors of RET, RET mutants and/or RET fusion proteins with potential clinical application value.

### Summary of the Invention

One purpose of the present invention is to provide a heterocyclic sulfoximine compound, or a racemate, an enantiomer, a pharmaceutically acceptable salt or a solvate thereof with an inhibitory activity for RET, RET mutant or RET fusion protein, and preparation method thereof.

Another purpose of the present invention is to provide a pharmaceutical composition containing the above-mentioned heterocyclic sulfoximine compound, or a racemate, an enantiomer, a pharmaceutically acceptable salt or a solvate thereof, and use of the above-mentioned heterocyclic sulfoximine compound, or a racemate, an enantiomer, a pharmaceutically acceptable salt or a solvate thereof, and pharmaceutical composition in the treatment of diseases caused by abnormal activity of RET, RET mutant, or RET fusion proteins.

Another purpose of the present invention is to provide an intermediate compound for synthesizing the above-mentioned heterocyclic sulfoximine compound, or a racemate, an enantiomer, a pharmaceutically acceptable salt or a solvate thereof, and preparation method thereof.

To achieve the above purposes, the present invention adopts the following technical solutions:
A heterocyclic sulfoximine compound, or a racemate, an enantiomer, a pharmaceutically acceptable salt or a solvate thereof, the molecular structure of the compound is represented by formula (I): 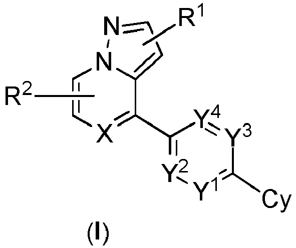 in the formula:
Cy is (Ia) or (Ib): 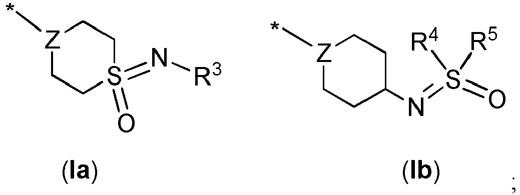 ^{∗} represents an attachment point between Cy and rest of the molecular structure represented by formula (I);
X is N or C-R;
Y¹, Y², Y³ and Y⁴ are the same or different, and each independently represent N or C-R';
Z is N or C-R";
R, R', or R" are the same or different, and each independently selected from hydrogen, deuterium, halogen, CN, OR^{a}, NR^{b}R^{c}, S(=O)_{w}R^{d}, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R, R', or R" can be optionally substituted by the same or different deuterium, halogen, CN, NO₂, OH, OCH₃, OCF₃ or CF₃;
R¹ and R² are the same or different, and each independently selected from one or two of the same or different hydrogen, deuterium, halogen, CN, NO₂, OR^{a}, NR^{b}R^{c}, S(=O)_{w}R^{d}, CO₂R^{e}, CONR^{b}R^{c}, C₁₋₁₂ alkyl, C₃₋₁₂cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl, wherein one or more hydrogens in the C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl can be optionally substituted by the same or different G¹;
R³ is selected from hydrogen, deuterium, S(=O)_{w}R^{d}, S(=O)_{w}NR^{f}R^{g}, C(=O)R^{h}, C(=O)NRⁱR^{j}, C(=O)OR^{k}, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R³ can be optionally substituted by the same or different G²;
R⁴ and R⁵ are the same or different, and each independently selected from hydrogen, deuterium, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R⁴ and R⁵ represent two same or different C₁₋₁₂ alkyl groups, the two same or different C₁₋₁₂ alkyl groups can be connected to each other and form a heteroalicycle together with the S atom to which the two same or different C₁₋₁₂ alkyl groups are commonly connected, the heteroalicycle can optionally contain one or more additional heteroatoms selected from O, N or S(=O)_{w}; one or more hydrogens in R₄ and R₅ can be optionally substituted by the same or different G³;
R^{a}, R^{b}, R^{c}, R^{d}, R^{e}, R^{f}, R^{g}, R^{h}, Rⁱ, R^{j}, or R^{k} is each independently selected from hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R^{a}, R^{b}, R^{c}, R^{d}, R^{e}, R^{f}, R^{g}, R^{h}, Rⁱ, R^{j}, or R^{k} can be optionally substituted by the same or different G⁴;
R^{b} and R^{c} are the same or different, when R^{b} and R^{c} represent two same or different C₁₋₁₂ alkyl groups connected to the same nitrogen atom, the two same or different C₁₋₁₂ alkyl groups can be optionally connected with each other, and form a heteroalicycle together with the nitrogen atom, the heteroalicycle can optionally contain one or more additional heteroatoms selected from O, N or S(=O)_{W};
R^{f} and R^{g} are the same or different, when R^{f} and R^{g} represent two same or different C₁₋₁₂ alkyl groups connected to the same nitrogen atom, the two same or different C₁₋₁₂ alkyl groups can be optionally connected with each other, and form a heteroalicycle together with the nitrogen atom, the heteroalicycle can optionally contain one or more additional heteroatoms selected from O, N or S(=O)_{W};
R¹ and R^{j} are the same or different, when Rⁱ and R^{j} represent two same or different C₁₋₁₂ alkyl groups connected to the same nitrogen atom, the two same or different C₁₋₁₂ alkyl groups can be optionally connected with each other, and form a heteroalicycle together with the nitrogen atom, the heteroalicycle can optionally contain one or more additional heteroatoms selected from O, N or S(=O)_{W};
G¹, G², G³, and G⁴ are the same or different, and each independently selected from one or more same or different deuterium, halogen, CN, NO₂, OH, OCF₃, CF₃, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, C₅₋₁₂ heteroaryl, R⁶O-, R⁷R⁸N-, R⁶S(=O)_{W}-, R⁷R⁸NS(=O)_{W}-, R⁶C(=O)-, R⁷R⁸NC(=O)-, R⁶OC(=O)-, R⁶C(=O)O-, R⁷R⁸NC(=O)O-, R⁶C(=O)NR⁹-, R⁷R⁸NC(=O)NR⁹-, R⁶OC(=O)NR⁹-, R⁶S(=O)_{W}NR⁹-, R⁷R⁸NS(=O)_{W}NR⁹-, R⁷R⁸NC(=NR¹⁰)NR⁹-, R⁷R⁸NC(=CHNO₂)NR⁹-, R⁷R⁸NC(=N-CN)NR⁹-, R⁷R⁸NC(=NR¹⁰)-, R⁶S(=O)(=NR¹⁰)NR⁹-, or R⁷R⁸NS(=O)(=NR¹⁰)-, wherein one or more hydrogens in the C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl can be optionally substituted by the same or different deuterium, halogen, CN, NO₂, OH, OCF₃, CF₃, C₁₋₁₂ alkyl, C₃₋₁₂ cycloalkyl, C3-12 heteroalicyclyl, C₂₋₁₂ alkenyl, C₂₋₁₂ alkynyl, C₆₋₁₂ aryl, C₅₋₁₂ heteroaryl, R⁶O-, R⁷R⁸N-, R⁶S(=O)_{W}-, R⁷R⁸NS(=O)_{W}-, R⁶C(=O)-, R⁷R⁸NC(=O)-, R⁶OC(=O)-, R⁶C(=O)O-, R⁷R⁸NC(=O)O-, R⁶C(=O)NR⁹-, R⁷R⁸NC(=O)NR⁹-, R⁶OC(=O)NR⁹-, R⁶S(=O)_{W}NR⁹-, R⁷R⁸NS(=O)_{W}NR⁹-, R⁷R⁸NC(=NR¹⁰)NR⁹-, R⁷R⁸NC(=CHNO₂)NR⁹-, R⁷R⁸NC(=N-CN)NR⁹-, R⁷R⁸NC(=NR¹⁰)-, R⁶S(=O)(=NR¹⁰)NR⁹- or R⁷R⁸NS(=O)(=NR¹⁰)-;
R⁶, R⁷, R⁸, R⁹, and R¹⁰ are the same or different, and each independently selected from hydrogen, deuterium, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R⁷ and R⁸ are two same or different C₁₋₁₂ alkyl groups connected to the same nitrogen atom, the two same or different C₁₋₁₂alkyl groups can be connected to each other, and form a heteroalicycle together with the nitrogen atom, the heteroalicycle can optionally comprise one or more additional heteroatoms selected from O, N or S(=O)_{W}; and one or more hydrogens in R⁶, R⁷, R⁸, R⁹ and R¹⁰ can be further optionally substituted by the same or different deuterium, halogen, OH, CN, NO₂, OCH₃, OCF₃, C₁₋₁₂ alkyl or C₃₋₁₂ cycloalkyl; and
w is 0, 1, or 2.

Preferably, in the above-mentioned heterocyclic sulfoximine compound, or a racemate, an enantiomer, a pharmaceutically acceptable salt or a solvate thereof, the structural formula of the compounds is (IIa) or (IIb): 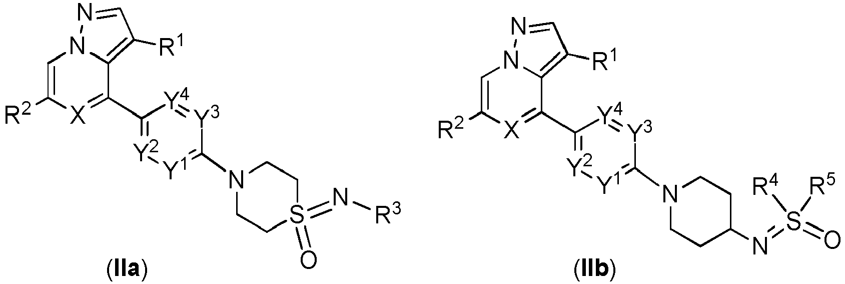 wherein, X, Y¹, Y², Y³, Y⁴, R¹, R², R³, R⁴ and R⁵ have the same definitions as described above.

Preferably, in the above-mentioned heterocyclic sulfoximine compound, or a racemate, an enantiomer, a pharmaceutically acceptable salt or a solvate thereof, the structural formula of the compounds is (IIIa), (IIIb), (IIIc) or (IIId): 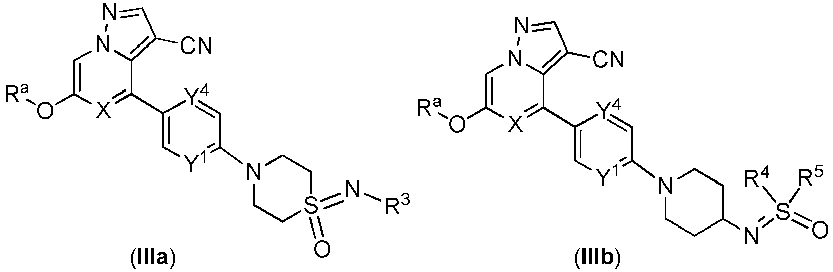 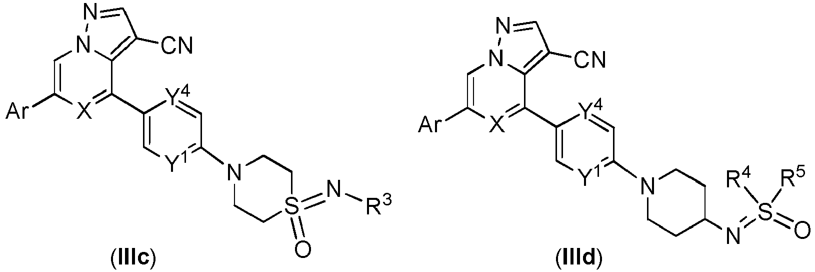 wherein,
X is N or C-R;
Y¹ and Y⁴ are the same or different N or C-R';
R^{a} is selected from hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R^{a} can be optionally substituted by the same or different G¹;
R and R' are each independently selected from hydrogen, deuterium, halogen, CN, C₁₋₁₂ alkyl, OH or C₁₋₁₂ alkyl-O-, one or more hydrogens in the C₁₋₁₂ alkyl groups can be optionally substituted by the same or different deuterium, halogen, CN, NO₂, OH, OCH₃, OCF₃ or CF₃;
Ar represents C₆₋₁₂ aryl or C₅₋₁₂ heteroaryl; one or more hydrogens in Ar can be optionally substituted by the same or different G¹; and
R³, R⁴, R⁵ and G¹ have the same definitions as described above.

Preferably, in the above-mentioned heterocyclic sulfoximine compound, or a racemate, an enantiomer, a pharmaceutically acceptable salt or a solvate thereof, the structural formula of the compounds is (IVa), (IVb), (IVc), (IVd), (IVe), (IVf), (IVg), or (IVh): 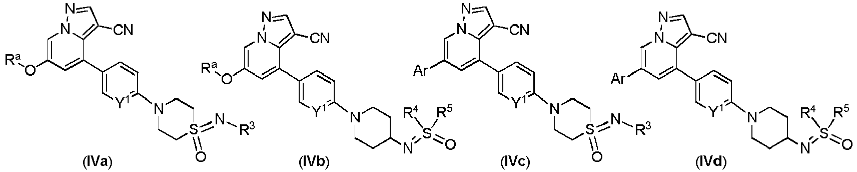 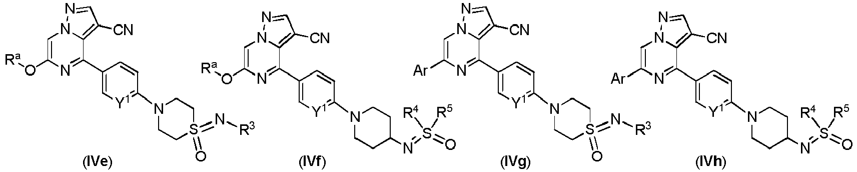 wherein,
Y¹ represents N or CH;
R^{a} is selected from hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R^{a} can be optionally substituted by the same or different G¹;
Ar represents C₆₋₁₂ aryl or C₅₋₁₂ heteroaryl; one or more hydrogens in Ar can be optionally substituted by the same or different G¹;
R³ represents hydrogen, deuterium, C₁₋₁₂ alkyl, C₂₋₁₂ alkenyl, C₂₋₁₂ alkynyl, C₃₋₁₂cycloalkyl, C₃₋₁₂ heteroalicyclyl, C₆₋₁₂ aryl, C₅₋₁₂ heteroaryl, S(=O)_{w}R^{d}, S(=O)_{w}NR^{f}R^{g}, C(=O)R^{h}, C(=O)NRⁱR^{j}, or C(=O)OR^{k}; one or more hydrogens in R³ can be optionally substituted by the same or different G²;
R^{d}, R^{f}, R^{g}, R^{h}, Rⁱ, R^{j} or R^{k} is each independently selected from hydrogen, deuterium, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R^{d}, R^{f}, R^{g}, R^{h}, Rⁱ, R^{j} or R^{k} can be further optionally substituted by the same or different deuterium, halogen, CN, NO₂, OH, OCH₃, OCF₃, C₁₋₁₂ alkyl or C₃₋₁₂ cycloalkyl;
R^{f} and R^{g} are the same or different, when R^{f} and R^{g} represent two same or different C₁₋₁₂ alkyl groups connected to the same nitrogen atom, the two same or different C₁₋₁₂ alkyl groups can be optionally connected with each other, and form a heteroalicycle together with the nitrogen atom, the heteroalicycle can optionally comprises one or more additional heteroatoms selected from O, N or S(=O)_{W};
Rⁱ and R^{j} are the same or different, when Rⁱ and R^{j} represent two same or different C₁₋₁₂ alkyl groups connected to the same nitrogen atom, the two same or different C₁₋₁₂ alkyl groups can be optionally connected with each other, and form a heteroalicycle together with the nitrogen atom, the heteroalicycle can optionally comprise one or more additional heteroatoms selected from O, N or S(=O)_{W};
R⁴ and R⁵ are the same or different, and each independently selected from hydrogen, deuterium, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R⁴ and R⁵ each represent a same or different C₁₋₁₂ alkyl groups, the two C₁₋₁₂ alkyl groups can be connected to each other, and form a heteroalicycle together with the sulfur atom to which the two C₁₋₁₂ alkyl groups are commonly connected, the heteroalicycle can optionally comprise one or more additional heteroatoms selected from O, N or S(=O)_{w}; one or more hydrogens in R⁴ and R⁵ can be optionally substituted by the same or different G³; and
w, G¹, G² and G³ have the same definitions as described above.

Preferably, in the above-mentioned heterocyclic sulfoximine compound, or a racemate, an enantiomer, a pharmaceutically acceptable salt or a solvate thereof,
R^{a} is selected from hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R^{a} can be optionally substituted by the same or different deuterium, halogen, CN, NO₂, OH, CH₃, OCH₃, OCF₃, CF₃;
preferably, R^{a} is selected from hydrogen, C₁₋₁₂ alkyl, C₃₋₁₂ cycloalkyl, C₃₋₁₂ heteroalicyclyl, C₂₋₁₂ alkenyl, or C₂₋₁₂ alkynyl;
more preferably, R^{a} is selected from hydrogen or C₁₋₁₂ alkyl.

Preferably, in the above-mentioned heterocyclic sulfoximine compound, or a racemate, an enantiomer, a pharmaceutically acceptable salt or a solvate thereof,
Ar represents C₆₋₁₂ aryl or C₅₋₁₂ heteroaryl; one or more hydrogens in Ar can be optionally substituted by the same or different hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl;
preferably, Ar represents C₅₋₁₂ heteroaryl; one or more hydrogens in Ar can be optionally substituted by the same or different hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl;
more preferably, one or more hydrogens in Ar can be optionally substituted by the same or different hydrogen or C₁₋₁₂ alkyl.

Preferably, in the above-mentioned heterocyclic sulfoximine compound, or a racemate, an enantiomer, a pharmaceutically acceptable salt or a solvate thereof,
R³ represents hydrogen, deuterium, C₁₋₁₂ alkyl, C₃₋₁₂ cycloalkyl, C₃₋₁₂ heteroalicyclyl, C₅₋₁₂ heteroaryl, or -C(=O)O-C₁₋₁₂ alkyl;
one or more hydrogens in R³ can be optionally substituted by the same or different deuterium, halogen, CN, NO₂, OH, OCF₃, CF₃, C₁₋₁₂ alkyl-O, C₃₋₁₂ cycloalkyl-O, C₃₋₁₂ heteroalicyclyl-O, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, C₅₋₁₂ heteroaryl or -C(=O)O-C₁₋₁₂ alky, wherein one or more hydrogens in the C₁₋₁₂ alkyl, C₂₋₁₂ alkenyl, C₂₋₁₂ alkynyl, C₃₋₁₂cycloalkyl, C₃₋₁₂ heteroalicyclyl, C₆₋₁₂ aryl, C₅₋₁₂ heteroaryl or -C(=O)O-C₁₋₁₂ alkyl can be further optionally substituted by the same or different deuterium, halogen, CN, NO₂, OH, OCF₃, CF₃, C₁₋₁₂ alkyl, C₁₋₁₂ alkyl-O, C₃₋₁₂ cycloalkyl-O or C₃₋₁₂ heteroalicyclyl-O;
more preferably, R³ represents hydrogen, deuterium, C₁₋₁₂ alkyl, C₃₋₁₂ cycloalkyl, C₃₋₁₂ heteroalicyclyl, C₅₋₁₂ heteroaryl, or -C(=O)O-C₁₋₁₂ alkyl; and
one or more hydrogens in R³ can be optionally substituted by the same or different deuterium, C₁₋₁₂ alkyl-O, C₃₋₁₂ cycloalkyl-O, C₃₋₁₂ heteroalicyclyl-O, C₁₋₁₂ alkyl, C₃₋₁₂ cycloalkyl, C₆₋₁₂ aryl, C₅₋₁₂ heteroaryl, or -C(=O)O-C₁₋₁₂ alkyl, wherein one or more hydrogens in the C₁₋₁₂ alkyl, C₃₋₁₂ cycloalkyl, C₆₋₁₂ aryl, C₅₋₁₂ heteroaryl or -C(=O)O-C₁₋₁₂ alkyl can be further optionally substituted by the same or different deuterium, C₁₋₁₂ alkyl-O, C₃₋₁₂ cycloalkyl-O or C₃₋₁₂ heteroalicyclyl-O.

Preferably, in the above-mentioned heterocyclic sulfoximine compound, or a racemate, an enantiomer, a pharmaceutically acceptable salt or a solvate thereof,
R⁴ and R⁵ are the same or different, and each independently selected from C₁₋₁₂ alkyl or C₆₋₁₂ aryl;
one or more hydrogens in R⁴ and R⁵ can be optionally substituted by the same or different deuterium, halogen, CN, NO₂, OH, OCF₃, CF₃, C₁₋₁₂ alkyl-O, C₃₋₁₂ cycloalkyl-O, C₃₋₁₂ heteroalicyclyl-O, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl, wherein one or more hydrogens in the C₁₋₁₂ alkyl, C₂₋₁₂ alkenyl, C₂₋₁₂ alkynyl, C₃₋₁₂ cycloalkyl, C₃₋₁₂ heteroalicyclyl, C₆₋₁₂ aryl, or C₅₋₁₂ heteroaryl can be further optionally substituted by the same or different deuterium, halogen, CN, NO₂, OH, OCF₃, CF₃, C₁₋₁₂ alkyl, C₁₋₁₂ alkyl-O, C₃₋₁₂ cycloalkyl-O or C₃₋₁₂ heteroalicyclyl-O;
preferably, R⁴ and R⁵ are the same or different, and each independently selected from C₁₋₆ alkyl or C₆ aryl; and
one or more hydrogens in R⁴ and R⁵ can be optionally substituted by the same or different deuterium, halogen, CN, NO₂, OH, OCF₃, CF₃, C₁₋₁₂ alkyl-O, C₃₋₁₂ cycloalkyl-O, C₃₋₁₂ heteroalicyclyl-O, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl, where one or more hydrogens in the C₁₋₁₂ alkyl, C₂₋₁₂ alkenyl, C₂₋₁₂ alkynyl, C₃₋₁₂ cycloalkyl, C₃₋₁₂ heteroalicyclyl, C₆₋₁₂ aryl, or C₅₋₁₂ heteroaryl can be further optionally substituted by the same or different deuterium, halogen, CN, NO₂, OH, OCF₃, CF₃, C₁₋₁₂ alkyl, C₁₋₁₂ alkyl-O, C₃₋₁₂ cycloalkyl-O, or C₃₋₁₂ heteroalicyclyl-O.

Preferably, in the above-mentioned heterocyclic sulfoximine compound, or a racemate, an enantiomer, a pharmaceutically acceptable salt or a solvate thereof, the structural formula of the compounds is any one of the following:

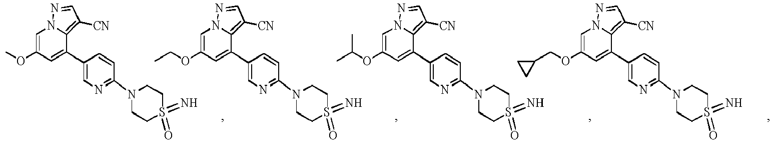

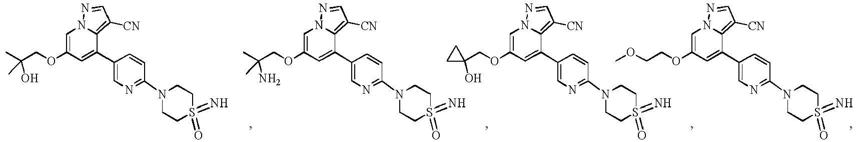

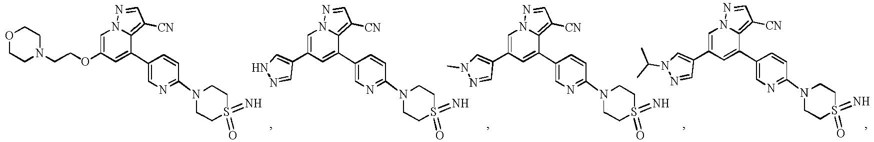

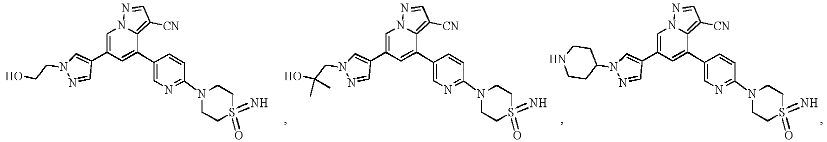

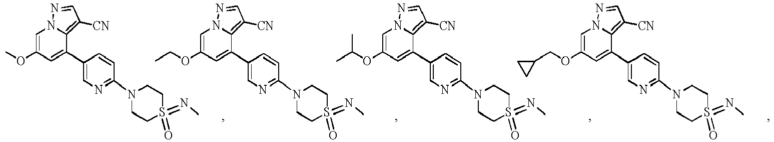

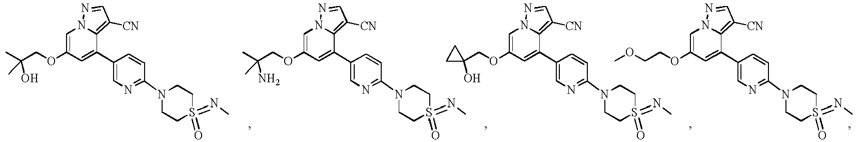

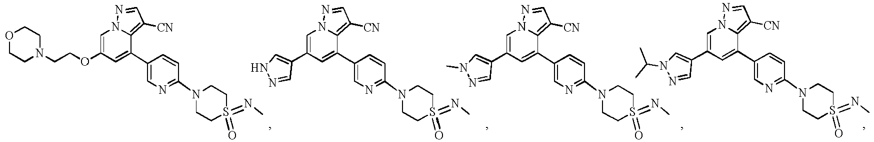

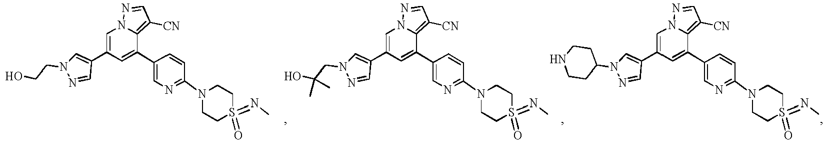

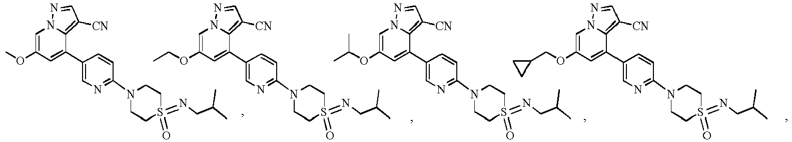

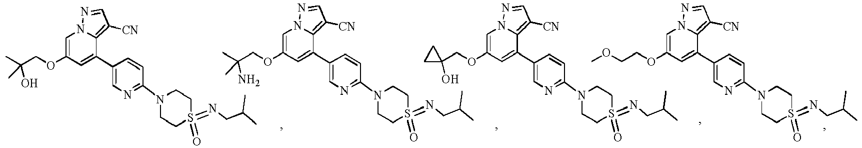

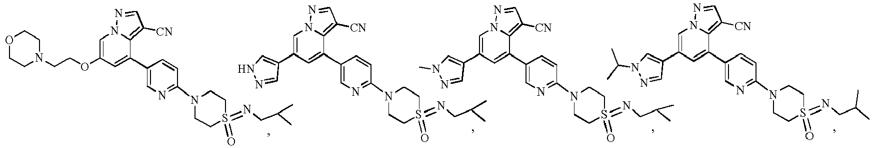

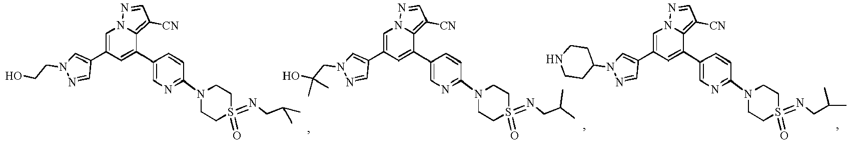

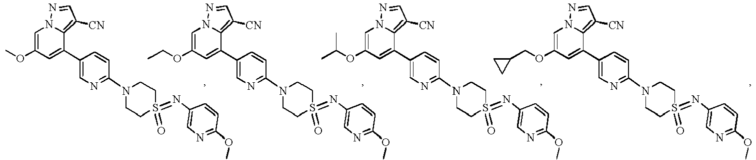

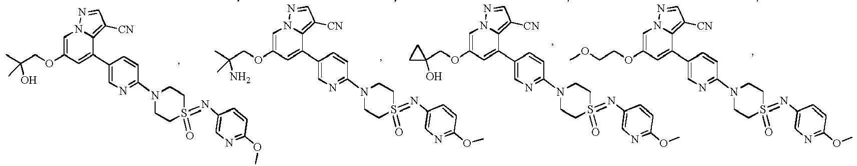

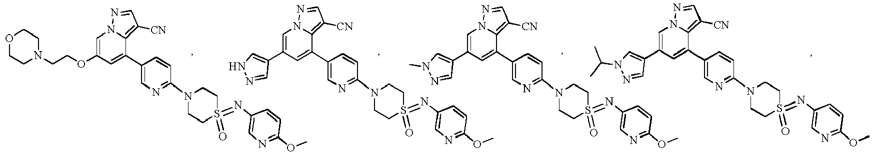

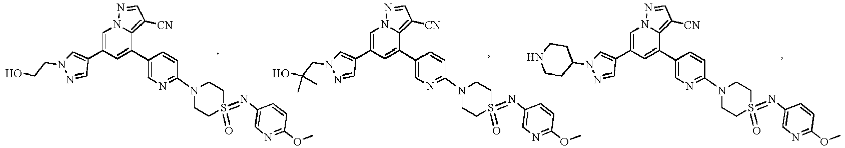

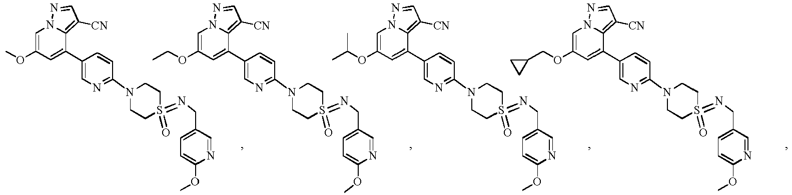

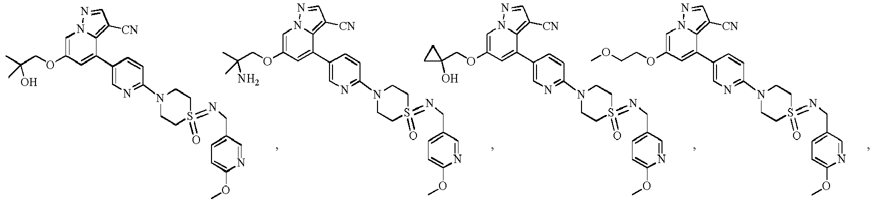

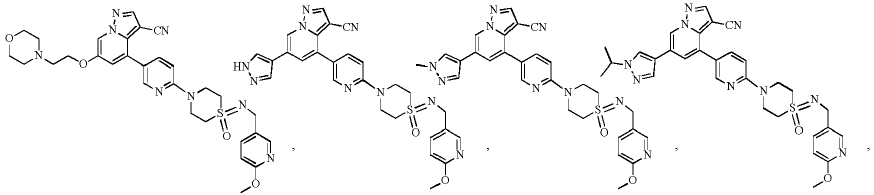

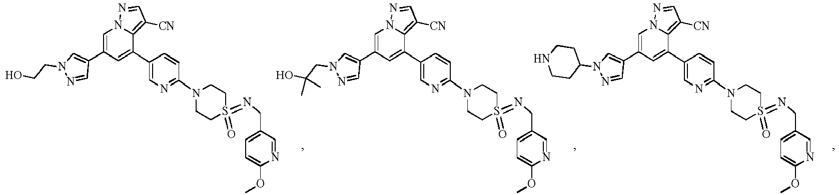

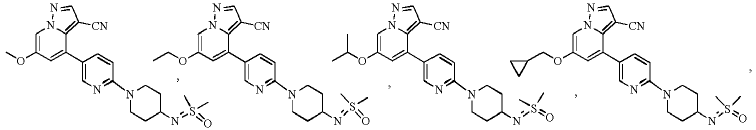

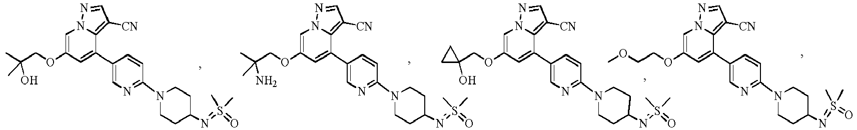

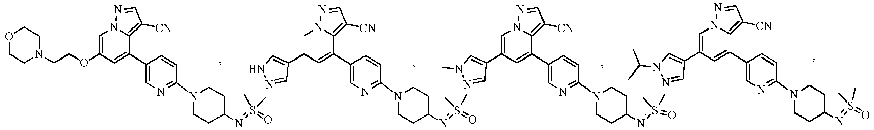

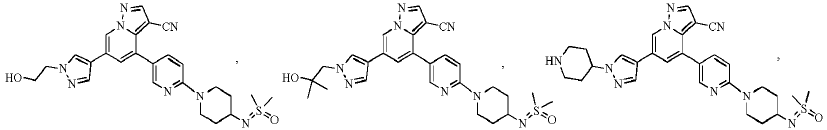

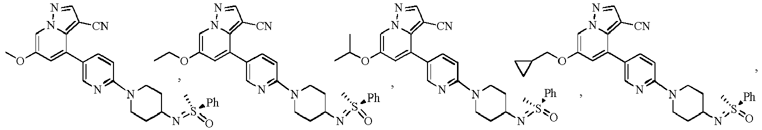

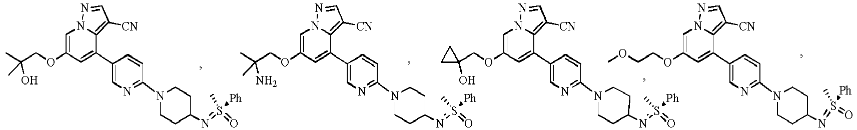

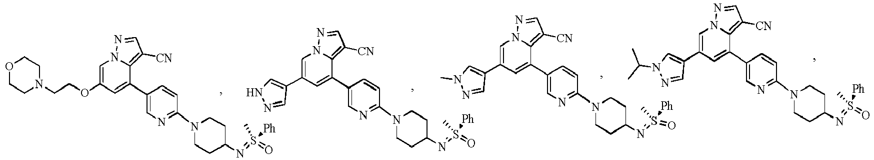

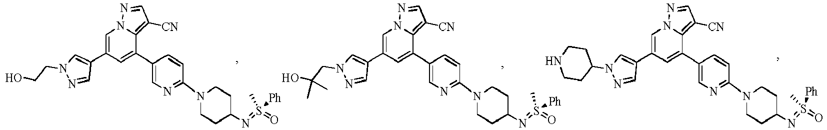

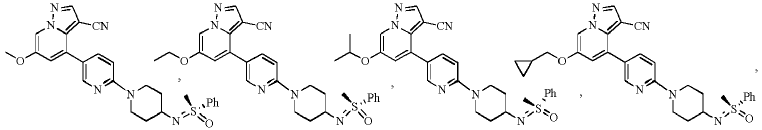

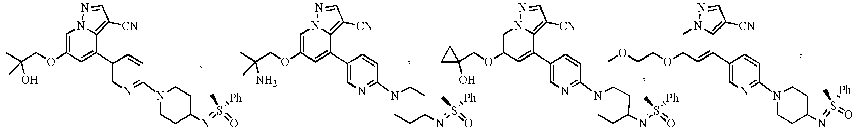

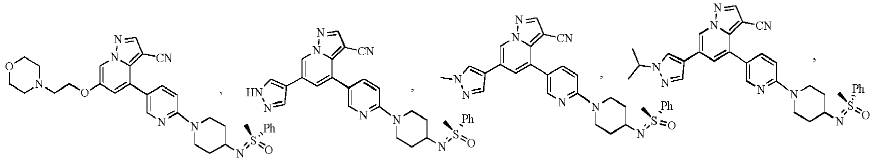

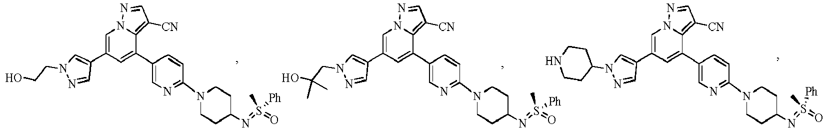

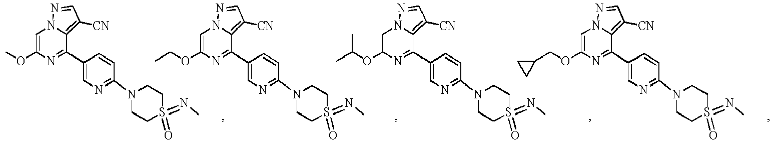

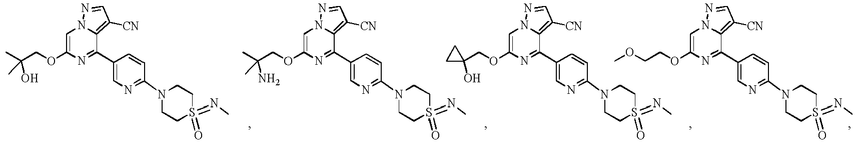

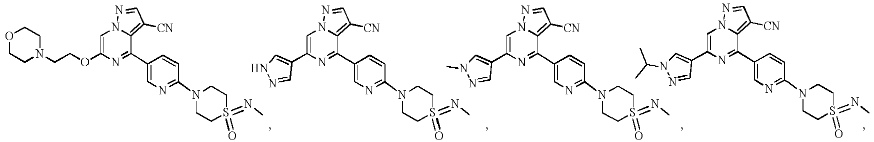

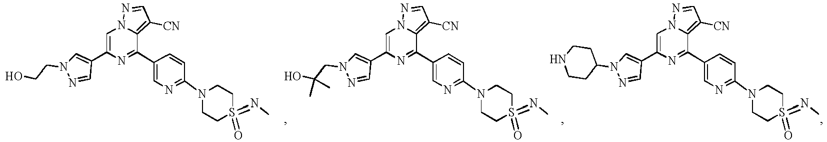

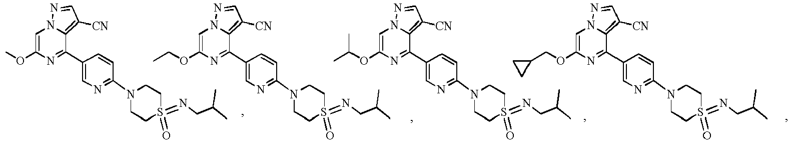

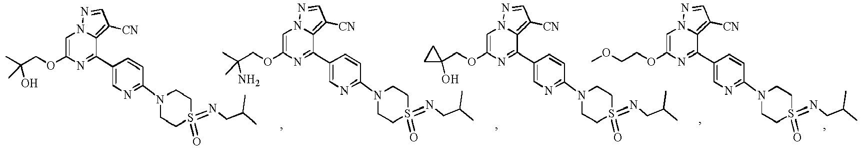

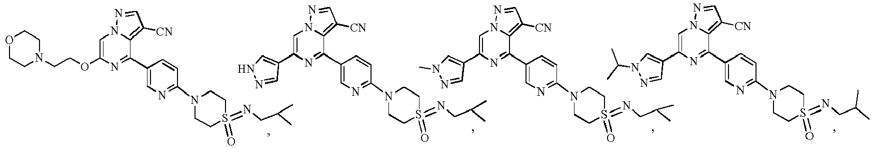

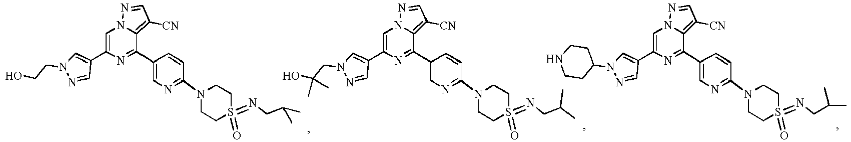

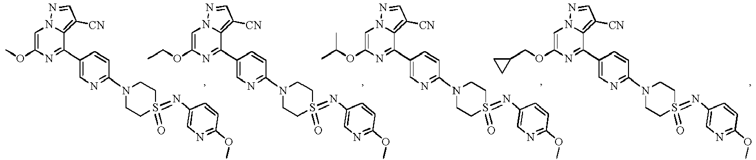

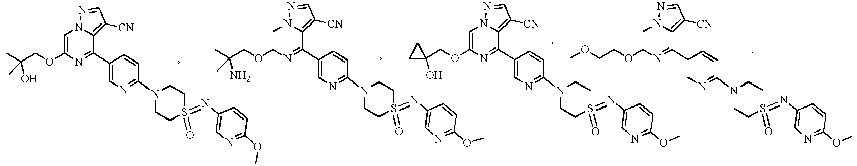

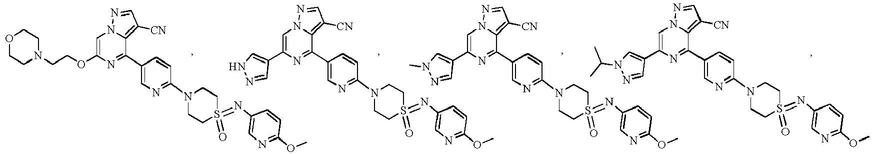

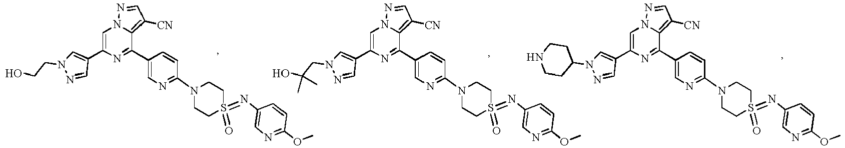

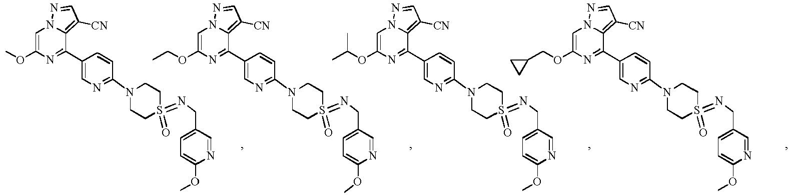

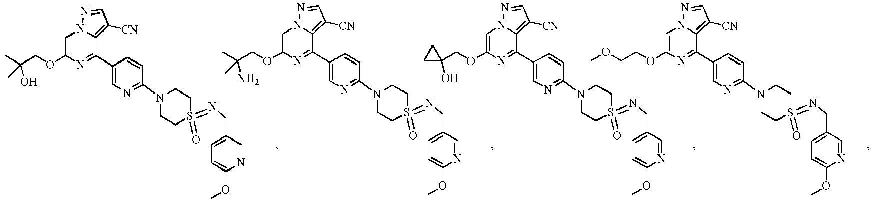

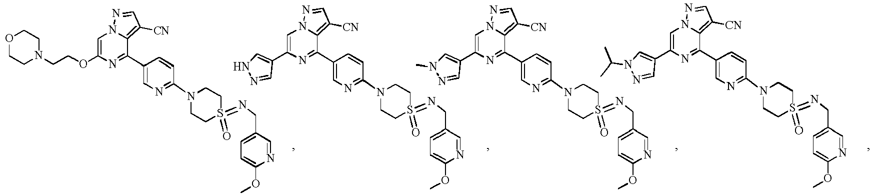

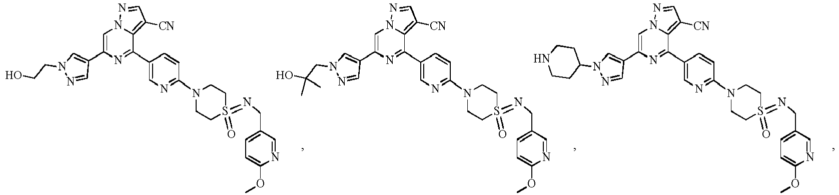

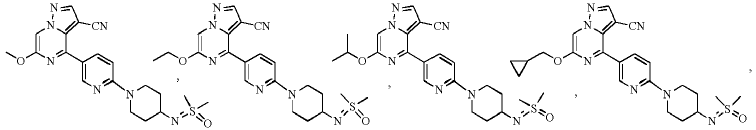

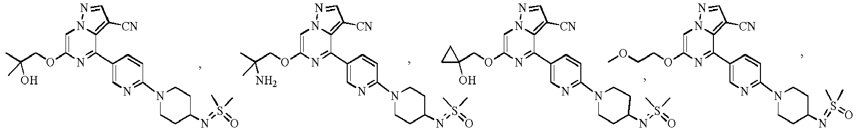

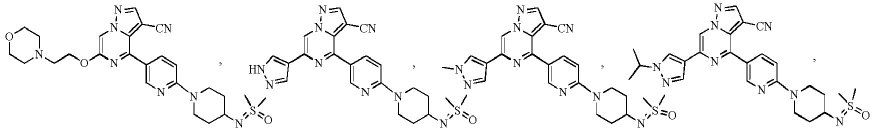

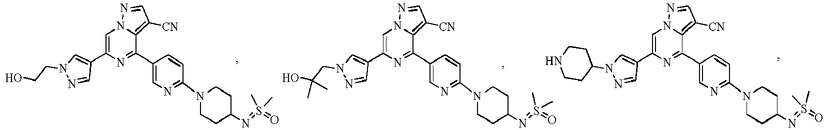

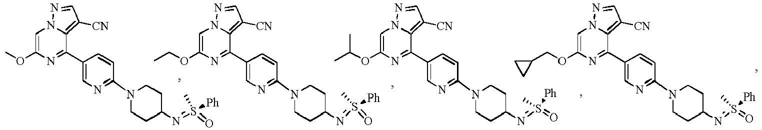

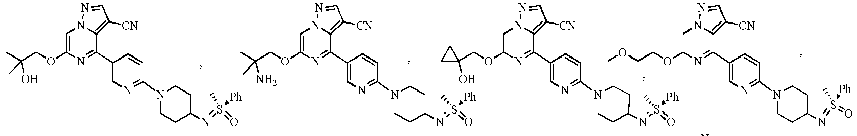

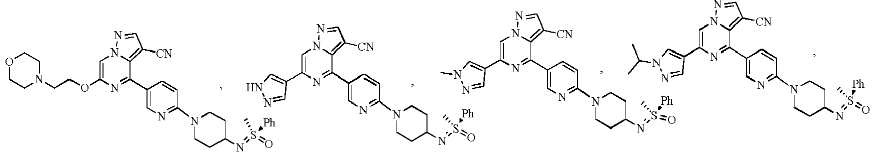

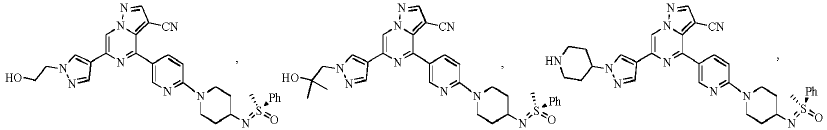

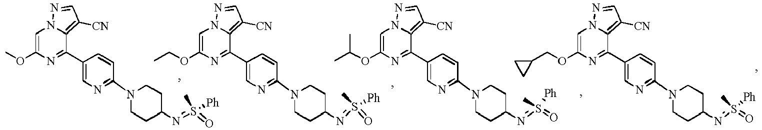

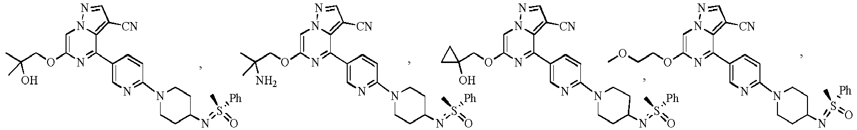

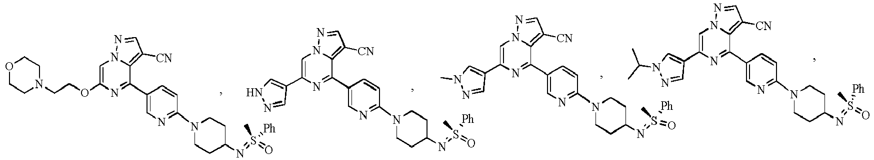

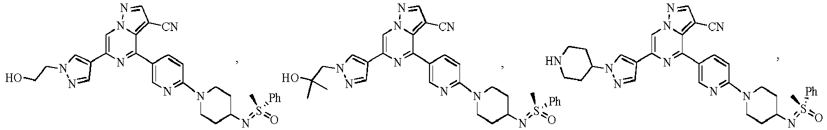

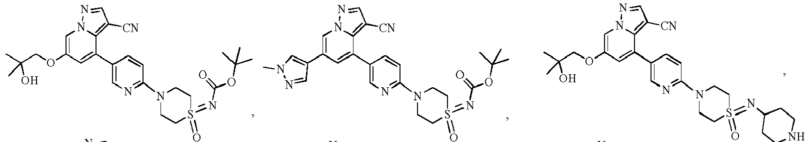

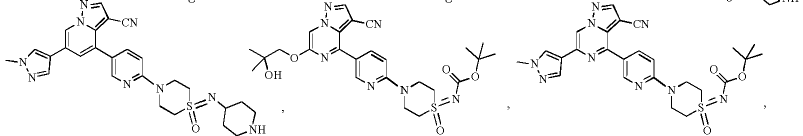

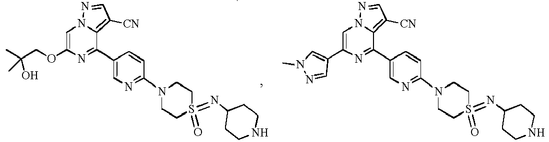

The present invention also provides an intermediate compound for synthesizing the above-mentioned heterocyclic sulfoximine compound, the structural formula of the intermediate compound is (Va), (Vb), (Vc), (Vd), or (Ve): 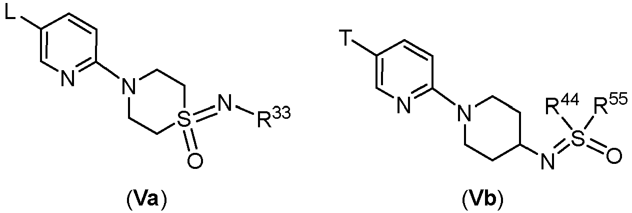 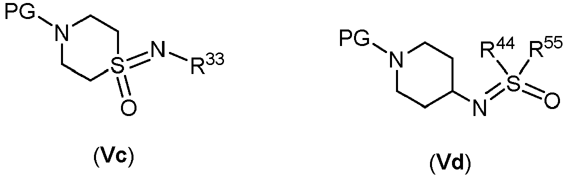 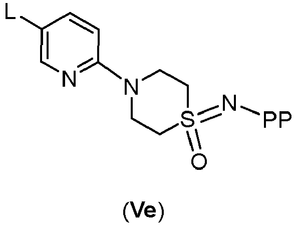 wherein:
L and T are independently selected from chlorine, bromine, iodine, F₃C-SO₃, a boronic acid group, a boronic ester group, or a boronic acid salt group;
PG is selected from hydrogen, (tert-butoxy)C(=O)-, (benzyloxy)C(=O)-, (p-methylbenzyloxy)C(=O)-, or benzyl;
PP is an N protecting group;
R³³ is selected from hydrogen, (tert-butoxy)C(=O)-, (benzyloxy) C(=O)-, (p-methylbenzyloxy)C(=O)-, benzyl, trifluoroacetyl, acetyl, S(=O)_{w}R^{d}, S(=O)_{w}NR^{f}R^{g}, C(=O)R^{h}, C(=O)NRⁱR^{j}, C(=O)OR^{k}, C₁₋₁₂ alkyl, C₃₋₁₂ cycloalkyl, C₃₋₁₂ heteroalicyclyl, C₂₋₁₂ alkenyl, C₂₋₁₂ alkynyl, C6-12 aryl, or C₅₋₁₂ heteroaryl, wherein one or more hydrogens in the C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl can be optionally substituted by the same or different G²;
R⁴⁴ and R⁵⁵ are the same or different, and each independently selected from C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R⁴⁴ and R⁵⁵ represent two same or different C₁₋₁₂ alkyl groups, the two same or different C₁₋₁₂ alkyl groups can be connected to each other, and form a heteroalicycle together with the sulfur atom to which the two C₁₋₁₂ alkyl groups are commonly connected, the heteroalicycle can optionally comprise one or more additional heteroatoms selected from O, N or S(=O)_{w}; one or more hydrogens in R⁴⁴ and R⁵⁵ can be optionally substituted by the same or different G³;
in the structural formula (Vd), when PG is (benzyloxy)C(=O)-, R⁴⁴ and R⁵⁵ cannot be methyl at the same time;
G², G³, R^{d}, R^{f}, R^{g}, R^{h}, Rⁱ, R^{j}, R^{k}, and w have the same definitions as described above;
preferably, the boronic acid group, boronic ester group or boronic acid salt group is selected from (HO)₂B, (CH₃O)₂B, (CH₃CH₂O)₂B, (isopropyl-O)₂B, 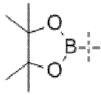 , BF₃K or BF₃Na; and
PP is selected from Boc, CBZ, trifluoroacetyl, acetyl, Bn or PMB.

The present invention also provides a synthetic method of the above-mentioned intermediate compound, and as an embodiment, this method can be composed of the steps shown in Scheme A: 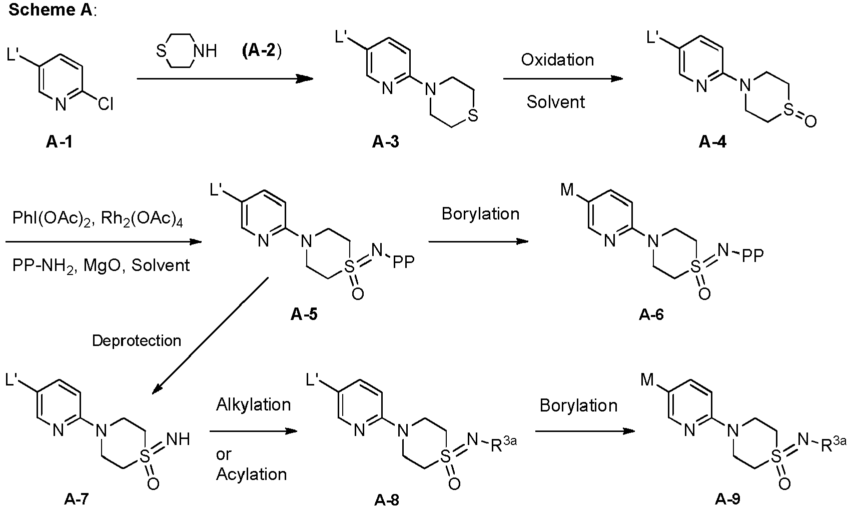 wherein:
the starting materials A-1 and A-2 can be purchased from commercial sources;
L' is selected from chlorine, bromine, iodine, or F₃C-SO₃;
M is selected from a boronic acid group, a boronic ester group or a boronic acid salt group;
R^{3a} is selected from S(=O)_{w}R^{d}, S(=O)_{w}NR^{f}R^{g}, C(=O)R^{h}, C(=O)NRⁱR^{j}, C(=O)OR^{k}, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl, wherein one or more hydrogens in the C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl can be optionally substituted by the same or different G²;
PP, w, G², R^{d}, R^{f}, R^{g}, R^{h}, Rⁱ, R^{j} and R^{k} have the same definitions as described above;
preferably, M is selected from (HO)₂B, (CH₃O)₂B, (CH₃CH₂O)₂B, (isopropyl-O)₂B, BF₃K, or BF₃Na.

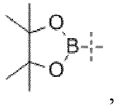

The oxidant used in oxidation includes, but is not limited to, NaIO₄, m-chloroperoxybenzoic acid (mCPBA), H₂O₂, CH₃CO₃H and the like;
"Borylation" refers to a reaction with the conversion of halogen or F₃C-SO₃ into boronic acid, boronic acid ester, or boronic acid salt. The reaction can generally proceed by two means of: (a) performing halogen-metal exchange followed by reacting with organoboronates including, but not limited to, methyl borates, ethyl borates, isopropyl borates and the like; (b) reacting with bis(pinacolato)diboron in the presence of Pd catalyst to form boronic acid ester;
"Deprotection" represents the removal of the protecting group from N. When the protecting group is Boc (i.e., CO₂Bu-*t*), the commonly used deprotection reagent includes, but not limited to, HCl, trifluoroacetic acid, H₂SO₄ and the like. When the protecting group is CBZ (i.e., -CO₂CH₂Ph), the commonly used deprotection reagent includes, but not limited to, concentrated HCl, H₂+Pd/C and the like. When the protecting group is Bn (i.e., CH₂Ph), the commonly used deprotection reagent includes, but not limited to, H₂+Pd/C, H₂+Pd(OH)₂, H₂+Pd/C+HCl and the like. When the protecting group is PMB (i.e., -CH₂C₆H₄-OCH₃-*p*), the commonly used deprotection reagent includes, but not limited to, trifluoroacetic acid, ceric ammonium nitrate and the like.
"Alkylation" refers to the introduction of an alkyl group on the N atom of the sulfoximine group, which is commonly achieved by two methods: (a) subjecting the sulfoximine intermediate A-7 to a nucleophilic substitution with alkyl halide or alkyl sulfonate; (b) subjecting the sulfoximine intermediate A-7 to a reductive amination with aldehyde or ketone compound in the presence of a reducing reagent including, but not limited to, NaBH₄, NaBH₃CN, NaB(OAc)₃H, BH₃, BH₃.THF, BH₃.S(CH₃)₂, BH₃.(2-methylpyridine), LiAlH₄, H₂+Pd/C and the like;
"Acylation" refers to a reaction of introducing an acyl group, a sulfonyl group, a carbamido, a sulfonamide group and the like on the N atom of a sulfoximine group, which is commonly achieved by two methods: (a) reacting the sulfoximine intermediate A-7 with acyl chloride, sulfonyl chloride, carbamoyl chloride, sulfamoyl chloride and the like; (b) subjecting the sulfoximine intermediate A-7 to a coupling reaction with carboxylic acid in the presence of a coupling reagent.

The meanings of "base", "solvent", "Pd catalyst" and "coupling reagent" are given in the "Definition of Terms" section.

The present invention also provides a preparation method for the above-mentioned heterocyclic sulfoximine compound, as an embodiment, the method can be composed of the steps shown in Scheme B: 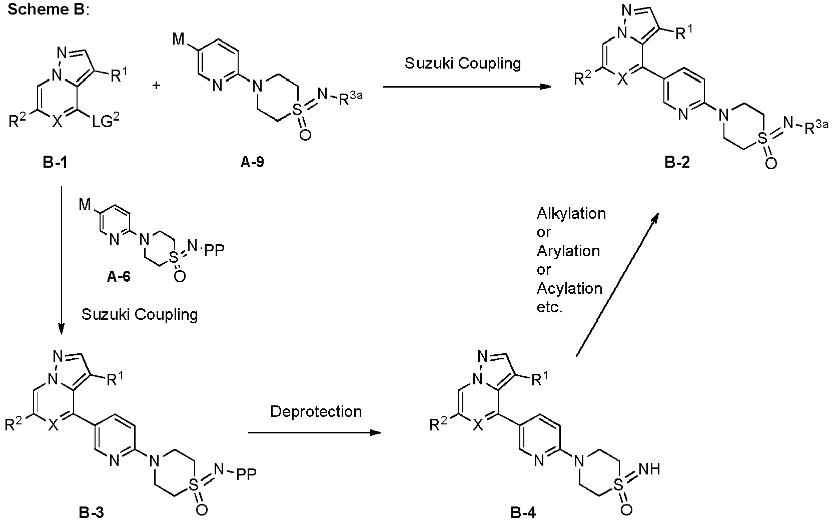 wherein:
the starting material B-1 can be synthesized by methods reported in the literatures, including, but not limited to, WO2015017610, WO2017011776, WO2019075092, WO2019075108, and the like;
LG² represents chlorine, bromine, iodine or F₃C-SO₃; and
R¹, R², X, PP, M and R^{3a} have the same definitions as described above.

"Arylation" refers to a reaction of the intermediate B-4 with aryl halide, heteroaryl halide, aryl triflate, heteroaryl triflate in the presence of palladium catalyst, or a reaction of the intermediate B-4 with aryl halide or heteroaryl halide in the presence of base, to obtain a product B-2.

"Suzuki coupling", which is one of the most commonly used chemical reactions in organic chemistry and pharmaceutical chemistry, refers to a reaction of the heteroaryl halide or triflate B-1with boric acid, boric acid ester, or boric acid salt A-9 or A-6 in the presence of palladium catalyst and base, to obtain a coupled product.

"Deprotection", "alkylation" and "acylation" mean the same as above. For the meanings of "base", "solvent" or "Pd catalyst" refers to "Definition of Terms" section.

The present invention also provides another preparation method of the above-mentioned intermediate compound, and as an embodiment, the method can be composed of the steps shown in Scheme C: 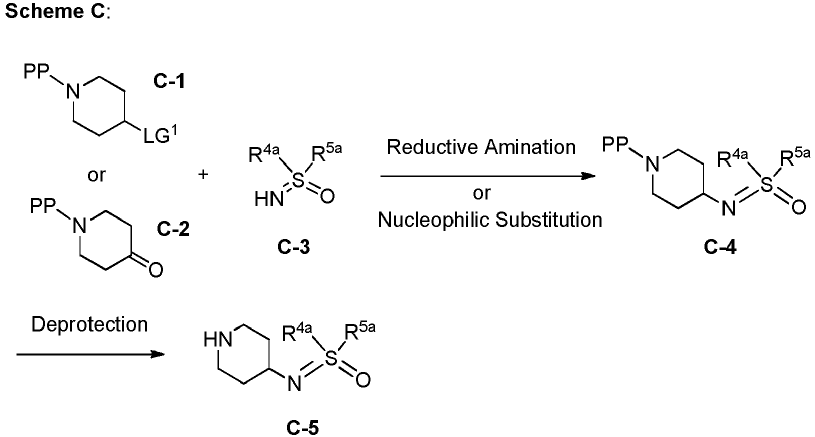 wherein:
the starting materials C-1, C-2 and C-3 can be purchased from commercial sources;
LG¹ is selected from fluorine, chlorine, bromine, iodine, H₃C-SO₃, F₃C-SO₃, C₆H₄SO₃, p-CH₃C₆H₄-SO₃, or *p*-O₂NC₆H₄-SO₃;
R^{4a} and R^{5a} are the same or different, and each independently selected from hydrogen, deuterium, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R^{4a} and R^{5a} represent two same or different C₁₋₁₂ alkyl groups, the two same or different C₁₋₁₂ alkyl groups can be connected to each other, and form a heteroalicycle together with the sulfur atom to which the two C₁₋₁₂ alkyl groups are commonly connected, the heteroalicycle can optionally comprise one or more additional heteroatoms selected from O, N or S(=O)_{w}; one or more hydrogens in R^{4a} and R^{5a} can be further optionally substituted by the same or different G³; and
PP, G³, w, deprotection, reductive amination and nucleophilic substitution have the same definitions as described above.

The present invention also provides another preparation method of the above-mentioned heterocyclic sulfoximine compound, and as an embodiment, the method can be composed of the steps shown in Scheme D: 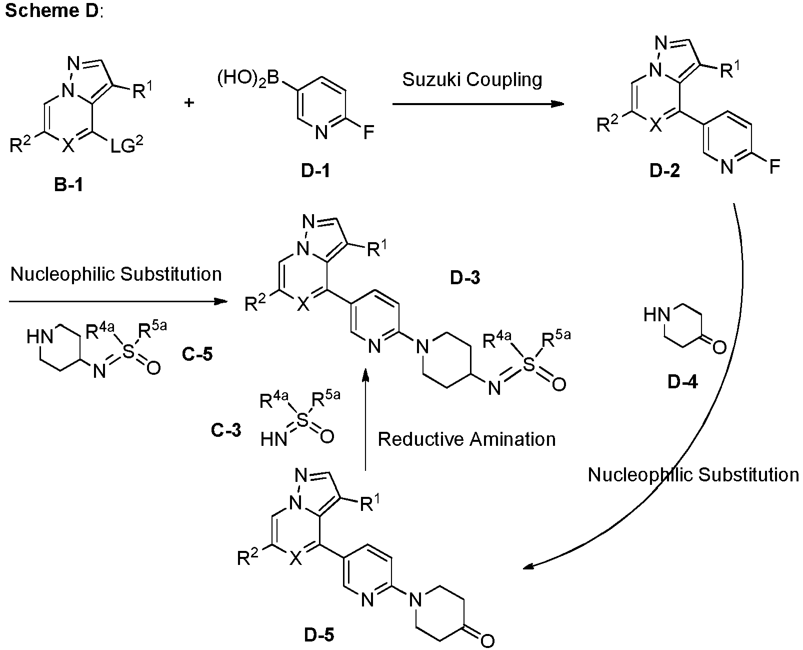 wherein:
the starting materials D-1 and D-4 can be purchased from commercial sources; the starting material B-1 is synthesized by methods reported in the literatures;
R¹, R², X, LG², R^{4a}, R^{5a}, Suzuki coupling, nucleophilic substitution and reductive amination have the same definitions as described above.

The present invention also provides a pharmaceutical composition comprising at least one of the above-mentioned heterocyclic sulfoximine compound, or a racemate, an enantiomer, a pharmaceutically acceptable salt or a solvate thereof.

Preferably, the above-mentioned pharmaceutical composition also comprises at least one of pharmaceutically acceptable carrier or diluent.

Preferably, a pharmaceutical composition that is convenient for administration is prepared with the above-mentioned heterocyclic sulfoximine compound, a racemate, an enantiomer, a diastereomer, a pharmaceutically acceptable salt, a hydrate, a solvate or a prodrug thereof, and a suitable pharmaceutically acceptable carrier, a pharmaceutically commonly used adjuvant through a formulation process.

Preferably, in the above-mentioned pharmaceutical composition, the preparation form of the pharmaceutical composition comprises: oral preparation, injection, anal plug, nostril inhalation, eye drops or skin patch.

Preferably, various dosage forms of the above-mentioned pharmaceutical composition can be prepared by methods commonly used in the pharmaceutical industry, for example, mixing, dissolving, granulating, grinding, emulsifying, encapsulating, sugar coating, freeze drying, freeze spraying, and the like.

Preferably, the above-mentioned pharmaceutical composition is used to treat mammals, such as human patients, with diseases caused by abnormal activity of RET, RET mutant, or RET fusion proteins.

Preferably, the content of the above-mentioned heterocyclic sulfoximine compound, or a racemate, an enantiomer, a pharmaceutically acceptable salt or a solvate thereof in the pharmaceutical composition is in the range of 0.001-100%. The effective amount of the pharmaceutical composition administered to mammals including humans is 0.1-500 mg per kilogram of body weight per day, and the preferred amount is 0.1-100 mg per kilogram of body weight per day. Within this effective amount range, the compounds of the present invention provide their pharmacological effects on inhibiting the activity of RET, RET mutants and/or RET fusion proteins and treating diseases (such as cancer) caused by abnormal activity of RET, RET mutants and/or RET fusion proteins.

The present invention also provides use of the above-mentioned heterocyclic sulfoximine compound, or a racemate, an enantiomer, a pharmaceutically acceptable salt or a solvate thereof or the above-mentioned pharmaceutical composition in the treatment of diseases caused by abnormal activity of RET, RET mutant, and/or RET fusion proteins. The disease is a tumor, comprising solid tumor and liquid tumor.

Preferably, in the above-mentioned use, the tumor is selected from one or any combination of lung cancer, bone cancer, pancreatic cancer, skin cancer, head and neck cancer, skin or intraocular melanoma, uterine cancer, ovarian cancer, rectal cancer, colorectal cancer, anal area cancer, stomach cancer, colon cancer, breast cancer, fallopian tube cancer, endometrial cancer, cervical cancer, vaginal cancer, vulva cancer, Hodgkin's disease, esophageal cancer, small bowel cancer, endocrine system cancer, thyroid cancer, parathyroid cancer, soft tissue sarcoma, urethral cancer, penile cancer, prostate cancer, chronic or acute leukemia, bladder cancer, kidney or ureter cancer, kidney cancer, adrenal cancer, renal cell carcinoma, renal pelvis cancer, brain glioma, brain stem glioma, neuroendocrine glioma, glioma, central nervous system (CNS) neoplasms, spinal axis tumors, pituitary adenoma, gastrointestinal stromal tumor, colorectal cancer, non-small cell lung cancer, small cell lung cancer, mastocytosis, glioma, sarcoma, lymphoma.

The present invention also provides a medicine for treating diseases caused by abnormal activity of RET, RET mutants or RET fusion proteins, comprising any one or any several of the above-mentioned heterocyclic sulfoximine compounds or pharmaceutically acceptable salts, solvates, prodrugs thereof, or any one or any several of racemates, enantiomers, diastereomers of the above-mentioned heterocyclic sulfoximine compounds, or a mixture of different enantiomers or diastereomers in any ratio, or pharmaceutically acceptable salts, solvates or prodrugs thereof.

Preferably, the above-mentioned medicines for treating diseases caused by abnormal activity of RET, RET mutants or RET fusion proteins also comprise one or more pharmaceutically acceptable carriers or/and diluents.

Preferably, the preparation forms of the above-mentioned medicines are as follows:
(1) oral preparation, (2) injection, (3) anal plug, (4) nostril inhalation, (5) eye drops or (6) skin patch.

Preferably, the routes of administration of the above-mentioned medicines can comprise: (1) oral administration: such as tablets, capsules and the like; (2) injection: such as intravenous injection, subcutaneous injection, intramuscular injection, eyeball injection, intraperitoneal injection and the like; (3) anal injection plugs: such as suppositories, gels and the like; (4) nostril inhalation: such as sprays, aerosols and the like; (5) eye drops; (6) skin patches. Drug delivery systems, such as liposomes, sustained-release technology, controlled-release technology and the like, may also be used, wherein oral administration and injection are preferred, and oral administration is more preferred.

Preferably, the frequency of use of the above-mentioned medicines varies according to the compound used or its pharmaceutical composition and the diseases to which it is applied. The pharmaceutical composition of the present invention is usually administered 1 to 6 times a day, and the optimal dosing frequency is 1 to 3 times a day.

Preferably, the packaging and storage of the above-mentioned medicines are similar to those of general western medicines. For example, medicines in solid dosage form can be directly packed into glass, plastic, paper, or metal bottles, and it is better to put desiccant and the like in the bottle to maintain the quality of medicines; medicines in liquid dosage form are generally packed into glass, plastic or metal bottles or hoses; medicines in aerosol dosage form are generally packed in pressure-resistant metal or plastic containers with components such as pressure reducing valves and the like.

After a series of experiments, it is proved that the heterocyclic sulfoximine compound, a racemate, an enantiomer, a pharmaceutically acceptable salt or a solvate thereof of the present invention, has the following beneficial effects:
(1) By measuring the biochemical IC₅₀ values of some compounds of the present invention to wild-type RET, the representative RET (V804M) of the RET mutant and the representative RET-CCDC6 of the RET fusion protein, it can be seen that the heterocyclic sulfoximine compounds of the present invention have strong inhibitory effects on RET, RET mutants and/or RET fusion proteins.
(2) By measuring the inhibition IC₅₀ values of some compounds of the present invention on Phospho-RET and Phospho-ERK in tumor cells LC-2/ad, it can be seen that the heterocyclic sulfoximine compounds of the present invention have strong inhibitory effects on Phospho-RET and Phospho-ERK in tumor cells.
(3) The heterocyclic sulfoximine compounds of the present invention can be used together with other anti-tumor drugs, including but not limited to traditional chemotherapy drugs, targeted therapy drugs or immunotherapy drugs, so as to have a synergistic or additive effect.
(4) The heterocyclic sulfoximine compounds of the present invention can be used together with other tumor therapy, such as surgery, radiotherapy, interventional therapy, and the like.

It can be seen that the heterocyclic sulfoximine compound, or a racemate, an enantiomer, a pharmaceutically acceptable salt or a solvate thereof of the present invention, can be used as medicines for effectively treating the diseases caused by abnormal activity of RET, RET mutanst and/or RET fusion proteins.

### Definition of terms

The following are definitions of terms involved in the present invention.

According to the common knowledge of a person skilled in the art, the chemical reaction needs to be carried out in a "solvent" in most cases, and the commonly used "solvents" for preparing the compounds of the present invention include, but not limited to, water, methanol, ethanol, isopropanol, n-propanol, n-butanol, isobutanol, tert-butanol, 2-methoxyethanol, 2,2,2-trifluoroethanol, dichloromethane, 1,2-dichloroethane, chloroform, tetrahydrofuran, methyltetrahydrofuran, dioxane, 1,2-dimethoxyethane, ethyl acetate, diethyl ether, methyl tert-butyl ether, hexane, cyclohexane, toluene, acetonitrile, dimethyl sulfoxide, dimethylformamide, dimethylacetamide, or a combination of two or more of these solvents.

In the preparation process of the compounds of the present invention, in some steps, the reactions need to be performed in the presence of a "base", and the base includes, but not limited to, an organic base, for example, MeNH₂, Me₂NH, Me₃N, EtNH₂, Et₂NH, Et₃N, *n*-PrNH₂, *n*-Pr₂NH, *n*-Pr₃N, *i*-PrNH₂, *i*-Pr₂NH, *i*-Pr₃N, *n*-BuNH₂, *n*-Bu₂NH, *n*-Bu₃N, *s*-BuNH₂, *s*-Bu₂NH, *s*-Bu₃N, *i*-BuNH₂, *i*-Bu₂NH, *i-*Bu₃N, *t*-BuNH2, *t*-Bu₂NH, *t*-Bu₃N, *i*-Pr₂NEt, 2-amino-2-(hydroxymethyl)propane-1,3-diol, cyclopropylamine, dicyclopropylamine, cyclobutylamine, dicyclobutylamine, cyclopentylamine, dicyclopentylamine, cyclohexylamine, dicyclohexylamine, pyridine, DBU, DABCO, tetramethylguanidine, pentamethylguanidine, tetraethylguanidine, pentaethylguanidine, morpholine, 1-methylmorpholine, piperidine, 1-methylpiperidine, 1-ethylpiperidine, piperazine, 1-methylpiperazine, 1-ethylpiperazine, 1,4-dimethylpiperazine, 1,4-diethylpiperazine, pyrrolidine, 1-methylpyrrolidine, 1-ethylpyrrolidine, MeONa, MeOK, MeOLi, EtOLi, EtONa, EtOK, n-PrOLi, *n-*PrONa, *n*-PrOK, *i*-PrOLi, *i*-PrONa, *i*-PrOK, *n*-BuOLi, *n*-BuONa, *n*-BuOK, *i*-BuOLi, *i*-BuONa, *i-*BuOK, s-BuOLi, s-BuONa, s-BuOK, *t*-BuOLi, *t*-BuONa, *t*-BuOK, *n*-BuLi, s-BuLi, *t*-BuLi, NaN(SiMe₃)₂, LiN(SiMe₃)₂, KN(SiMe₃)₂ and the like. The base also includes, but not limited to, an inorganic base, for example, ammonia, liquor ammonia, LiOH, NaOH, KOH, RbOH, CsOH, Cs₂CO₃, Rb₂CO₃, Li₂CO₃, Na₂CO₃, K₂CO₃, NaHCO₃, KHCO₃, LiF, NaF, KF, RbF, CsF, K₃PO₃, K₂HPO₄, KH₂PO₄, Na₃PO₃, Na₂HPO₄, NaH₂PO₄, Li₃PO₃, Li₂HPO₄, LiH₂PO₄, NaH, LiH, KH, RbH, CsH, CaO, Ca(OH)₂, Ca₂CO₃, Ba(OH)₂, MgO, Mg(OH)₂, Mg₂CO₃ and the like, or a combination of two or more of the above-mentioned bases.

In some steps of the preparation of the compounds of the present invention, "Pd catalyst" is required for use, and the "Pd catalyst" includes, but not limited to, one or a combination of several of Pd/C, Pd(PPh₃)₄, Pd₂(dba)₃, PdCl₂, Pd(OAc)₂, Pd(O₂CCF₃)₂, PdCl₂(dppf), PdCl₂(dppp), Pd(PPh₃)₂Cl₂, Pd(PhCN)₂Cl₂, Pd(OH)₂, RuPhos Pd G2 (CAS#: 1375325-68-0), RuPhos Pd G3 (CAS#: 1445085-77-7), RuPhos Pd G4 (CAS#: 1599466-85-9) and the like.

In some cases, the chemical reaction needs to be performed in the presence of a "coupling reagent", and the commonly used coupling reagent for preparing the compounds of the present invention includes, but not limited to, one or more combinations of DCC, EDC, HATU, TBTU, PyBOP, HCTU, BOP, T3P, DIC, HOBt, HOAt, CDI, DEPBT, COMU and the like.

The reactions for preparing the compounds of the present invention are usually performed at room temperature, but sometimes it needs to be lowered to -78°C or heated to 200°C; the reactions are usually performed under the conditions of aforementioned solvents, temperature and conventional stirring, but sometimes need to be performed in a microwave oven; when the bases, reagents, and catalysts used are sensitive to water or oxygen, the reactions need to be performed under anhydrous and oxygen-free conditions. In this case, protic solvents cannot be used.

"Substituted" as used herein means that any group is mono- or polysubstituted with a specified substituent to the extent that such mono- or polysubstitution (including multiple substitutions at the same position) is chemically permissible, each substituent may be located at any available position on the group and can be connected through any available atom on the substituent. "Any available position" refers to any position on the group that is chemically accessible by methods known in the art or taught herein and that does not create an unduly labile molecule. When there are two or more substituents on any group, each substituent is defined independently from any other substituent, and thus the substituents may be the same or different.

At various portions in this specification, the substituents of the compounds of the present invention are disclosed as the forms of groups or ranges. This specifically means that the present invention includes each member of such groups and ranges or each individual subcombination of members. For example, the term "C₁₋₄ alkyl" specifically means that methyl, ethyl, C₃ alkyl and C₄ alkyl are disclosed individually.

"Compounds of the present invention" (unless specifically indicated otherwise) as used herein refer to compounds of formula (I), all pure and mixed stereoisomers, geometric isomers, tautomers, racemates, enantiomers, diastereomers, N-oxides, S-oxides, solvates, metabolites, prodrugs and isotopically-labeled compounds, and any pharmaceutically acceptable salts thereof, and also include the amorphous form, one or more crystal forms (polymorph), or a mixture of two or more different crystal forms in which the above-mentioned compounds may exist. Isotopes used for isotopic labelling include, but not limited to, ²H, ³H, ¹³C, ¹⁴C, ¹⁵N, ¹⁸O and the like.

"Solvate" refers to a stable substance formed by the compound of the present invention and a solvent commonly used in chemistry through covalent bonds, hydrogen bonds, ionic bonds, van der Waals forces, complexation, inclusion and the like. The solvent can be: methanol, ethanol, propanol, butanol, ethylene glycol, propylene glycol, polyethylene glycol, acetone, acetonitrile, diethyl ether, methyl tert-butyl ether, dimethyl sulfoxide, dimethyl formamide, dimethyl acetamide and the like.

"Hydrate" refers to a solvate in which the solvent is water.

"Pharmaceutically acceptable" means that the compound or composition must be chemically, pharmacologically and/or toxicologically compatible with the other ingredients that make up the formulation and/or the mammal to be treated.

"Prodrug" refers to that the compound of the present invention is converted into another compound by chemical synthesis or physical methods, and after the another compound is administered to a mammal, the another compound is converted into the compound of the present invention in the animal body. The use of a "prodrug" approach is usually to overcome the poor or suboptimal physicochemical properties or druggability of the pharmaceutical molecule itself.

"racemates (racemic mixtures)", "enantiomers", "diasteromers", "*cis*/*trans* isomers(*E-*/*Z-*isomers)" or "other steroisomers"refer to different compounds, which have the same molecular formula and molecular weight, but are formed with different bonding methods and/or spatial arrangements between atoms. Such compounds are called isomers or stereoisomers. When these stereoisomers are mirror images of each other, that is, they look very similar, but cannot be completely superimposed, such as left-hand and right-hand, and these compounds are called enantiomers. The absolute configurations of enantiomers are usually designated by (R)- and (*S*)- or R- and *S*-. Specific rules for determining the absolute configuration of enantiomers are found in Chapter 4 of "Advanced Organic Chemistry," 4th edition (by J. March, John Wiley and Sons, New York, 1992). The (R)- and (*S*)-enantiomers have opposite rotational effects on polarized light, i.e., levorotatory and dextrorotatory. When the (R)- and (*S*)-enantiomers are mixed or present in a ratio of 1:1, the mixture has no rotational effect on polarized light, and the mixture is called a racemate. When there are two or more chiral centers in a compound molecule, there may be diastereomers, that is, among all chiral centers in the molecule, at least one has same absolute configuration, and at least one has different absolute configuration.

"Tautomers" as used herein refers to structural isomers with different energies that can cross a low energy barrier and thereby convert into each other. For example, proton tautomers include interconversion by proton transfer, such as enol-keto tautomers and imine-enamine tautomers, or tautomeric forms of heteroaryl groups containing ring atoms attached to the ring -NH- and ring=N-moieties, such as pyrazoles, imidazoles, benzimidazoles, triazoles and tetrazoles. Valence tautomers comprise an interconversion by recombination of some of the bonding electrons.

The tautomers, rotamers, cis/trans isomers and the like may exist in the compounds of the present invention, and these concepts can all be found in J. March's "Advanced Organic Chemistry," 4th edition and be understood. As long as these isomers have the same or similar effect of inhibiting the activity of RET, RET mutants and/or RET fusion proteins as the compounds of the present invention, these isomers are also encompassed by the present invention.

After the compounds of the present invention are administered to mammals (such as humans), according to common knowledge in the art, they may be metabolized into various metabolites by different enzymes in the animal body, as long as these metabolites have the same effect of inhibiting the activity of RET, RET mutants and/or RET fusion proteins as the compounds of the present invention, these metabolites are also encompassed by the present invention.

"Pharmaceutical composition" refers to a formulation prepared by mixing one or more of the compounds of the present invention, pharmaceutically acceptable salts or solvates or hydrates or prodrugs with other chemical components (for example, pharmaceutically acceptable carrier or diluent). The purpose of a pharmaceutical composition is to facilitate the process of administration to an animal. In the above-mentioned pharmaceutical composition, in addition to a pharmaceutically acceptable carrier, it can also include adjuvants commonly used in pharmaceutical formulations, such as: antibacterial agent, antifungal agent, antimicrobial agent, preservative agent, toner, solubilizers, thickeners, surfactants, complexing agents, proteins, amino acids, fats, carbohydrates, vitamins, minerals, trace elements, sweeteners, pigments, flavors or their combinations and the like.

"Pharmaceutically acceptable carrier" or "diluent" refers to the inactive ingredients in the pharmaceutical composition, including but not limited to: calcium carbonate, calcium phosphate, magnesium carbonate, silica gel, various sugars (e.g., lactose, mannitol and the like), starch, cyclodextrin, magnesium stearate, cellulose, acrylic polymer, methacrylic acid polymer, gel, water, polyethylene glycol, propylene glycol, ethylene glycol, castor oil, hydrogenated castor oil, polyethoxylated hydrogenated castor oil, sesame oil, corn oil, peanut oil and the like.

"Pharmaceutically acceptable salts" refers to a salt formed by chemical reaction of a compound of the present invention with an inorganic acid, organic acid, inorganic base, or organic base, and such salt retains the biological activity and effectiveness of the compound of the present invention. The inorganic or organic acids include, but not limited to: hydrochloric acid, hydrobromic acid, hydroiodic acid, sulfuric acid, nitric acid, carbonic acid, phosphoric acid, perchloric acid, acetic acid, citric acid, oxalic acid, lactic acid, malic acid, salicylic acid, tartaric acid, methanesulfonic acid, ethanesulfonic acid, benzenesulfonic acid, substituted benzenesulfonic acid (e.g., p-toluenesulfonic acid), camphorsulfonic acid, isonicotinic acid, oleic acid, tannic acid, pantothenic acid, ascorbic acid, butylated diacid, maleic acid, gentisic acid, fumaric acid, gluconic acid, uronic acid, glucaric acid or sucrose acid, formic acid, benzoic acid, glutamic acid, pamoic acid, sorbic acid and the like; the inorganic or organic bases include, but not limited to, sodium hydroxide, potassium hydroxide, lithium hydroxide, iron hydroxide, calcium hydroxide, barium hydroxide, aluminum hydroxide, magnesium hydroxide, zinc hydroxide, ammonia water, organic hydroxide quaternary ammonium salt, sodium carbonate, potassium carbonate, lithium carbonate, calcium carbonate, barium carbonate, magnesium carbonate, carbonated organic quaternary ammonium salt, sodium bicarbonate, potassium bicarbonate, lithium bicarbonate, calcium bicarbonate, barium bicarbonate, magnesium bicarbonate, hydrogen carbonate organic quaternary ammonium salt and the like.

"Alkyl" refers to a saturated hydrocarbon group with straight or branched chain, having the specified number of carbon atoms, e.g., C₁₋₁₂ alkyl refers to a straight or branched chain group containing at least one and at most twelve carbon atoms. C₀ alkyl represents a covalent single bond. The alkyl group in the present invention includes, but not limited to: methyl, ethyl, propyl, butyl, isopropyl, neopentyl, 2-methyl-1-hexyl and the like. The alkyl group in the present invention sometimes also refers to "alkylene", and the alkylene group refers to the group formed by the loss of one hydrogen atom of the alkyl group. One or all hydrogen atoms in an alkyl or alkylene group can be optionally substituted by the following groups: cycloalkyl, aryl, heteroaryl, heteroalicyclyl, halogen, amino, hydroxy, cyano, nitro, carboxyl, mercapto, oxo, alkoxy, aryloxy, alkyl mercapto, aryl mercapto, carbonyl, thiocarbonyl, C-amide, *N*-amide, O-aminocarbonyloxy, *N-* aminocarbonyloxy, *O-*thioaminocarbonyloxy, *N*-thioaminocarbonyloxy, C-ester, O-ester and -NR^{a}R^{b}, wherein R^{a} and R^{b} are respectively selected from: hydrogen, alkyl, cycloalkyl, aryl, acetyl, carbonyl, sulfonyl, trifluoromethanesulfonyl and the like, and R^{a} and R^{b} may form a 5- or 6-membered heteroalicycle together with the nitrogen atom.

"Cycloalkyl" or "cycloalkane" refers to a mono-, bi- or polycyclic hydrocarbon group with the specified number of carbon atoms, which can be combined in the form of a "fused ring" (two or more rings share two adjacent carbon atoms), "spiro" (two or more rings share one carbon atom), or "bridged ring" (two or more rings share two or more nonadjacent carbon atoms) as bicyclic or polycyclic. For example, C₁₋₁₂ cycloalkyl refers to a mono-, bi- or polycyclic hydrocarbon group containing at least one and at most twelve carbon atoms. C₀ cycloalkyl represents a covalent single bond. Cycloalkyl groups can contain double or triple bonds, but do not have a fully conjugated π electron system. The cycloalkyl group in the present invention sometimes also refers to a cycloalkylene group, i.e., a group formed by the loss of one hydrogen atom of the cycloalkyl group. The cycloalkyl groups of the present invention include, but not limited to: cyclopropyl, cyclobutyl, cyclohexyl, cyclopentenyl, cycloheptatrienyl, adamantane and the like (for example, Table A):

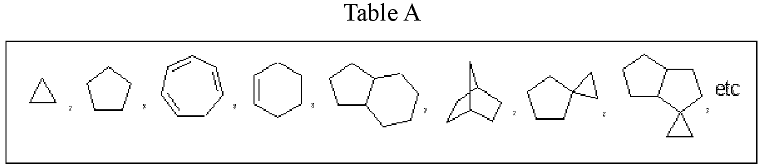

One or all of the hydrogen atoms in a cycloalkyl or cycloalkane can be substituted by the following groups: alkyl, aryl, heteroaryl, the heteroalicyclyl, halogen, amino, hydroxy, cyano, nitro, carboxyl, mercapto, oxo, alkoxy, aryloxy, alkyl mercapto, arylmercapto, carbonyl, thiocarbonyl, *C*-amide, *N-*amide, *O*-aminocarbonyloxy, *N*-aminocarbonyloxy, *O*-thioaminocarbonyloxy, *N-*thioaminocarbonyloxy, *C*-ester, *O*-ester and -NR^{a}R^{b}, wherein, R^{a} and R^{b} are respectively selected from: hydrogen, alkyl, cycloalkyl, aryl, acetyl, carbonyl, sulfonyl, trifluoromethanesulfonyl and the like, and R^{a} and R^{b} can form a 5- or 6-membered heteroalicycle together with the nitrogen atom.

"Heteroalicyclyl (heterocycloalkyl)" or "heteroalicycle" or "heterocycloalkane" refers to a monocyclic, bicyclic, or polycyclic ring system composed of 3 to 18 non-hydrogen ring atoms, wherein at least one ring atom is a heteroatom selected from O, N, S or P and the remaining ring atoms are carbon atoms, e.g. C₈ heteroalicyclyl refers to a monocyclic, bicyclic, or polycyclic ring group composed of 8 non-hydrogen ring atoms, wherein at least one ring atom is selected from O, N, S or P. C₈ here does not mean 8 carbon atoms, but 8 ring atoms consisting of carbon atoms, O, N, S or P. In addition to the single bond, this ring can also contain double bonds or triple bonds, but these double bonds or triple bonds do not constitute all conjugated aromatic structures. These monocyclic, bicyclic or polycyclic ring systems may exist in the form of fused, bridged or spiro rings. The heteroalicyclyl in the present invention sometimes also refers to a heteroalicyclylene group, that is, a group formed by the loss of one hydrogen atom of heteroalicyclyl. The heteroalicyclyl or the heteroalicycle in the present invention includes, but not limited to: piperidine, morpholine, piperazine, pyrrolidine, indoline, tetrahydropyridine, tetrahydrofuran, tropine and the like (for example, Table B):

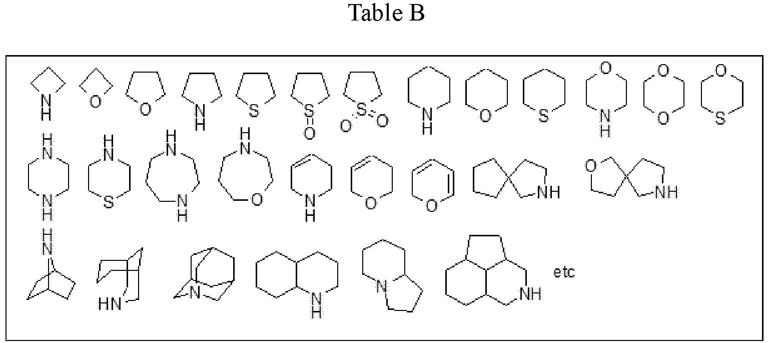

One or all of the hydrogen atoms in a heteroalicyclyl or a heteroalicycle can be substituted by the following groups: alkyl, cycloalkyl, aryl, heteroaryl, heteroalicycle, halogen, amino, hydroxy, cyano, nitro, carboxyl, mercapto, oxo, alkoxy, aryloxy, alkyl mercapto, arylmercapto, carbonyl, thiocarbonyl, *C*-amide, *N*-amide, *O*-aminocarbonyloxy, *N*-aminocarbonyloxy, *O*-thioaminocarbonyloxy, *N-*thioaminocarbonyloxy, *C*-ester, *O*-ester and -NR^{a}R^{b}, wherein, R^{a} and R^{b} are respectively selected from: hydrogen, alkyl, cycloalkyl, aryl, acetyl, carbonyl, sulfonyl group, trifluoromethanesulfonyl and the like, and R^{a} and R^{b} can form a 5- or 6-membered heteroalicycle together with the nitrogen atom.

"Alkenyl" refers to a straight or branched chain hydrocarbon group containing at least two carbon atoms and at least one double bond, e.g., C₂₋₁₂ alkenyl refers to a straight or branched chain unsaturated group with at least one double bond, containing at least two and at most twelve carbon atoms. The alkenyl group in the present invention includes, but not limited to, vinyl group, 2-propenyl group, 1-pentenyl group and the like.

"Alkynyl" refers to a straight or branched chain hydrocarbon group containing at least two carbon atoms and at least one triple bond, e.g., C₂₋₁₂ alkynyl refers to a straight or branched chain unsaturated group with at least one triple bond, containing at least two and at most twelve carbon atoms. The alkynyl in the present invention includes, but not limited to: ethynyl, propynyl, pentynyl, and the like.

"Halogen" refers to fluorine, chlorine, bromine, or iodine.

"Alkoxy" refers to that an alkyl group having the specified number of carbon atoms is attached to other groups through an oxygen atom. The alkoxy in the present invention includes, but not limited to: methoxy, ethoxy, propoxy, butoxy, cyclopentyloxy, cyclohexyloxy, isopropoxy, neopentyloxy, 2-methyl-1-hexyloxy and the like.

"Cycloalkoxy" refers to that a cycloalkyl group having the specified number of carbon atoms is attached to other groups through an oxygen atom. The cycloalkoxy in the present invention includes, but not limited to, cyclopropanoxy, cyclobutanoxy, cyclohexaneoxy and the like.

"Heteroalicyclyloxy" refers to that a heteroalicyclyl is attached to other groups through an oxygen atom. The heteroalicyclyloxy in the present invention includes, but not limited to, piperidin-4-yloxy, oxetan-3-yloxy and the like.

"Aryl" refers to a monocyclic, bicyclic or polycyclic group composed of the specified number of carbon atoms, wherein at least one ring has a fully conjugated π electron system and conforms to the N+2 rule, i.e., aromatic, but the entire group need not be all conjugated. For example, C₆ aryl refers to phenyl. Aryl group can also occur in the form of arylene groups, i.e., the aryl struction has two or more points of attachment to other groups. Aryl groups in the present invention include, but not limited to: phenyl, naphthyl, indenyl, dihydroindenyl, tetrahydronaphthyl and the like. One or all of the hydrogen atoms in an aryl can be substituted by the following groups: alkyl, cycloalkyl, heteroaryl, heteroalicycle, halogen, amino, hydroxy, cyano, nitro, carboxyl, mercapto, oxo, alkoxy, aryloxy, alkyl mercapto, arylmercapto, carbonyl, thiocarbonyl, C-amide, N-amide, O-aminocarbonyloxy, N-aminocarbonyloxy, O-thioaminocarbonyloxy, N-thioaminocarbonyloxy, C-ester, O-ester and -NR^{a}R^{b}, wherein, R^{a} and R^{b} are respectively selected from: hydrogen, alkyl, cycloalkyl, aryl, acetyl, carbonyl, sulfonyl group, trifluoromethanesulfonyl and the like, and R^{a} and R^{b} can form a 5- or 6-membered heteroalicycle together with the nitrogen atom.

"Heteroaryl" refers to a monocyclic, bicyclic or polycyclic group composed of the specified number of non-hydrogen ring atoms, wherein at least one ring atom is a heteroatom selected from O, N, S or P and the remaining ring atoms are carbon atoms, and at least one of the rings has a fully conjugated π electron system and conforms to the N+2 rule, i.e., aromatic, but the entire group need not be all conjugated, for example, C₅ heteroaryl refers to an aromatic ring group composed of 5 non-hydrogen ring atoms, wherein at least one ring atom is selected from O, N, S or P, and the remaining ring atoms are carbon atoms. Heteroaryl groups can also occur in the form of heteroarylene groups, i.e., the heteroaryl structure has two or more points of attachment to other groups. Heteroaryl groups in the present invention include, but not limited to: pyridinyl, pyridonyl, tetrahydropyridonyl, pyrimidinyl, pyrazinyl, pyridazinyl, imidazolyl, thiazolyl, thiophenyl, furanyl, indolyl, azaindolyl, benzimidazolyl, indolinyl, indolinonyl, quininyl and the like (for example, Table C):

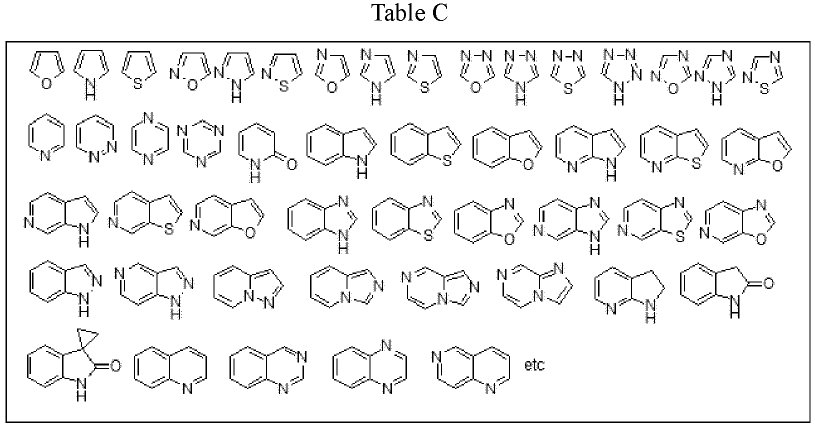

One or all of the hydrogen atoms in a heteroaryl can be substituted by the following groups: alkyl, cycloalkyl, aryl, heteroalicycle, halogen, amino, hydroxy, cyano, nitro, carboxyl, mercapto, oxo, alkoxy, aryloxy, alkyl mercapto, arylmercapto, carbonyl, thiocarbonyl, *C*-amide, *N*-amide, *O-*aminocarbonyloxy, *N*-aminocarbonyloxy, *O*-thioaminocarbonyloxy, *N*-thioaminocarbonyloxy, *C*-ester, *O*-ester and -NR^{a}R^{b}, wherein, R^{a} and R^{b} are respectively selected from: hydrogen, alkyl, cycloalkyl, aryl, acetyl, carbonyl, sulfonyl group, trifluoromethanesulfonyl and the like, and R^{a} and R^{b} can form a 5- or 6-membered heteroalicycle together with the nitrogen atom.

"Nitrogen-containing heteroaryl" refers to a heteroaryl group, but the heteroaryl group contains at least one nitrogen atom. The nitrogen-containing heteroaryl groups in the present invention include, but not limited to, pyridyl, quinolyl, pyrazinyl, pyridazinyl and the like.

"Aryloxy" refers to an aryl group attached to other groups through an oxygen atom. The aryloxy groups in the present invention include, but not limited to: phenoxy, naphthoxy and the like.

"Heteroaryloxy" refers to a heteroaryl group attached to other groups through an oxygen atom. The heteroaryloxy groups in the present invention include, but not limited to: 4-pyridyloxy, 2-thienyloxy and the like.

"N-oxide" refers to that the N atom is connected with the O atom through a double bond to form an N=O or N⁺-O⁻ structure in the molecule.

"Amino" refers to H₂N- or H₂N- in which a hydrogen atom is substituted, i.e., R^{a}HN- and R^{a}R^{b}N-.

"Oxo" or "oxy" refers to =O or -O-, i.e., the oxygen atom is connected to carbon or heteroatoms such as N, S, P and the like through a double bond or a single bond. The examples of substitution with oxy include, but not limited to, the substances shown in Table D:

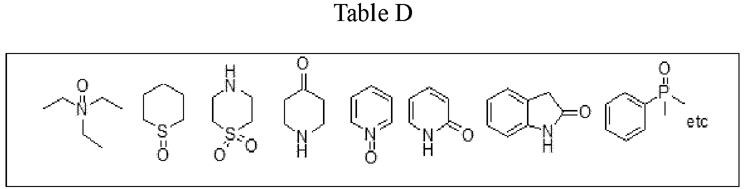

"Hydroxy" refers to -OH.
"Nitro" refers to -NO₂.
"Carboxyl" refers to -CO₂H.
"Mercapto" refers to -SH.
"Alkyl mercapto" refers to alkyl-S-.
"Arylmercapto" refers to aryl-S-.
"Carbonyl" refers to -C(=O)-.
"Thiocarbonyl" refers to -C(=S)-.
"*C*-amide" refers to -C(=O)NR^{a}R^{b}.
"*N*-amide" refers to C(=O)NR^{a}-.
"*O*-aminocarbonyloxy" refers to -O-C(=O)NR^{a}R^{b}.
"*N*-aminocarbonyloxy" refers to O-C(=O)NR^{a}-.
"*O*-thioaminocarbonyloxy" refers to -O-C(=S)NR^{a}R^{b}.
"*N*-thioaminocarbonyloxy" refers to O-C(=S)NR^{a}-.
"*C*-ester" refers to -C(=O)OR^{a}.
"*N*-ester" refers to C(=O)O-.
"Acetyl" refers to CH₃C(=O)-.
"Sulfonyl group" refers to -SO₂R^{a}.
"Trifluoromethanesulfonyl" refers to CF₃SO₂-.
"Ph" refers to phenyl.

### Specific Modes for Carrying Out the Embodiments

The present invention will be described in further detail below in conjunction with specific Examples, but not intended to limit the scope of the present invention.

The English abbreviations and corresponding Chinese meanings appearing in the Examples are listed below. If an abbreviation not listed here appears in the Examples, it represents the generally accepted meaning.
h: hour(s)
rt: room temperature
°C: degree Celsius
TLC: Thin Layer Chromatography
HPLC: High Performance Liquid Chromatography
LC-MS: Liquid Chromatography-Mass Spectrometry
g: gram(s)
mg: milligram(s)
mmol: millimole(s)
nM: nanomolar (concentration unit)
µM: micromolar (concentration unit)
M: mole (concentration unit)
mL: milliliter(s)
(M+H)⁺: Molecular ion peak in mass spectrum
m/z: mass-to-charge ratio
NMR: Nuclear Magnetic Resonance Spectroscopy
δ: chemical shift
DMSO-*d₆*: hexadeuterated dimethyl sulfoxide
CDCl₃: deuterated chloroform
CD₃OD: deuterated methanol
DMSO: dimethyl sulfoxide
DMF: *N,N*-dimethylformamide
DMA: *N,N-*dimethylacetamide
MeOH: methanol
EtOAc: ethyl acetate
DCM: dichloromethane
Dioxane: dioxane
DCE: 1,2-dichloroethane
HCl: hydrogen chloride or hydrochloric acid
TFA: trifluoroacetic acid
AcOH: acetic acid
KOAc: potassium acetate
NaHCO₃: sodium bicarbonate
Na₂CO₃: sodium carbonate
K₂CO₃: potassium carbonate
Cs₂CO₃: cesium carbonate
K₃PO₄: potassium phosphate
Pd/C: palladium on carbon
DIPEA: diisopropylethyl amine
BBr₃: boron tribromide
NaIO₄: sodium periodate
PhI(OAc)₂: (diacetoxyiodo)benzene
MgO: magnesium oxide
Na₂SO₄: sodium sulfate
Na₂S₂O₃: sodium thiosulfate
NH₃·H₂O: ammonia water
BocNH₂: tert-butyl carbamate
NaB(OAc)₃H: sodium triacetoxyborohydride
NaBH₃CN: sodium cyanoborohydride
Rh₂(OAc)₄: rhodium(II) acetate dimer
Pd(PPh₃)₄: Tetra(triphenylphosphine)palladium(0)
Pd₂(dba)₃:tris(dibenzylideneacetone)dipalladium(0)
Pd(dppf)Cl₂: [1,1'-bis(diphenylphosphino)ferrocene]dichloropalladium(II)
Xantphos: 4,5-bis(diphenylphosphine)-9,9-dimethylxanthene
XPhos: 2-dicyclohexylphosphino-2',4',6'-triisopropylbiphenyl
PhN(Tf)₂: *N,N'*-bis(trifluoromethylsulfonyl)aniline
Pin₂B₂ or B₂Pin₂:bis(pinacol) diboron
4Å MS: molecular sieve with a pore size of 4Å

General experimental conditions:
¹H NMR and ¹³C NMR spectra were obtained by Varian 300 or 400 MHz or Bruker 300 or 400 MHz instrument (with deuterated dimethyl sulfoxide, deuterated chloroform, deuterated methanol and the like as solvents, with or without tetramethylsilane as an internal standard). Mass spectra were obtained by liquid chromatography-mass spectrometry combined instrument (Waters or Agilent in the United States). High performance liquid chromatography was performed using a high performance liquid chromatography instrument of Waters company or Agilent company, unless otherwise specified.

Starting materials, reagents, and solvents were purchased from the following suppliers: Sigma-Aldrich, Milwaukee, WI, USA; Acros, Morris Plains, NJ, USA; Frontier Scientific, Logan, Utah, USA; Alfa Aesar, Ward Hill, MA, USA; Shanghai Aladdin Bio-Chem Technology Co., Ltd, Shanghai, China; Shanghai Macklin Bio-Chemical Co., Ltd., Shanghai, China; WuXi LabNetwork, Shanghai, China, and the like, or were synthesized by methods reported in the literatures. Unless otherwise specified, the solvent is generally not dried, and the products of the suppliers are directly used or are used after being dried through molecular sieves.

### Intermediate 1: 4-bromo-6-hydroxypyrazolo[1,5-a]pyridin-3-carbonitrile (Int-1):

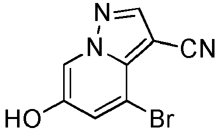

Int-1 (CAS#: 2068065-05-2) was prepared according to the method reported in the literature (WO2019075092).

### Intermediate 2: 4-bromo-6-(2-hydroxy-2-methylpropoxy)pyrazolo[1,5-a]pyridin-3-carbonitrile (Int-2):

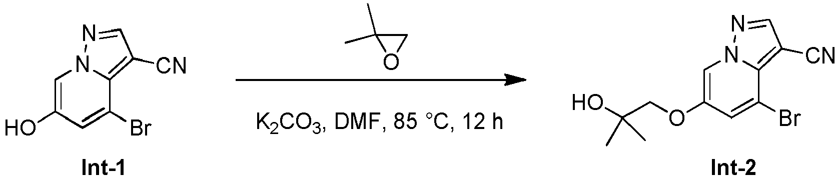

2,2-Dimethyloxirane (3.03 g, 3.73 mL, 42.0 mmol) was added to a mixture of 4-bromo-6-hydroxypyrazolo[1,5-a]pyridin-3-carbonitrile (Int-1, 10.0 g, 42.0 mmol) and K₂CO₃ (17.4 g, 126 mmol) in DMF (50 mL). The resulting mixture was stirred at 85°C for 12 h, and TLC showed that the reaction was completed. After cooling to room temperature, the reaction mixture was poured into water (400 mL) and stirred for 1 h. The product was collected by filtration and dried to give 4-bromo-6-(2-hydroxy-2-methylpropoxy)pyrazolo[1,5-a]pyridin-3-carbonitrile (Int-2, 11.0 g, yield: 84%) as a brown solid.

### Intermediate 3: 4-(6-fluoropyridin-3-yl)-6-(2-hydroxy-2-methylpropoxy)pyrazolo[1,5-a]pyridin-3-carbonitrile (Int-3):

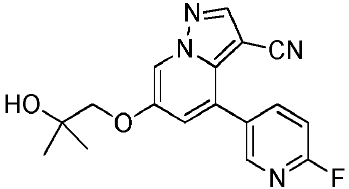

Int-3 (CAS#: 2222653-74-7) was prepared according to the method reported in the literature (WO2019075108).

### Intermediate 4: S,S-dimethyllsulfoximine (Int-4):

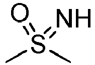

Int-4 (CAS#: 1520-31-6) was purchased from WuXi LabNetwork.

### Intermediate 5: (S)-(+)-S-methyl-S-phenylsulfonimide (Int-5):

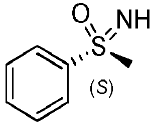

Int-5 (CAS#: 33903-50-3) was prepared according to the method reported in the literature (M. R. Yadav et al, Chem. Eur. J. 2012, 18, 5541-5545).

### Intermediate 6: (R)-(-)-S-methyl-S-phenylsulfonimide (Int-6):

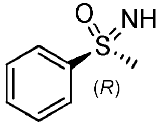

Int-6 (CAS#: 60933-65-5) was prepared according to the method reported in the literature (H. Zhao et al, Mol. Catalysis 2018, 455, 210-213).

### Intermediate 7: tert-butyl (1-oxo-4-(5-(4,4,5,5-tetramethyl-1,3,2-dioxaborolane-2-yl)pyridin-2-yl)thiomorpholine-1-imino)carboxylate (Int-7):

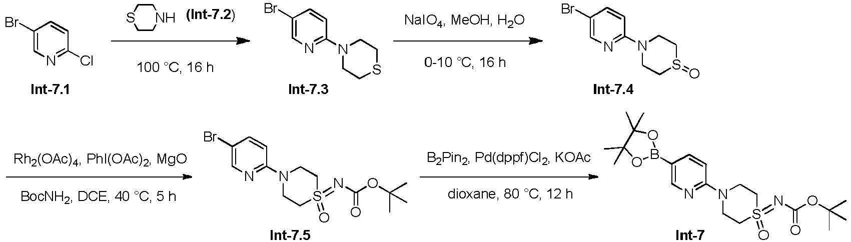

### Step 1: 4-(5-bromopyridin-2-yl)thiomorpholine (Int-7.3):

A mixture of 5-bromo-2-chloropyridine (Int-7.1, 38.0 g, 197 mmol) and thiomorpholine (Int-7.2, 102.0 g, 93.5 mL, 987 mmol) was stirred at 100°C for 16 h, and TLC showed that the reaction was completed. After cooling to room temperature, saturated NaHCO₃ solution (400 mL) was added to the reaction mixture, the resulting mixture was extracted with ethyl acetate (3 × 350 mL). The organic layers were combined, washed with water (500 mL) and saturated brine (500 mL) in turn, dried with anhydrous Na₂SO₄, filtered, and the filtrate was concentrated under reduced pressure to obtain a residue, which was purified by silica gel column chromatography (eluent: petroleum ether : ethyl acetate = 50:1 to 5:1) to obtain 4-(5-bromopyridin-2-yl)thiomorpholine (Int-7.3, 30.0 g, yield: 58%) as a colorless oily liquid. Analytical data: ¹H-NMR (400 MHz, CDCl₃):δ = 8.18 (d, *J=* 2.4 Hz, 1 H), 7.51-7.54 (m, 1 H), 6.51 (d, *J=* 9.2 Hz, 3 H), 3.90-3.92 (m, 4 H), 2.64- 2.66 (m, 4 H). Mass spectrum (ESI) *m*/*z*:259 (M+H, ⁷⁹Br)⁺, 261 (M+H, ⁸¹Br)⁺.

### Step 2: 4-(5-bromopyridin-2-yl)thiomorpholine-1-oxide (Int-7.4):

NaI0₄ (27.2 g, 127 mmol) was added to a mixture of 4-(5-bromopyridin-2-yl)thiomorpholine (Int-7.3, 30.0 g, 116 mmol) in MeOH (300 mL) and water (300 mL) at 0°C. The resulting mixture was stirred at 0-10°C for 16 h, and TLC showed that the reaction was completed. The reaction mixture was filtered, the aqueous layer was extracted with DCM (3×200 mL), the organic layers were combined and washed successively with saturated Na₂S₂O₃ solution (200 mL) and saturated brine (200 mL), dried with anhydrous Na₂SO₄, filtered, and the filtrate was concentrated under reduced pressure to obtain 4-(5-bromopyridin-2-yl)thiomorpholine-1-oxide (Int-7.4, 25.8 g, yield: 74.5%) as a white solid. Analytical data: ¹H-NMR (400 MHz, CDCl₃):δ = 8.23 (d, *J* = 2.4 Hz, 1 H), 7.60 (dd, *J* = 2.4, 8.8 Hz, 1 H), 6.65 (d, *J=* 8.8 Hz, 1 H), 4.08-4.19 (m, 4 H), 2.74-2.80 (m, 4 H). Mass spectrum (ESI) *m*/*z*:275.1 (M+H, ⁷⁹Br)⁺, 277.1 (M+H, ⁸¹Br)⁺.

### Step 3: tert-butyl (4-(5-bromopyridin-2-yl)-1-oxothiomorpholine-1-imino)carboxylate (Int-7.5):

Under nitrogen gas, Rh₂(OAc)₄ (1.20 g, 2.73 mmol) was added to a mixture of tert-butyl 4-(5-bromopyridin-2-yl)thiomorpholine-1-oxide (Int-7.4, 15.0 g, 54.5 mmol), amino carboxylate (12.8 g, 109 mmol), magnesium oxide (8.79 g, 218 mmol), and PhI(OAc)₂ (26.3 g, 81.8 mmol) in 1,2-dichloroethane (40 mL). The resulting mixture was stirred at 40°C for 5 h, and TLC showed that the reaction was completed. After cooling to room temperature, the reaction mixture was filtered, the filtrate was diluted with water (100 ml), and extracted with DCM (2 × 100 mL). The organic layers were combined, dried with anhydrous Na₂SO₄, filtered, and the filtrate was concentrated under reduced pressure to obtain a residue, which was purified by silica gel column chromatography (eluent: petroleum ether: ethyl acetate = 10:1 to 1:1) to obtain tert-butyl (4-(5-bromopyridin-2-yl)-1-oxothiomorpholine-1-imino)carboxylate (Int-7.5, 11.5 g, yield:54%), as a colorless oily liquid. Analytical data: ¹H-NMR (400 MHz, CDCl₃): δ = 8.25 (d, *J=* 2.0 Hz, 1 H), 7.65 (dd, *J* = 2.0, 8.8 Hz, 1 H), 6.67 (d, *J=* 8.8 Hz, 1 H), 4.18-4.35 (m, 2 H), 4.00 (ddd, *J=* 2.0, 8.4, 14.4 Hz, 2 H), 3.63 (dd, *J=* 6.7, 14.4 Hz, 2 H), 3.23-3.35 (m, 2 H), 1.50 (s, 9 H). Mass spectrum (ESI) *m*/*z:* 289.9 (M+H-100, ⁷⁹Br)⁺, 291.9 (M+H-100, ⁸¹Br)⁺.

### Step 4: tert-butyl (1-oxo-4-(5-(4,4,5,5-tetramethyl-1,3,2-dioxaborolane-2-yl)pyridin-2-yl)thiomorpholine-1-imino)carboxylate (Int-7):

Under nitrogen gas, Pd(dppf)Cl₂ (562 mg, 0.769 mmol) was added to a mixture of tert-butyl (4-(5-bromopyridin-2-yl)-1-oxothiomorpholine-1-imino)carboxylate (Int-7.5, 3.00 g, 7.69 mmol), potassium acetate (2.26 g, 23.1 mmol) and bis(pinacol) diboron (Pin₂B₂, 5.86 g, 23.1 mmol) in dioxane (30 mL). The resulting mixture was stirred at 80°C for 12 h, and LC-MS showed that the reaction was completed. After cooling to room temperature, the reaction mixture was diluted with ethyl acetate (30 mL) and filtered, and the filtrate was concentrated under reduced pressure to obtain crude product, which was washed with petroleum ether (80 mL) to obtain tert-butyl (1-oxo-4-(5-(4,4,5,5-tetramethyl-1,3,2-dioxaborolane-2-yl)pyridin-2-yl)thiomorpholine-1-imino)carboxylate (Int-7, 3.2 g, crude product) as a dark brown solid. It was used in the next reaction without further purification. Mass spectrum (ESI) *m*/*z*: 438.4 (M+H)⁺.

### Intermediate 8: tert-butyl 4-(1-oxo-4-(5-(4,4,5,5-tetramethyl-1,3,2-dioxaborolane-2-yl)pyridin-2-yl)thiomorpholine-1-imino)piperidin-1-carboxylate (Int-8):

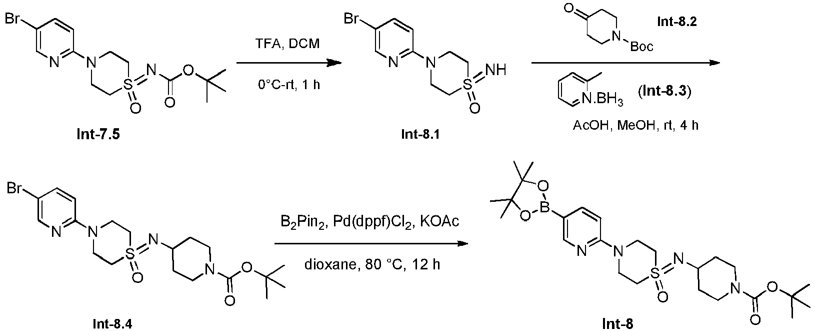

### Step 1: 4-(5-bromopyridin-2-yl)-1-iminothiomorpholine-1-oxide (Int-8.1):

At 0°C, trifluoroacetic acid (5 mL, 67.5 mmol) was added to a solution of tert-butyl (4-(5-bromopyridin-2-yl)-1-oxothiomorpholine-1-imino) carboxylate (Int-7.5, 1.50 g, 3.84 mmol) in DCM (10 mL). The resulting mixture was stirred at room temperature for 1 h, and TLC showed that the reaction was completed. The reaction mixture was concentrated under reduced pressure, and the residue was suspended in methyl tert-butyl ether (20 mL). The solid product 4-(5-bromopyridin-2-yl)-1-iminothiomorpholine-1-oxide (Int-8.1, 1.2 g, crude product) was collected by filtration as a yellow solid. The crude product was basified with saturated aqueous sodium carbonate to obtain the free base, which was used in the next reaction without further purification. Analytical data: ¹H-NMR (400 MHz, DMSO-*d*₆):δ = 8.24 (d, *J=* 2.4 Hz, 1 H), 7.80 (dd, *J=* 2.6, 9.0 Hz, 1 H), 7.04 (d, *J=* 9.2 Hz, 1 H), 4.47 (d, *J=* 15.4 Hz, 2 H), 3.65-3.76 (m, 2 H), 3.52-3.61 (m, 2 H), 3.40-3.49 (m, 2 H).

### Step 2: tert-butyl 4-(4-(5-bromopyridin-2-yl)-1-oxothiomorpholine-1-imino)piperidin-1-ylcarboxylate (Int-8.4):

2-Methylpyridin-borane complex (Int-8.3, 221 mg, 2.07 mmol) was added to a mixture of 4-(5-bromopyridin-2-yl)-1-iminothiomorpholine-1-oxide (Int-8.1, 0.50 g, 1.72 mmol), tert-butyl 4-oxopiperidin-1-carboxylate (Int-8.2, 1.37 g, 6.89 mmol) and AcOH (1 mL, 17.5 mmol) in MeOH (3 mL). The resulting reaction mixture was stirred at 25 °C for 4 h, and LC-MS showed that the reaction was completed. The reaction mixture was filtered, and the filtrate was concentrated under reduced pressure to obtain a residue, which was purified by silica gel column chromatography (eluent: petroleum ether: ethyl acetate = 30:1 to 1:1) to obtained tert-butyl 4-(4-(5-bromopyridin-2-yl)-1-oxothiomorpholine-1-imino)piperidin-1-ylcarboxylate (Int-8.4, 0.34 g, yield: 41.7%) as a yellow solid. Analytical data: Mass spectrum (ESI) *m*/*z*: 473.1 (M+H, ⁷⁹Br)⁺, 475.1 (M+H, ⁸¹Br)⁺.

### Step 3: tert-butyl 4-(1-oxo-4-(5-(4,4,5,5-tetramethyl-1,3,2-dioxaborolane-2-yl)pyridin-2-yl)thiomorpholine-1-imino)piperidin-1-ylcarboxylate (Int-8):

Under nitrogen gas, Pd(dppf)Cl₂ (52.6 mg, 0.0718 mmol) was added to a mixture of tert-butyl 4-(4-(5-bromopyridin-2-yl)-1-oxothiomorpholine-1-imino)piperidin-1-ylcarboxylate (Int-8.4, 340 mg, 0.718 mmol), potassium acetate (211 mg, 2.15 mmol) and bis(pinacol) diboron(Pin₂B₂, 547 mg, 2.15 mmol) in dioxane (5 mL). The resulting mixture was stirred at 80°C for 12 h, and LC-MS showed that the reaction was completed. The reaction mixture was cooled to room temperature and filtered. The filtrate was concentrated under reduced pressure and dried to obtain tert-butyl 4-(1-oxo-4-(5-(4,4,5,5-tetramethyl-1,3,2-dioxaborolane-2-yl)pyridin-2-yl)thiomorpholine-1-imino)piperidin-1-ylcarboxylate (Int-8, 360 mg, crude product, yield: 96%) as a brown solid. It was used in the next reaction without further purification. Analytical data: Mass spectrum (ESI) *m*/*z:* 521.3 (M+H)⁺.

### Intermediate 9: N-(dimethyloxo-λ⁴-sulfinyl)-4-piperidinamine (Int-9):

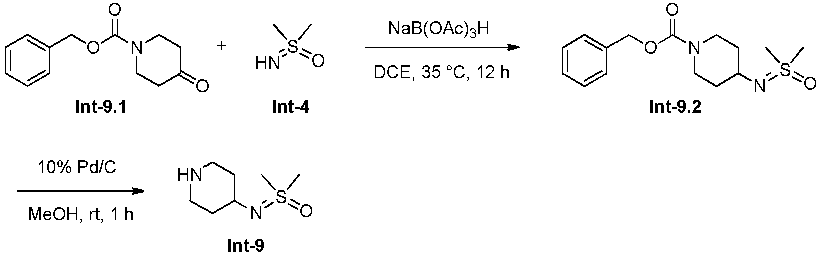

### Step 1: benzyl 4-[(dimethylloxo-λ⁴-sulfinyl)amino]piperidin-1-ylcarboxylate (Int-9.2):

NaB(OAc)₃H (45.5 g, 215 mmol) was added to a solution of *S*,*S*-dimethyllsulfoximine (Int-4, 5.0 g, 53.7 mmol) and benzyl 4-oxopiperidin-1-carboxylate (Int-9.1, 25.0 g, 107 mmol) in 1,2-dichloroethane (250 mL). The resulting mixture was stirred at 35°C for 12 h, and LC-MS showed that the reaction was completed. The reaction mixture was filtered, the filtrate was concentrated under reduced pressure, the residue was diluted with water, adjusted to about 8 of pH with saturated aqueous sodium bicarbonate solution, extracted with DCM (3x200 mL). The organic layers were combined, dried with anhydrous Na₂SO₄, filtered, and the filtrate was concentrated under reduced pressure to obtain a residue, which was purified by silica gel column chromatography (eluent: petroleum ether:ethyl acetate = 5:1 to 1:1) to obtain benzyl 4-[(dimethylloxo-λ⁴-sulfinyl)amino]piperidin-1-ylcarboxylate (Int-9.2, 5.3 g, yield: 32%), as a white solid. Analytical data: ¹H-NMR (400 MHz, CDCl₃):δ = 7.19-7.28 (m, 5 H), 5.04 (s, 2 H), 3.80-3.91 (m, 2 H), 3.30 (m, 1 H), 2.91-2.97 (m, 2 H), 2.94 (s, 6 H), 1.69-1.72 (m, 2 H), 1.43-1.46 (m, 2 H). Mass spectrum (ESI) *m*/*z:* 311.0 (M+H)⁺.

### Step 2: N-(dimethyloxo-λ⁴-sulfinyl)-4-piperidinamine (Int-9):

10% Pd/C (400 mg) was added to a solution of benzyl 4-[(dimethyloxo-λ⁴-sulfinyl)amino]piperidin-1-ylcarboxylate (Int-9.2, 2.0 g, 6.44 mmol) in MeOH (30 mL). The resulting mixture was hydrogenated at room temperature for 1 h, and TLC showed that the reaction was completed. The reaction mixture was filtered, and the filtrate was concentrated under reduced pressure to obtain *N*-(dimethyloxo-λ⁴-sulfinyl)-4-piperidinamine (Int-9, 1.10 g, yield: 96%), as a white solid. Analytical data: ¹H-NMR (400 MHz, CDCl₃):δ = 3.47 (br, m, 1 H), 3.81-3.95 (m, 2 H), 3.20 (m, 1 H), 3.01 (s, 6 H), 2.80-2.83 (m, 2 H), 1.90-1.93 (m, 2 H), 1.58-1.62 (m, 2 H).

### Intermediate 10: N-((S)-methyl-oxo-phenyl-λ⁴-sulfinyl)-4-piperidinamine (Int-10):

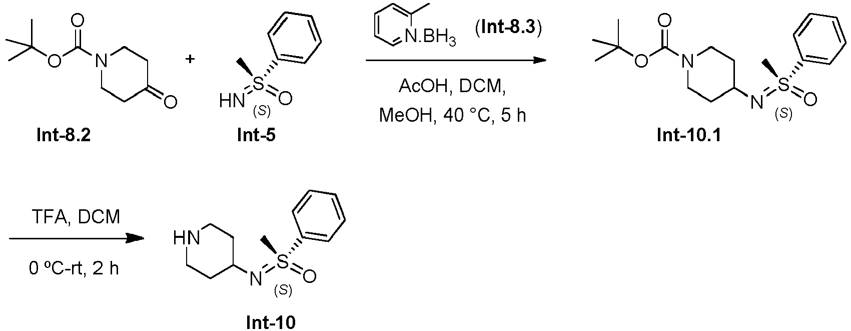

### Step 1: tert-butyl 4-(((S)-methyl-oxo-phenyl-λ⁴-sulfinyl)amino)piperidin-1-ylcarboxylate (Int-10.1):

The acetic acid (14 mL, 244 mmol) and 2-methylpyridin-borane complex (Int-8.3, 3.62 g, 33.8 mmol) were added to a solution of (*S*)-(+)-*S*-methyl-*S*-phenylsulfonimide (Int-5, 3.50 g, 22.6 mmol) and tert-butyl 4-oxopiperidin-1-ylcarboxylate (Int-8.2, 22.5 g, 112 mmol) in MeOH (50 mL) and DCM (25 mL). The resulting mixture was stirred at 40°C for 5 h, and LC-MS showed that the reaction was completed. The reaction mixture was diluted with water (50 mL) and extracted with DCM(3 × 50 mL). The organic layers were combined, dried with anhydrous Na₂SO₄, filtered, and the filtrate was concentrated under reduced pressure to obtain a residue, which was purified by silica gel column chromatography (eluent: petroleum ether:ethyl acetate = 10:1 to DCM:MeOH = 10:1) to obtain tert-butyl 4-(((*S*)-methyl-oxo-phenyl-λ⁴-sulfinyl)amino)piperidin-1-ylcarboxylate (Int-10.1, 1.7 g, yield: 22%). Analytical data: Mass spectrum (ESI) *m*/*z:* 339.4 (M+H)⁺.

### Step 2: N-((S)-methyl-oxo-phenyl-λ⁴-sulfinyl)-4-piperidinamine (Int-10):

At 0°C, trifluoroacetic acid (5 mL, 67.5 mmol) was added to a solution of tert-butyl 4-(((*S*)-methyl-oxo-phenyl-λ⁴-sulfinyl)amino)piperidin-1-ylcarboxylate (Int-10.1, 1.5 g, 4.43 mmol) in DCM (15 mL). The resulting mixture was stirred at room temperature for 2 h, and TLC showed that the reaction was completed. The reaction mixture was concentrated under reduced pressure to obtain a crude product (2.2 g), which was basified with saturated NaHCO₃ solution to give *N*-((*S*)-methyl-oxo-phenyl-λ⁴-sulfinyl)-4-piperidinamine (Int-10), as a yellow oily liquid, which was used in the next reaction without further purification. Analytical data: Mass spectrum (ESI) *m*/*z*:239 (M+H)⁺.

### Intermediate 11: N-((R)-methyl-oxo-phenyl-λ⁴-sulfinyl)-4-piperidinamine (Int-11):

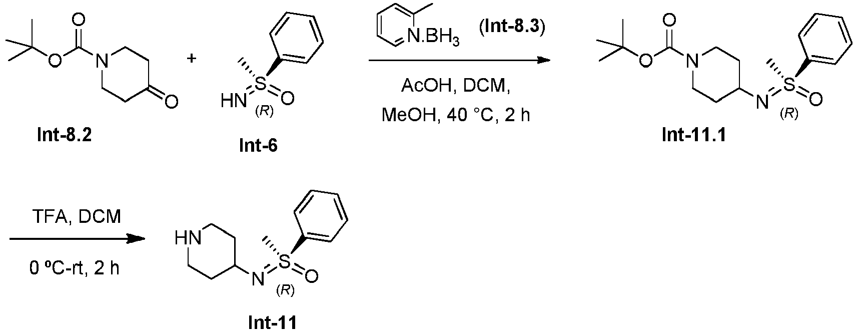

### Step 1: tert-butyl 4-(((R)-methyl-oxo-phenyl-λ⁴-sulfinyl)amino)piperidin-1-ylcarboxylate (Int-11.1):

The acetic acid (10.4 mL, 182 mmol) and 2-methylpyridin-borane complex (Int-8.3, 2.78 g, 26 mmol) were added to a solution of (*R*)-(-)-*S*-methyl-*S*-phenylsulfonimide (Int-6, 2.70 g, 17.3 mmol) and tert-butyl 4-oxopiperidin-1-carboxylate (Int-8.2, 17.3 g, 86.7 mmol) in MeOH (50 mL) and DCM (25 mL). The resulting mixture was stirred at 40°C for 2 h, and LC-MS showed that the reaction was completed. The reaction mixture was diluted with water (50 mL) and extracted with DCM (3 × 50 mL). The organic layers were combined, dried with anhydrous Na₂SO₄, filtered, and the filtrate was concentrated under reduced pressure to obtain a residue, which was purified by silica gel column chromatography (eluent: petroleum ether : ethyl acetate = 10:1 to DCM : MeOH = 10:1) to obtain tert-butyl 4-(((*R*)-methyl-oxo-phenyl-λ⁴-sulfinyl)amino)piperidin-1-ylcarboxylate (Int-11.1, 1.6 g, yield: 24%). Analytical data: ¹H-NMR (400 MHz, CDCl₃):δ = 7.91-7.93 (m, 2 H), 7.55-7.59 (m, 3 H), 4.74 (d, *J=* 8.0 Hz, 2 H), 3.84-3.89 (m, 2 H), 3.08 (s, 3 H), 3.04 (m, 1 H), 2.74-2.80 (m, 2 H), 1.59-1.61 (m, 2 H), 1.43 (s, 9 H). Mass spectrum (ESI) *m*/*z:* 339.1 (M+H)⁺.

### Step 2: N-((R)-methyl-oxo-phenyl-λ⁴-sulfinyl)-4-piperidinamine (Int-11):

At 0°C, trifluoroacetic acid (6.09 mL, 82.2 mmol) was added to a solution of tert-butyl 4-(((R)-methyl-oxo-phenyl-λ⁴-sulfinyl)amino)piperidin-1-ylcarboxylate (Int-11.1, 1.6 g, 4.2 mmol) in DCM (16 mL). The resulting mixture was stirred at room temperature for 2 h, and TLC showed that the reaction was completed. The reaction mixture was concentrated under reduced pressure to obtain a crude product (2.2 g), which was basified with saturated NaHCO₃ solution to give *N*-((*R*)-methyl-oxo-phenyl-λ⁴-sulfinyl)-4-piperidinamine (Int-11), as a yellow oily liquid, which was used in the next reaction without further purification. Analytical data: Mass spectrum (ESI) *m*/*z:* 239 (M+H)⁺.

### Intermediate 12: 3-cyano-6-(1-methyl-1H-pyrazole-4-yl) pyrazolo[1,5-a]pyridin-4-yl trifluoromethanesulfonate (Int-12):

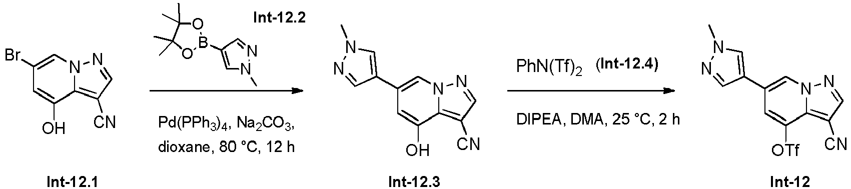

### Step 1: 4-hydroxy-6-(1-methyl-1H-pyrazole-4-yl)pyrazolo[1,5-a]pyridin-3-carbonitrile (Int-12.3):

Under nitrogen gas, Pd(PPh₃)₄ (3.64 g, 3.15 mmol) was added to a mixture of 6-bromo-4-hydroxypyrazolo[1,5-a]pyridin-3-carbonitrile (CAS#: (2068065-16-5), which was prepared according to the method reported in the literature (WO2019075092), Int-12.1, 15.0 g, 63.0 mmol) and 1-methyl-4-(4,4,5,5-tetramethyl-1,3,2-dioxaborolane-2-yl)-1H-pyrazole (Int-12.2, 15.7 g, 75.6 mmol) in dioxane (150 mL) and aqueous Na₂CO₃ solution (2 M, 94.5 mL, 189 mmol). The resulting mixture was stirred at 80°C for 12 h, and LC-MS showed that the reaction was completed. The reaction mixture was filtered, and the filter cake was washed with water (3 × 100 mL) and DCM (100 mL). The aqueous phases were combined, adjusted to about 5 of pH with 1M HCl. The solid product was collected by filtration, washed with water (2 × 50 mL), and dried to give 4-hydroxy-6-(1-methyl-1*H*-pyrazole-4-yl)pyrazolo[1,5-a]pyridin-3-carbonitrile (Int-12.3, 11.1 g, yield: 42%), as a brown solid. It was used in the next reaction without further purification. Analytical data: Mass spectrum (ESI) *m*/*z:* 240.1 (M+H)⁺.

### Step 2: 3-cyano-6-(1-methyl-1H-pyrazole-4-yl)pyrazolo[1,5-a]pyridin-4-yl trifluoromethanesulfonate (Int-12):

Diisopropylethylamine (7.43 mL, 42.6 mmol) was added to a suspension of 4-hydroxy-6-(1-methyl-1H-pyrazole-4-yl)pyrazolo[1,5-a]pyridin-3-carbonitrile (Int-12.3, 5.10 g, 21.3 mmol) in *N,N-*dimethyllacetamide (50 mL), and then *N*-phenyl-bis(trifluoro methanesulfonimide) (Int-12.4, 8.38 g, 23.5 mmol) was added. The resulting solution was stirred at room temperature for 2 h, and LC-MS showed that the reaction was completed. The reaction mixture was diluted with water (100 mL) and extracted with DCM (2x200 mL). The organic layers were combined, dried with anhydrous Na₂SO₄, filtered, and the filtrate was concentrated under reduced pressure to obtain a residue, which was purified by silica gel column chromatography (eluent: petroleum ether : ethyl acetate = 10:1 to 1:1) to obtain 3-cyano-6-(1-methyl-1*H*-pyrazole-4-yl)pyrazolo[1,5-a]pyridin-4-yl trifluoromethanesulfonate (Int-12, 5.40 g, yield: 25%). Analytical data: Mass spectrum (ESI) *m*/*z:* 372.1 (M+H)⁺

### Intermediate 13: 4-(6-fluoropyridin-3-yl)-6-(1-methyl-1H-pyrazole-4-yl)pyrazolo[1,5-a]pyridin-3-carbonitrile (Int-13):

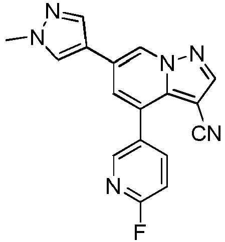

Int-13 (CAS#: 2068064-98-0) was prepared according to the method reported in the literature (WO2017011776). Analytical data: Mass spectrum (ESI) *m*/*z:* 319.1 (M+H)⁺.

### Intermediate 14: 3-cyano-6-(1-methyl-1H-pyrazole-4-yl)pyrazolo[1,5-a]pyrazine-4-yl trifluoromethanesulfonate (Int-14):

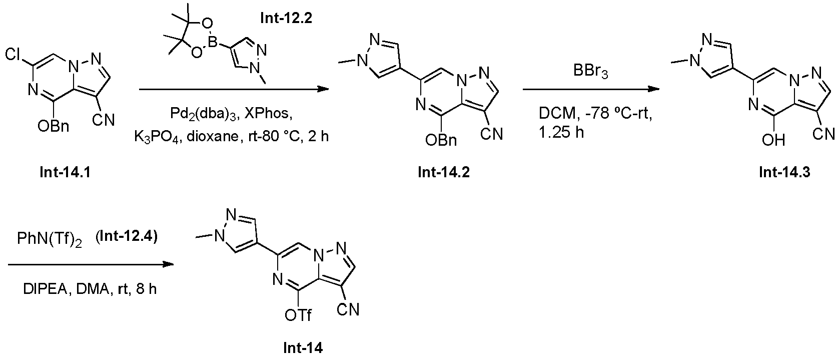

### Step 1: 4-(benzyloxy)-6-(1-methyl-1H-pyrazole-4-yl)pyrazolo[1,5-a]pyrazine-3-carbonitrile (Int-14.2):

Under nitrogen gas, Pd₂(dba)₃ (2.12 g, 2.32 mmol) and XPhos (2.21 g, 4.64 mmol) were added to a mixture of 4-(benzyloxy)-6-chloropyrazolo[1,5-a]pyrazine-3-carbonitrile (Int-14.1, CAS#: 1650547-68-4, which was prepared according to the method reported in WO2015017610, 13.2 g, 46.3 mmol) and 1-methyl-4-(4,4,5,5-tetramethyl-1,3,2-dioxaborolane-2-yl)-1H-pyrazole (Int-12.2, 10.6 g, 51.0 mmol) in K₃PO₄ aqueous solution (2M, 69.5 mL, 139 mmol) and dioxane (130 mL). The resulting mixture was stirred at 80°C for 2 h, and LC-MS showed that the reaction was completed. The reaction mixture was diluted with water (250 mL), extracted with DCM (3×200 mL). The organic layers were combined, washed with saturated brine (100 mL), dried with anhydrous Na₂SO₄, filtered, and the filtrate was concentrated under reduced pressure to obtain a residue. The trituration with petroleum ether: ethyl acetate = 5:1 at room temperature was performed for 5 minutes to give 4-(benzyloxy)-6-(1-methyl-1H-pyrazole-4-yl)pyrazolo[1,5-a]pyrazine-3-carbonitrile (Int-14.2, 9.0 g, yield: 53%, purity: 98.6%), as a yellow solid. Analytical data: Mass spectrum(ESI) *m*/*z:* 331.1 (M+H)⁺.

### Step 2: 4-hydroxy-6-(1-methyl-1Hpyrazole-4-yl)pyrazolo[1,5-a]pyrazine-3-carbonitrile (Int-14.3):

A solution of BBr₃ (12.9 mL, 33.7 g, 134 mmol) in DCM (135 mL) was added to a solution of 4-(benzyloxy)-6-(1-methyl-1Hpyrazole-4-yl)pyrazolo[1,5-a]pyrazine-3-carbonitrile (Int-14.2, 9.0 g, 26.9 mmol) in DCM (135 mL) at -78°C under nitrogen gas. The resulting mixture was stirred at room temperature for 1.25 h, and TLC showed that the reaction was completed. The reaction was quenched with water (500 mL) and the solid product was collected by filtration to give 4-hydroxy-6-(1-methyl-1*H*-pyrazole-4-yl)pyrazolo[1,5-a]pyrazine-3-carbonitrile (Int-14.3, 7 g, crude product), as a yellow solid. It was used in the next reaction without further purification. Analytical data: ¹H-NMR (400 MHz, DMSO-*d*₆):δ = 12.07 (m, 1 H), 8.53 (t, *J=* 10.0 Hz, 1 H), 8.33 (d, *J=* 9.2 Hz, 1 H), 8.22-8.27 (m, 1 H), 8.08 (s, 1 H), 3.88 (s, 3 H). Mass spectrum (ESI) *m*/*z:* 241.1 (M+H)⁺.

### Step 3: 3-cyano-6-(1-methyl-1H-pyrazole-4-yl)pyrazolo[1,5-a]pyrazine-4-yl trifluoromethanesulfonate (Int-14):

*N*-phenyl-bis(trifluoro methanesulfonimide) (Int-12.4, 9.67 g, 27.1 mmol) and diisopropylethylamine (8.34 mL, 47.9 mmol) were added to a solution of 4-hydroxy-6-(1-methyl-1*H-*pyrazole-4-yl)pyrazolo[1,5-a]pyrazine-3-carbonitrile (Int-14.3, 5.0 g, 20.8 mmol) in *N,N-*dimethyllacetamide (50 mL). The resulting mixture was stirred at room temperature for 8 h, and TLC showed that the reaction was completed. The resultant of reaction was diluted with water (100 mL), and the product was collected by filtration to give 3-cyano-6-(1-methyl-1*H*-pyrazole-4-yl)pyrazolo[1,5-a]pyrazine-4-yl trifluoromethanesulfonate (Int-14, 1.0 g, yield: 9.8%), as a yellow solid. It was used in the next reaction without further purification. Analytical data: ¹H-NMR (400 MHz, DMSO-*d*₆): δ = 9.57 (s, 1 H), 8.93 (m, 1 H), 8.24 (s, 1 H), 8.07 (s, 1 H), 3.92 (s, 3 H). Mass spectrum (ESI) *m*/*z:* 373.0 (M+H)⁺.

### Intermediate 15: 4-(6-fluoropyridin-3-yl)-6-(1-methyl-1H-pyrazole-4-yl)pyrazolo[1,5-a]pyrazine-3-carbonitrile (Int-15):

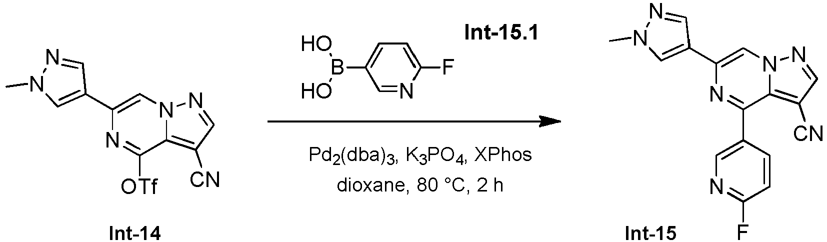

Under nitrogen gas, Pd₂(dba)₃ (84.3 mg, 0.092 mmol) and XPhos (87.7 mg, 0.184 mmol) were added to a mixture of 3-cyano-6-(1-methyl-1*H*-pyrazole-4-yl)pyrazolo[1,5-a]pyrazine-4-yl trifluoromethanesulfonate (Int-14, 901 mg, 1.84 mmol) and (6-fluoropyridin-3-yl)boronic acid (Int-15.1, 311 mg, 2.21 mmol) in aqueous K₃PO₄ solution(2M, 2.76 mL, 5.52 mmol) and dioxane (10 mL). The resulting mixture was degassed and stirred at 80°C for 2 h, and LC-MS showed that the reaction was completed. After cooling to 0°C, the reaction mixture was diluted with water (50 mL), extracted with DCM (2×100 mL). The organic layers were combined, washed with saturated brine (200 mL), dried with anhydrous Na₂SO₄, filtered, and the filtrate was concentrated under reduced pressure to obtain a residue, which was purified by silica gel column chromatography (eluent: dichloromethane : methanol = 1:0 to 100:1) to obtain 4-(6-fluoropyridin-3-yl)-6-(1-methyl-1*H*-pyrazole-4-yl)pyrazolo[1,5-a]pyrazine-3-carbonitrile (Int-15, 300 mg, yield: 43%), as a yellow solid. Analytical data: Mass spectrum (ESI) *m*/*z:* 319.9 (M+H)⁺.

### Example 1:

### tert-butyl (4-(5-(3-cyano-6-(2-hydroxy-2-methylpropoxy)pyrazolo[1,5-a]pyridin-4-yl)pyridin-2-yl)-1-oxothiomorpholine-1-imino)carboxylate (Ex. 1):

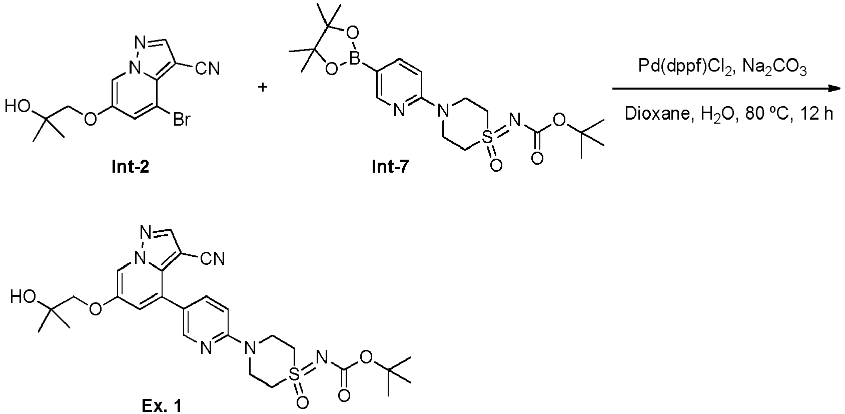

Under nitrogen gas, Pd(dppf)Cl₂ (209 mg, 0.286 mmol) was added to a mixture of tert-butyl (1-oxo-4-(5-(4,4,5,5-tetramethyl-1,3,2-dioxaborolane-2-yl)pyridin-2-yl)thiomorpholine-1-imino)carboxylate (Int-7, 2.50 g, 5.72 mmol), 4-bromo-6-(2-hydroxy-2-methylpropoxy)pyrazolo[1,5-a]pyridin-3-carbonitrile (Int-2, 886 mg, 2.86 mmol, 1eq) and Na₂CO₃ (606 mg, 5.72 mmol) in water (15 mL) and dioxane (25 mL). The resulting mixture was stirred at 80°C for 12 h, and LC-MS showed that the reaction was completed. After cooling to room temperature, the reaction mixture was filtered, the filtrate was diluted with water (30 ml), and extracted with DCM (3×30 mL). The organic layers were combined, washed with saturated brine (30 mL), dried with anhydrous Na₂SO₄, filtered, and the filtrate was concentrated under reduced pressure to obtain a residue, which was purified by preparative HPLC (column: Phenomenex luna C18 150^{∗}40 mm^{∗}15 um; mobile phase: [water (0.225% formic acid)-acetonitrile]; B%: 1% to 25%, 9 minutes) to obtain tert-butyl (4-(5-(3-cyano-6-(2-hydroxy-2-methylpropoxy)pyrazolo[1,5-a]pyridin-4-yl)pyridin-2-yl)-1-oxothiomorpholine-1-imino)carboxylate (Ex. 1, 1.30 g, yield: 84%), as a yellow solid. Analytical data: ¹H-NMR (400 MHz, CDCl₃):δ = 8.39 (s, 1 H), 8.19-8.20 (m, 2 H), 7.80 (d, *J =* 7.0 Hz, 1 H), 7.19 (s, 1 H), 6.91 (d, *J =* 8.8 Hz, 1 H), 4.38-4.42 (m, 2 H), 4.08 (dd, *J=* 8.0, 13.2 Hz, 2 H), 3.87 (s, 2 H), 3.67-3.70 (m, 2 H), 3.28-3.48 (m, 2 H), 1.50 (s, 9 H), 1.39 (s, 6 H). Mass spectrum (ESI) *m*/*z:* 441.2 (M+H-100)⁺.

### Example 2: 6-(2-hydroxy-2-methylpropoxy)-4-(6-(1-imino-1-oxothiomorpholinyl)pyridin-3-yl)pyrazolo[1,5-a]pyridine-3-carbonitrile (Ex.2):

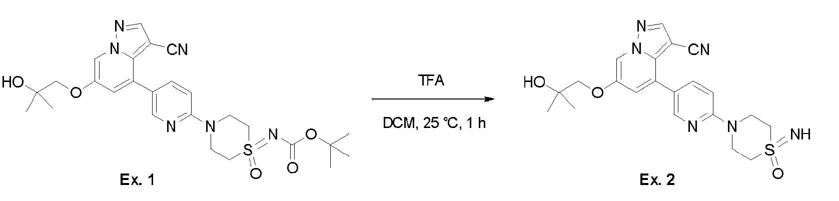

Trifluoroacetic acid (5 mL, 67.5 mmol) was added to a solution of tert-butyl (4-(5-(3-cyano-6-(2-hydroxy-2-methylpropoxy)pyrazolo[1,5-a]pyridin-4-yl)pyridin-2-yl)-1-oxothiomorpholine-1-imino)carboxylate (Ex. 1, 1.30 g, 2.40 mmol) in DCM (10 mL). The resulting mixture was stirred at 25°C for 1 h, and LC-MS showed that the reaction was completed. The reaction mixture was concentrated under reduced pressure to obtain a crude product, which was washed with petroleum ether, dissolved in MeOH, and neutralized by dropwise addition of NH₃.H₂O. The obtained solid product was collected by filtration and dried to obtain 6-(2-hydroxy-2-methylpropoxy)-4-(6-(1-imino-1-oxothiomorpholinyl)pyridine-3-yl)pyrazolo[1,5-a]pyridin-3-carbonitrile (Ex. 2, 26 mg, yield: 2.4%, purity: 98.7%), as a white solid. Analytical data: ¹H-NMR (400 MHz, DMSO-*d*₆): δ = 8.69 (s, 1 H), 8.57 (s, 1 H), 8.39 (d, *J*= 2.4 Hz, 1 H), 7.85 (dd, *J =* 2.4, 8.8 Hz, 1 H), 7.34 (d, *J =* 20 Hz, 1 H), 7.14 *(d, J =* 8.8 Hz, 1 H), 4.70 (s, 1 H), 4.29 (d, *J =* 16.0 Hz, 2 H), 3.87-3.93 (m, 5 H), 2.97-3.02 (m, 4 H), 1.22 (s, 6 H). Mass spectrum (ESI) *m*/*z:* 441.1 (M+H)⁺.

### Example 3: 6-(2-hydroxy-2-methylpropoxy)-4-(6-(1-((6-methoxypyridin-3-yl)imino)-1-oxothiomorpholinyl)pyridin-3-yl)pyrazolo[1,5-a]pyridine-3-carbonitrile (Ex. 3):

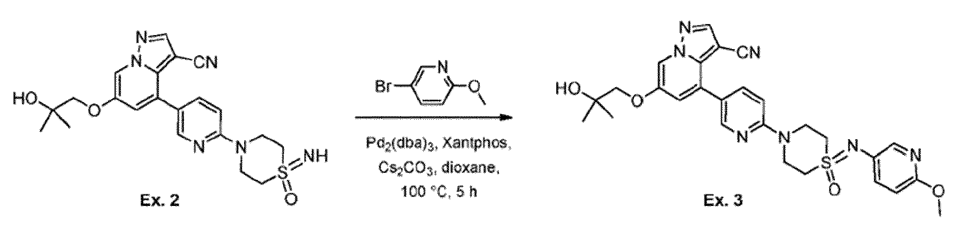

Under nitrogen gas, Xantphos (20.9 mg, 0.036 mmol) and Pd₂(dba)₃ (16.5 mg, 0.018 mmol) were added to a mixture of 6-(2-hydroxy-2-methylpropoxy)-4-(6-(1-imino-1-oxothiomorpholinyl)pyridin-3-yl)pyrazolo[1,5-a]pyridine-3-carbonitrile (Ex. 2, 159 mg, 0.361 mmol), 5-bromo-2-methoxypyridine (67.8 mg, 0.361 mmol) and CS₂CO₃ (353 mg, 1.08 mmol) in dioxane (1 mL). The resulting mixture was stirred at 100°C for 5 h, and LC-MS showed that the reaction was completed. The reaction mixture was filtered, and the filtrate was concentrated under reduced pressure to obtain a residue, which was purified by preparative HPLC (column: Phenomenex luna C18 150^{∗}40 mm^{∗} 15 um; mobile phase: [water (0.225% formic acid)-acetonitrile]; B%: 1% to 25%, 9 minutes) to give 6-(2-hydroxy-2-methylpropoxy)-4-(6-(1-((6-methoxypyridin-3-yl)imino)-1-oxothiomorpholinyl)pyridin-3-yl)pyrazolo[1,5-a]pyridine-3-carbonitrile (Ex. 3, 30 mg, yield: 14.6%), as a white solid. Analytical data: ¹H-NMR (400 MHz, DMSO-*d*₆):δ = 8.69 (d, *J =* 2.0 Hz, 1 H), 8.57 (s, 1 H), 8.41 (d, *J =* 2.4 Hz, 1 H), 7.88-7.89 (m, 2 H), 7.39 (dd, *J =* 2.8, 8.8 Hz, 1 H), 7.34 (d, *J* 2.0 Hz, 1 H), 7.17 (d, *J* = 9.2 Hz, 1 H), 6.71 (d, *J =* 8.8 Hz, 1 H), 4.71 (s, 1 H), 4.43 (d, *J =* 15.4 Hz, 2 H), 3.87-3.90 (m, 4 H), 3.79 (s, 3 H), 3.39-3.42 (m, 2 H), 3.19-3.24 (m, 2 H), 1.23 (s, 6 H). Mass spectrum(ESI) m/z:548.2 (M+H)⁺.

### Example 4: 6-(2-hydroxy-2-methylpropoxy)-4-(6-(1-(isobutylimino)-1-oxothiomorpholinyl)pyridin-3-yl)pyrazolo[1,5-a]pyridine-3-carbonitrile (Ex. 4):

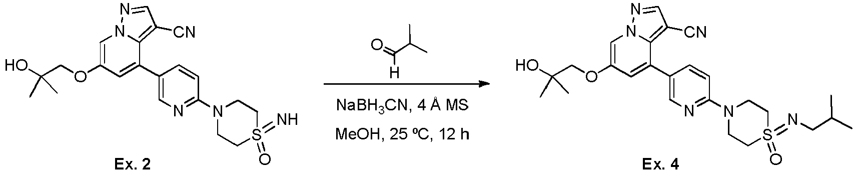

NaBH₃CN (42.5 mg, 0.676 mmol) was added to a mixture of 6-(2-hydroxy-2-methylpropoxy)-4-(6-(1-imino-1-oxothiomorpholinyl)pyridin-3-yl)pyrazolo[1,5-a]pyridine-3-carbonitrile (Ex. 2, 199 mg, 0.451 mmol), isobutyraldehyde (163 mg, 0.206 mL, 2.25 mmol), and 4Å molecular sieves (200 mg) in MeOH (2 mL).The resulting mixture was stirred at 25°C for 12 h, and LC-MS showed that the reaction was completed. The reaction mixture was filtered, and the filtrate was concentrated under reduced pressure to obtain a residue, which was purified by preparative HPLC (column:Phenomenex luna C18 150^{∗}40 mm^{∗}15 um; mobile phase: [water (0.225% formic acid)-acetonitrile]; B%: 1% to 25%, 9 minutes) to obtain 6-(2-hydroxy-2-methylpropoxy)-4-(6-(1-(isobutylimino)-1-oxothiomorpholinyl)pyridin-3-yl)pyrazolo[1,5-a]pyridine-3-carbonitrile (Ex. 4, 30 mg, yield: 13%), as a white solid. Analytical data: ¹H-NMR (400 MHz, DMSO-*d*₆):δ = 8.69 (d, *J =* 2.0 Hz, 1 H), 8.57 (s, 1 H), 8.39 (d, *J =* 2.4 Hz, 1 H), 7.86 (dd, *J =* 2.4, 8.8 Hz, 1 H), 7.34 (d, *J =* 2.0 Hz, 1 H), 7.15 (d, *J =* 8.8 Hz, 1 H), 4.71 (s, 1 H), 4.27-4.32 (m, 2 H), 3.81-3.85 (m, 4 H), 3.19 (dd, *J=* 3.6, 12.4 Hz, 2 H), 2.97-3.02 (m, 2 H), 2.79 (d, *J=* 6.4 Hz, 2 H), 1.66 (dt, *J=* 6.6, 13.2 Hz, 1 H), 1.22 (s, 6 H), 0.90 (d, *J* = 6.8 Hz, 6 H). Mass spectrum (ESI) *m*/*z:* 497.3 (M+H)⁺.

### Example 5: 6-(2-hydroxy-2-methylpropoxy)-4-(6-(1-(((6-methoxypyridin-3-yl)methyl)imino)-1-oxothiomorpholinyl)pyridin-3-yl)pyrazolo[1,5-a]pyridine-3-carbonitrile (Ex. 5):

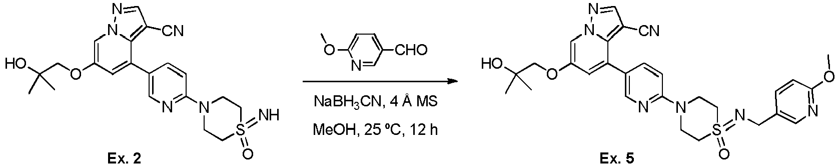

It was prepared using the same method as Example 4 (Ex. 4). Analytical data: Analytical data: ¹H-NMR (400 MHz, DMSO-*d*₆): δ = 8.68 (d, *J=* 2.4 Hz, 1 H), 8.57 (s, 1 H), 8.39 (d, *J=* 2.4 Hz, 1 H), 8.13 (d, *J =* 2.0 Hz, 1 H), 8.86 (dd, *J =* 2.4, 8.8 Hz, 1 H), 7.70 (dd, *J* = 2.4, 8.4 Hz, 1 H), 7.34 (d, *J* = 2.4 Hz, 1 H), 7.16 (d, *J=* 8.8 Hz, 1 H), 6.76 (d, *J* = 8.4 Hz, 1 H), 4.71 (s, 1 H), 4.28-4.32 (m, 2 H), 4.18 (s, 2 H), 3.83-3.92 (m, 4 H), 3.82 (s, 3 H), 3.25-3.28 (m, 2 H), 3.04-3.10 (m, 2 H), 1.23 (s, 6 H). Mass spectrum (ESI) *m*/*z:* 562.2 (M+H)⁺.

### Example 6: tert-butyl 4-(4-(5-(3-cyano-6-(2-hydroxy-2-methylpropoxy)pyrazolo[1,5-a]pyridin-4-yl)pyridin-2-yl)-1-oxothiomorpholine-1-imino)piperidine-1-carboxylate (Ex. 6):

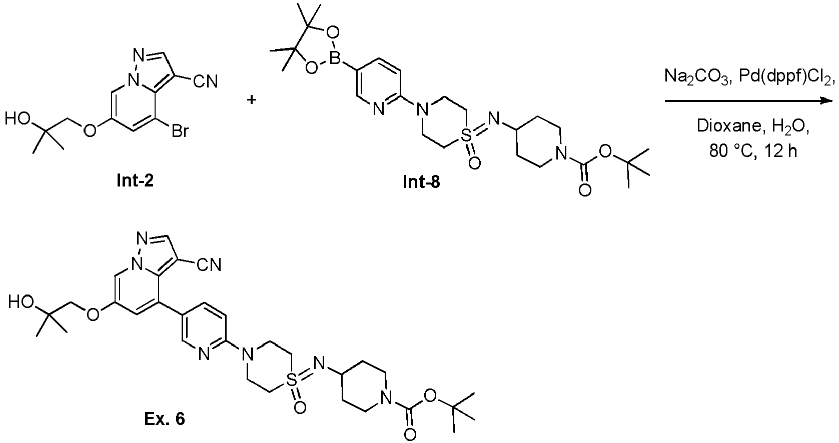

Under nitrogen gas, Pd(dppf)Cl₂ (33.7 mg, 0.046 mmol) was added to a mixture of tert-butyl 4-(1-oxo-4-(5-(4,4,5,5-tetramethyl-1,3,2-dioxaborolane-2-yl)pyridin-2-yl)thiomorpholine-1-imino)piperidine-1-carboxylate (Int-8, 360 mg, 0.692 mmol), 4-bromo-6-(2-hydroxy-2-methylpropoxy)pyrazolo[1,5-a]pyridin-3-carbonitrile (Int-2, 143 mg, 0.461 mmol) and Na₂CO₃ (97.7 mg, 0.922 mmol) in water (0.5 mL) and dioxane (2 mL). The resulting mixture was stirred at 80°C for 12 h, and LC-MS showed that the reaction was completed. The reaction mixture was filtered, and the filtrate was concentrated under reduced pressure to obtain a residue, which was purified by preparative HPLC (column: Phenomenex luna C18 150^{∗}40 mm^{∗} 15 um; mobile phase: [water (0.225% formic acid)-acetonitrile]; B%: 1% to 25%, 9 minutes) to obtain tert-butyl 4-(4-(5-(3-cyano-6-(2-hydroxy-2-methylpropoxy)pyrazolo[1,5-a]pyndin-4-yl)pyridin-2-yl)-1-oxothiomorpholine-1-imino)piperidine-1-carboxylate (Ex. 6, 107 mg, yield: 37%), as a yellow solid. Analytical data: Mass spectrum (ESI) *m*/*z:* 624.3 (M+H)⁺.

### Example 7: 6-(2-hydroxy-2-methylpropoxy)-4-(6-(1-oxo-1-(piperidin-4-ylimino)thiomorpholinyl)pyridin-3-yl)pyrazolo[1,5-a]pyridine-3-carbonitrile (Ex. 7):

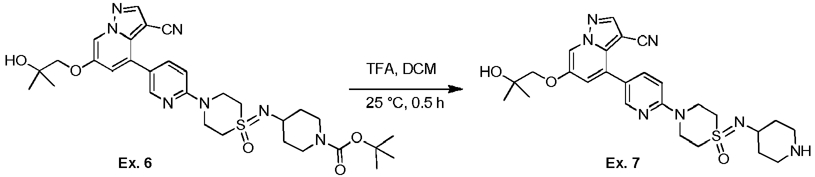

At 0°C, trifluoroacetic acid (0.332 mL, 4.46 mmol) was added to a solution of tert-butyl 4-(4-(5-(3-cyano-6-(2-hydroxy-2-methylpropoxy)pyrazolo[1,5-a]pyridin-4-yl)pyridin-2-yl)-1-oxothiomorpholine-1-imino)piperidine-1-carboxylate (Ex. 6, 110 mg, 0.172 mmol) in DCM (1.5 mL). The resulting mixture was stirred at room temperature for 0.5 h, and TLC showed that the reaction was completed. The reaction mixture was concentrated under reduced pressure to obtain a residue, which was purified by preparative HPLC (column: Phenomenex luna C18 150^{∗}40 mm^{∗}15 um; mobile phase: [water (0.225% formic acid)-acetonitrile]; B%: 1% to 25%, 9 minutes) to obtain 6-(2-hydroxy-2-methylpropoxy)-4-(6-(1-oxo-1-(piperidin-4-ylimino)thiomorpholinyl)pyridin-3-yl)pyrazolo[1,5-a]pyridine-3-carbonitrile (Ex. 7, 49.8 mg, yield: 55%), as a yellow solid. Analytical data: ¹H-NMR (400 MHz, CD₃OD):δ = 8.48 (d, *J* = 2.0 Hz, 1 H), 8.39 (d, *J* = 2.4 Hz, 1 H), 8.33 (s, 1 H), 7.86 (dd, *J* = 2.4, 8.8 Hz, 1 H), 7.34 (d, *J =* 2.0 Hz, 1 H), 7.14 (d, *J* = 8.8 Hz, 1 H), 4.36 (dd, *J* = 3.8, 14.6 Hz, 2 H), 4.10-4.13 (m, 2 H), 3.92 (s, 2 H), 3.72 (m, 1 H), 3.31-3.40 (m,4 H), 3.09-3.13 (m, 2 H), 2.05-2.10 (m, 2 H), 1.72-1.82 (m, 2 H), 1.36 (s, 6 H). Mass spectrum (ESI) *m*/*z:* 524.3 (M+H)⁺.

### Example 8: 4-(6-(4-((dimethyloxo-λ⁴-sulfinyl)amino)piperidin-1-yl)pyridin-3-yl)-6-(2-hydroxy-2-methylpropoxy)pyrazolo[1,5-a]pyridine-3-carbonitrile (Ex. 8):

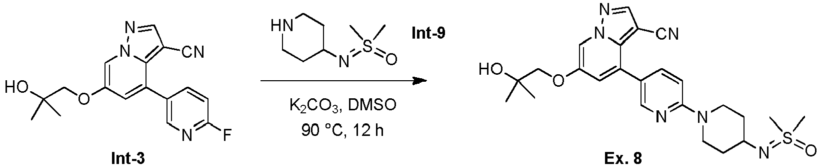

A mixture of *N*-(dimethyloxo-λ⁴-sulfinyl)-4-piperidinamine (Int-9, 200 mg, 1.13 mmol), 4-(6-fluoropyridin-3-yl)-6-(2-hydroxy-2-methylpropoxy)pyrazolo[1,5-a]pyridine-3-carbonitrile (Int-3, 473 mg, 1.13 mmol) and K₂CO₃ (313 mg, 2.27 mmol) in DMSO(2 mL) was stirred at 90°C for 12 h, and LC-MS showed that the reaction was completed. The solid product was collected by filtration and purified by preparative HPLC (column: Phenomenex luna C18 150^{∗}40 mm^{∗}15 um; mobile phase: [water (0.225% formic acid)-acetonitrile]; B%: 1% to 25%, 9 minutes) to obtain 4-(6-(4-((dimethyloxo-λ⁴-sulfinyl)amino)piperidin-1-yl)pyridin-3-yl)-6-(2-hydroxy-2-methylpropoxy)pyrazolo[1,5-a]pyridine-3-carbonitrile (Ex. 8, 40 mg, yield: 17%), as a yellow solid. Analytical data: ¹H-NMR (400 MHz, CD₃OD):δ = 8.43 (d, *J* = 2.0 Hz, 1 H), 8.33 (s, 1 H), 8.26 (dd, *J* = 2.0, 12.4 Hz, 1 H), 7.75 (dd, *J* = 2.4, 8.8 Hz, 1 H), 7.29 (d, *J* = 2.0 Hz, 1 H), 6.94 (d, *J* = 8.8 Hz, 1 H), 4.31 (d, *J* = 13.2 Hz, 2 H), 3.91 (s, 2 H), 3.54 (m, 1 H), 3.13 (s, 6 H), 3.08-3.12 (m, 2 H), 1.92 (d, *J* = 10.0 Hz, 2 H), 1.54-1.64 (m, 2 H), 1.35 (s, 6 H). Mass spectrum (ESI) *m*/*z:* 483.2 (M+H)⁺.

### Example 9: 4-(6-(4-(((S)-S-methyl-S-phenyl-oxo-λ⁴-sulfinyl)amino)piperidin-1-yl)pyridin-3-yl)-6-(2-hydroxy-2-methylpropoxy)pyrazolo[1,5-a]pyridine-3-carbonitrile (Ex. 9):

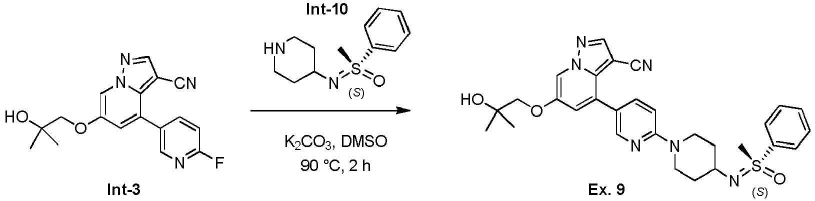

A mixture of *N*-((*S*)-methyl-oxo-phenyl-λ⁴-sulfinyl)-4-piperidinamine (Int-10, 541 mg, 2.27 mmol), 4-(6-fluoropyridin-3-yl)-6-(2-hydroxy-2-methylpropoxy)pyrazolo[1,5-a]pyridine-3-carbonitrile (Int-3, 123 mg, 0.306 mmol) and K₂CO₃ (414.6 mg, 4.0 mmol) in DMSO (2 mL) was stirred at 90°C for 2 h, and LC-MS showed that the reaction was completed. After cooling to room temperature, the solid product was collected by filtration, which was purified by preparative HPLC (column: Shim-pack C18 150^{∗}25^{∗}10 um; mobile phase: [water (0.225% formic acid)-acetonitrile]; B%: 16% to 49%,11 minutes) to obtain 4-(6-(4-(((*S*)-*S*-methyl-*S*-phenyl-oxo-λ⁴-sulfinyl)amino)piperidin-1-yl)pyridin-3-yl)-6-(2-hydroxy-2-methylpropoxy)pyrazolo[1,5-a]pyridine-3-carbonitrile (Ex. 9, 36.9 mg, yield: 12%), as a light brown solid. Analytical data: ¹H-NMR (400 MHz, DMSO-*d*₆):δ = 8.66 (d, *J=* 2.4 Hz, 1 H), 8.57 (s, 1 H), 8.29 (d, *J=* 2.4 Hz, 1 H), 7.91 (dd, *J=* 1.6, 8.4 Hz, 2 H), 7.65-7.72 (m, 4 H), 7.28 (d, *J=* 2.0 Hz, 1 H), 6.91 (d, *J=* 9.2 Hz, 1 H), 4.71 (s, 1 H), 4.10-4.16 (m, 2 H), 3.86 (s, 2 H), 3.14 (s, 3 H), 2.99-3.06 (m, 2 H), 1.36-1.82 (m, 5 H), 1.22 (s, 6 H). Mass spectrum (ESI) *m*/*z:* 545.2 (M+H)⁺.

### Example 10: 4-(6-(4-(((R)-S-methyl-S-phenyl-oxo-λ⁴-sulfinyl)amino)piperidin-1-yl)pyridin-3-yl)-6-(2-hydroxy-2-methylpropoxy)pyrazolo[1,5-a]pyridine-3-carbonitrile (Ex. 10):

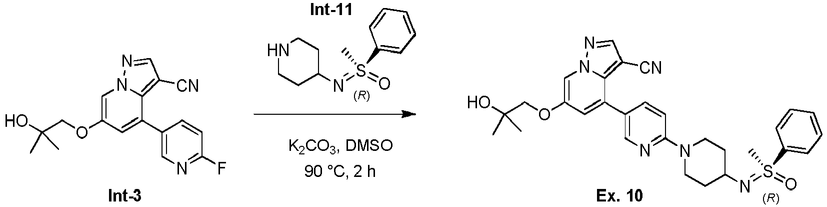

A mixture of *N*-[(*R*)-methyl-oxo-phenyl-λ⁴-sulfinyl]-4-piperidinamine (Int-11, 183 mg, 0.519 mmol), 4-(6-fluoropyridin-3-yl)-6-(2-hydroxy-2-methylpropoxy)pyrazolo[1,5-a]pyridine-3-carbonitrile (Int-3, 127 mg, 0.307 mmol) and K₂CO₃ (207 mg, 2.0 mmol) in DMSO (2 mL) was stirred at 90°C for 2 h, and LC-MS showed that the reaction was completed. After cooling to room temperature, the solid product was collected by filtration, which was purified by preparative HPLC (column: Shim-pack C18 150^{∗}25^{∗}10 um; mobile phase: [water (0.225% formic acid)-acetonitrile]; B%: 16% to 49%, 11 minutes) to obtain 4-(6-(4-(((*R*)-*S*-methyl-*S*-phenyl-oxo-λ⁴-sulfinyl)amino)piperidin-1-yl)pyridin-3-yl)-6-(2-hydroxy-2-methylpropoxy)pyrazolo[1,5-a]pyridine-3-carbonitrile (Ex. 10, 40 mg, yield: 18%), as a yellow solid. Analytical data: ¹H-NMR (400 MHz, DMSO-*d*₆):δ = 8.66 (d, *J=* 2.4 Hz, 1 H), 8.57 (s, 1 H), 8.29 (d, *J=* 2.4 Hz, 1 H), 7.91 (dd, *J=* 1.6, 8.4 Hz, 2 H), 7.65-7.72 (m, 4 H), 7.28 (d, *J=* 2.0 Hz, 1 H), 6.91 (d, *J* = 9.2 Hz, 1 H), 4.71 (s, 1 H), 4.10-4.16 (m, 2 H), 3.86 (s, 2 H), 3.14 (s, 3 H), 2.99-3.06 (m, 2 H), 2.51-2.52 (m, 1 H), 1.36-1.75 (m, 4 H), 1.22 (s, 6 H). Mass spectrum (ESI) *m*/*z:* 545.2 (M+H)⁺.

### Example 11: tert-butyl (4-(5-(3-cyano-6-(1-methyl-1H-pyrazole-4-yl)pyrazolo[1,5-a]pyridin-4-yl)pyridin-2-yl)-1-oxothiomorpholine-1-imino)carboxylate (Ex. 11):

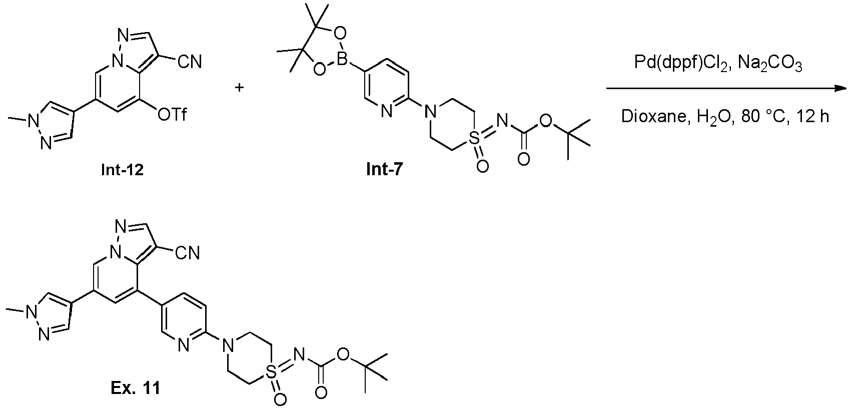

Under nitrogen gas, Pd(dppf)Cl₂ (394 mg, 0.539 mmol) was added to a solution of 3-cyano-6-(1-methyl-1*H*-pyrazole-4-yl)pyrazolo[1,5-a]pyridin-4-yl trifluoromethanesulfonate (Int-12, 2.0 g, 5.39 mmol) and tert-butyl (1-oxo-4-(5-(4,4,5,5-tetramethyl-1,3,2-dioxaborolane-2-yl)pyridin-2-yl)thiomorpholine-1-imino)carboxylate (Int-7, 2.83 g, 6.46 mmol) in dioxane (20 mL) and aqueous Na₂CO₃ solution (2 M, 8.08 mL, 16.16 mmol). The resulting mixture was stirred at 80°C for 12 h, and TLC showed that the reaction was completed. After cooling to room temperature, the reaction mixture was diluted with water (50 mL) and extracted with DCM (2×50 mL). The organic layers were combined, dried with anhydrous Na₂SO₄, filtered, and the filtrate was concentrated under reduced pressure to obtain a residue, which was purified by silica gel column chromatography (eluent: petroleum ether : ethyl acetate = 10:1 to 1:1) to obtain tert-butyl (4-(5-(3-cyano-6-(1-methyl-1*H*-pyrazole-4-yl)pyrazolo[1,5-a]pyridin-4-yl)pyridin-2-yl)-1-oxothiomorpholine-1-imino)carboxylate (Ex. 11, 1.90 g, yield: 65.6%), as a yellow solid. Analytical data: ¹H-NMR (400 MHz, CDCl₃):δ = 8.68 (s, 1 H), 8.41 (d, *J* = 2.4 Hz, 1 H), 8.27 (s, 1 H), 7.83 (dd, *J* = 2.4, 8.8 Hz, 1 H), 7.81 (s, 1 H), 7.71 (s, 1 H), 7.43 (d, *J* = 1.6 Hz, 1 H), 6.92 (d, *J* = 8.8 Hz, 1 H), 4.40-4.45 (m, 2 H), 4.10-4.13 (m, 2 H), 4.01 (s, 3 H), 3.66-3.68 (m, 2 H), 3.34-3.38 (m, 2 H), 1.51 (s, 9 H). Mass spectrum (ESI) *m*/*z:* 533.2 (M+H)⁺, 433.1 (M+H-100)⁺.

### Example 12: 4-(6-(1-imino-1-oxothiomorpholinyl)pyridin-3-yl)-6-(1-methyl-1H-pyrazole-4-yl)pyrazolo[1,5-a]pyridine-3-carbonitrile (Ex. 12):

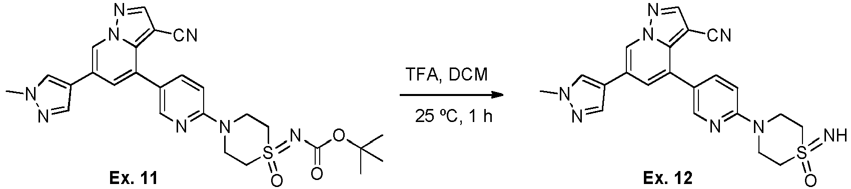

Trifluoroacetic acid (7.84 mL, 106 mmol) was added to a solution of tert-butyl (4-(5-(3-cyano-6-(1-methyl-1*H*-pyrazole-4-yl)pyrazolo[1,5-a]pyridin-4-yl)pyridin-2-yl)-1-oxothiomorpholine-1-imino)carboxylate (Ex. 11, 1.90 g, 3.53 mmol) in DCM (20 mL). The resulting mixture was stirred at room temperature for 1 h, and TLC showed that the reaction was completed. The reaction mixture was concentrated under reduced pressure to obtain a residue, and the residue was suspended in methyl tert-butyl ether (20 mL) at room temperature, and the solid product, 4-(6-(1-imino-1-oxothiomorpholinyl)pyridin-3-yl)-6-(1-methyl-1Hpyrazole-4-yl)pyrazolo[1,5-a]pyridine-3-carbonitriletrifluoroacetate (Ex. 12.TFA, 2.40 g, crude product), was collected from the filtrate as a yellow solid. The solid product was used in the next reaction without further purification, a small part of which was purified using preparative HPLC (column: Phenomenex luna C18 150^{∗}40 mm^{∗} 15 um; mobile phase: [water (0.225% formic acid)-acetonitrile]; B%: 1% to 25%, 9 minutes) to obtain a free base sample (Ex. 12) with >99% purity for biological activity assays. ¹H-NMR (400 MHz, DMSO-*d₆):δ* = 9.25 (d, *J* = 1.2 Hz, 1 H), 8.65 (s, 1 H), 8.44 (d, *J* = 2.4 Hz, 1 H), 8.40 (s, 1 H), 8.13 (s, 1 H), 7.89-7.92 (m, 1 H), 7.82 (d, *J* = 2.4 Hz, 1 H), 7.18 (d, *J* = 8.8 Hz, 1 H), 4.29-4.32 (m, 2 H), 3.88 (s, 3 H), 3.86-3.90 (m, 3 H), 2.98-3.04 (m, 4 H). Mass spectrum (ESI) *m*/*z:* 433.1 (M+H)⁺.

### Example 13: 4-(6-(1-(isobutylimino)-1-oxothiomorpholinyl)pyridin-3-yl)-6-(1-methyl-1H-pyrazole-4-yl)pyrazolo[1,5-a]pyridine-3-carbonitrile (Ex. 13):

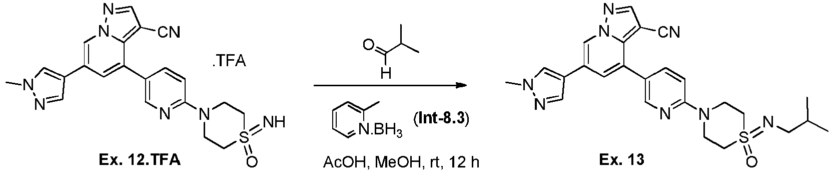

2-Methylpyridin-boronic acid complex (Int-8.3, 143 mg, 1.33 mmol) was added to a mixture of 4-(6-(1-imino-1-oxothiomorpholinyl)pyridin-3-yl)-6-(1-methyl-1H-pyrazole-4-yl)pyrazolo[1,5-a]pyridine-3-carbonitrile trifluoroacetate (Ex. 12.TFA, 500 mg, 0.887 mmol), isobutyraldehyde (256 mg, 0.323 mL, 3.55 mmol) and acetic acid (242 mg, 0.23 mL, 4.03 mmol) in MeOH (3 mL). The resulting mixture was stirred at room temperature for 12 h, and LC-MS showed that the reaction was completed. The reaction mixture was filtered, and the filtrate was concentrated under reduced pressure to obtain a residue, which was purified by preparative HPLC (column: Phenomenex luna C18 150^{∗}40 mm^{∗}15 um; mobile phase: [water (0.225% formic acid)-acetonitrile]; B%: 17% to 47%, 9 minutes) to obtain 4-(6-(1-(isobutylimino)-1-oxothiomorpholinyl)pyridin-3-yl)-6-(1-methyl-1*H*pyrazole-4-yl)pyrazolo[1,5-a]pyridine-3-carbonitrile (Ex. 13, 30 mg, yield: 6.8%), as a white solid. Analytical data: ¹H-NMR (400 MHz, CDCl₃):δ = 8.67 (d, *J* = 1.6 Hz, 1 H), 8.41 (d, *J* = 2.0 Hz, 1 H), 8.27 (s, 1 H), 7.82 (dd, *J* = 2.8, 7.2 Hz, 1 H), 7.80 (s, 1 H), 7.71 (s, 1 H), 7.43 (s, 1 H), 6.90 (d, *J* = 8.8 Hz, 1 H), 4.29-4.31 (m, 2 H), 4.06-4.11 (m, 2 H), 4.01 (s, 3 H), 3.19-3.21 (m, 2 H), 3.12-3.15 (m, 2 H), 2.92 (d, *J* = 6.8 Hz, 2 H), 1.78 (m, 1 H), 0.98 (d, *J* = 6.4 Hz, 6 H). Mass spectrum(ESI) *m*/*z:* 489.2 (M+H)⁺.

### Example 14: 4-(6-(1-(((6-methoxypyridin-3-yl)methyl)imino)-1-oxothiomorpholinyl)pyridin-3-yl)-6-(1-methyl-1H-pyrazole-4-yl)pyrazolo[1,5-a]pyridine-3-carbonitrile (Ex. 14):

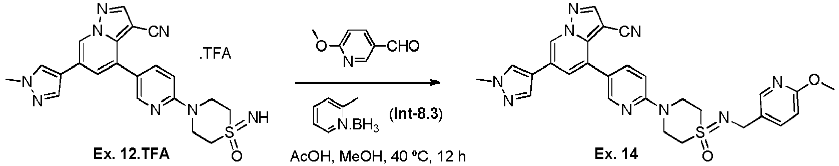

2-Methylpyridin-boronic acid complex (Int-8.3, 143 mg, 1.33 mmol) was added to a mixture of 4-(6-(1-imino-1-oxothiomorpholinyl)pyridin-3-yl)-6-(1-methyl-1H-pyrazole-4-yl)pyrazolo[1,5-a]pyridine-3-carbonitrile trifluoroacetate (Ex. 12.TFA, 500 mg, 0.887 mmol), 6-methoxynicotinaldehyde (487 mg, 3.55 mmol) and acetic acid (249 mg, 0.237 mL, 4.15 mmol) in MeOH (3 mL). The resulting mixture was stirred at 40°C for 12 h, and LC-MS showed that the reaction was completed. The reaction mixture was filtered, and the filtrate was concentrated under reduced pressure to obtain a residue, which was purified by preparative HPLC (column: Phenomenex luna C18 150^{∗}40 mm^{∗}15 um; mobile phase: [water (0.225% formic acid)-acetonitrile]; B%: 17% to 47%, 9 minutes) to obtain 4-(6-(1-(((6-methoxypyridin-3-yl)methyl)imino)-1-oxothiomorpholinyl)pyridin-3-yl)-6-(1-methyl-1//-pyrazole-4-yl)pyrazolo[1,5-a]pyridine-3-carbonitrile (Ex. 14, 249 mg, yield: 50.5%), as a white solid. Analytical data: ¹H-NMR (400 MHz, CDCl₃):δ = 8.67 (d, *J=* 1.6 Hz, 1 H), 8.40 (d, *J* = 2.0 Hz, 1 H), 8.27 (s, 1 H), 8.17 (d, *J* = 2.0 Hz, 1 H), 7.80-7.82 (m, 2 H), 7.70-7.73 (m, 2 H), 7.43 (d, *J* = 1.6 Hz, 1 H), 6.88 (d, *J* = 8.4 Hz, 1 H), 6.75 (d, *J* = 8.4 Hz, 1 H), 4.26-4.30 (m, 4 H), 4.09-4.12 (m, 2 H), 4.01 (s, 3 H), 3.94 (s, 3 H), 3.10-3.16 (m, 4 H). Mass spectrum (ESI) *m*/*z:* 554.3 (M+H)⁺.

### Example 15: 4-(6-(1-((6-methoxypyridin-3-yl)imino)-1-oxothiomorpholinyl)pyridin-3-yl)-6-(1-methyl-1H-pyrazole-4-yl)pyrazolo[1,5-a]pyridine-3-carbonitrile (Ex. 15):

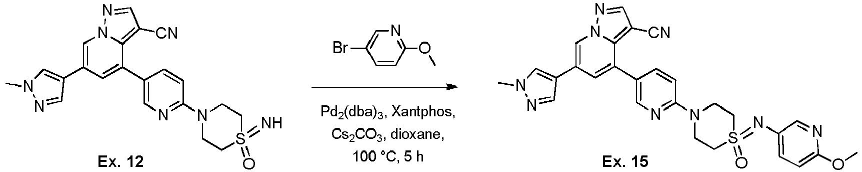

Under nitrogen gas, Pd₂(dba)₃ (21.2 mg, 0.023 mmol) was added to a mixture of 4-(6-(1-imino-1-oxothiomorpholinyl)pyridin-3-yl)-6-(1-methyl-1*H*-pyrazole-4-yl)pyrazolo[1,5-a]pyridine-3-carbonitrile (Ex. 12, 200 mg, 0.462 mmol), 5-bromo-2-methoxypyridine (87 mg, 0.462 mmol), CS₂CO₃ (452 mg, 1.39 mmol) and Xantphos (26.8 mg, 0.046 mmol) in dioxane (5 mL). The resulting mixture was stirred at 100°C for 5 h, and LC-MS showed that the reaction was completed. After cooling to room temperature, the mixture was filtered, and the filtrate was concentrated under reduced pressure to obtain a residue, which was purified by preparative HPLC (column: Phenomenex luna C18 150^{∗}40 mm^{∗}15 um; mobile phase: [water (0.225% formic acid)-acetonitrile]; B%: 20% to 50%, 9 minutes) to obtain 4-(6-(1-((6-methoxypyridin-3-yl)imino)-1-oxothiomorpholinyl)pyridin-3-yl)-6-(1-methyl-1*H* pyrazole-4-yl)pyrazolo[1,5-a]pyridine-3-carbonitrile (Ex.15, 60 mg, yield: 23%), as a yellow solid. Analytical data: ¹H-NMR (400 MHz, CDCl₃):δ = 8.67 (d, *J* = 1.6 Hz, 1 H), 8.42 (d, *J =* 2.4 Hz, 1 H), 8.28 (s, 1 H), 8.00 (d, *J* = 2.4 Hz, 1 H), 7.81-7.85 (m, 2 H), 7.71 (s, 1 H), 7.42-7.45 (m, 2 H), 6.91 (d, *J* = 8.8 Hz, 1 H), 6.68 (d, *J* = 8.4 Hz, 1 H), 4.45-4.49 (m, 2 H), 4.03-4.07 (m, 2 H), 4.01 (s, 3 H), 3.91 (s, 3 H), 3.34-3.36 (m, 2 H), 3.21-3.26 (m, 2 H). Mass spectrum (ESI) *m*/*z:* 540.3 (M+H)⁺.

### Example 16: 4-(6-(4-((dimethyloxo-λ⁴-sulfinyl)amino)piperidin-1-yl)pyridin-3-yl)-6-(1-methyl-1H-pyrazole-4-yl)pyrazolo[1,5-a]pyridine-3-carbonitrile (Ex. 16):

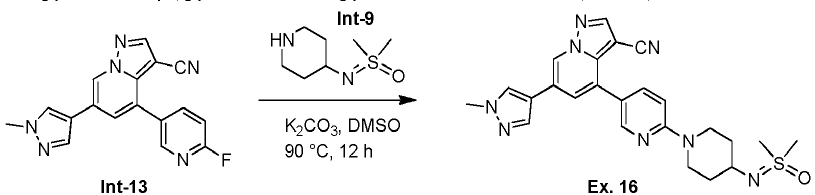

A mixture of *N*-(dimethyloxo-λ⁴-sulfinyl)-4-piperidinamine (Int-9, 166 mg, 0.942 mmol), 4-(6-fluoropyridin-3-yl)-6-(1-methyl-1H-pyrazole-4-yl)pyrazolo[1,5-a]pyridine-3-carbonitrile (Int-13, 300 mg, 0.942 mmol) and K₂CO₃ (261 mg, 1.88 mmol) in DMSO (3 mL) was stirred at 90°C for 12 h, and LC-MS showed that the reaction was completed. After cooling to room temperature, the solid product was collected by filtration, and purified by preparative HPLC (column: Phenomenex luna C18 150^{∗}40 mm^{∗}15 um; mobile phase: [water (0.225% formic acid)-acetonitrile]; B%: 1% to 25%, 9 minutes) to obtain 4-(6-(4-((dimethyloxo-λ⁴-sulfinyl)amino)piperidin-1-yl)pyridin-3-yl)-6-(1-methyl-1*H-*pyrazole-4-yl)pyrazolo[1,5-a]pyridine-3-carbonitrile (Ex. 16, 25 mg, yield: 5.4%), as a light brown solid. Analytical data: ¹H-NMR (400 MHz, CDCl₃):δ = 8.63 (s, 1 H), 8.36 (d, *J =* 2.4 Hz, 1 H), 8.26 (s, 1 H), 7.79 (s, 1 H), 7.73 (dd, *J* = 2.4, 8.8 Hz, 1 H), 7.69 (s, 1 H), 7.39 (d, *J* = 1.6 Hz, 1 H), 6.81 (d, *J* = 8.8 Hz, 1 H), 4.28 (d*, J* = 13.2 Hz, 2 H), 4.00 (s, 3 H), 3.50 (s, 1 H), 3.15 (t, *J=* 10.8 Hz, 2 H), 3.07 (s, 6 H), 1.93 (dd, *J* = 3.6, 13.2 Hz, 2 H), 1.68-1.75 (m, 2 H). Mass spectrum (ESI) *m*/*z:* 475.3 (M+H)⁺.

### Example 17: 4-(6-(4-(((S)-S-methyl-S-phenyl-oxo-λ⁴-sulfinyl)amino)piperidin-1-yl)pyridin-3-yl)-6-(1-methyl-1H-pyrazole-4-yl)pyrazolo[1,5-a]pyridine-3-carbonitrile (Ex. 17):

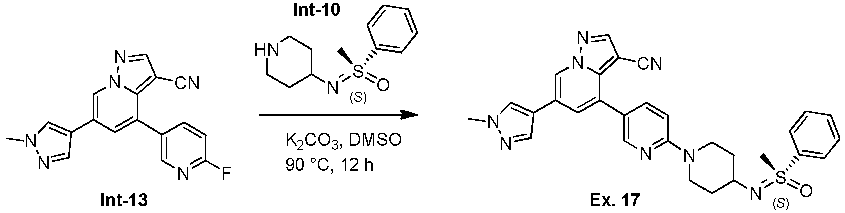

A mixture of *N*-((*S*)-methyl-oxo-phenyl-λ⁴-sulfinyl)-4-piperidinamine (Int-10, 225 mg, 0.942 mmol), 4-(6-fluoropyridin-3-yl)-6-(1-methyl-1*H*-pyrazole-4-yl)pyrazolo[1,5-a]pyridine-3-carbonitrile (Int-13, 100 mg, 0.314 mmol) and K₂CO₃ (174 mg, 1.26 mmol) in DMSO (2 mL) was stirred at 90°C for 12 h, and LC-MS showed that the reaction was completed. The solid product was collected by filtration, and purified by preparative HPLC (column: Phenomenex Synergi C18 150^{∗}25^{∗}10 um; mobile phase: [water (0.225% formic acid)-acetonitrile]; B%: 12% to 42%, 10 minutes) to obtain 4-(6-(4-(((*S*)-*S*-methyl-*S*-phenyl-oxo-λ⁴-sulfinyl)amino)piperidin-1-yl)pyridin-3-yl)-6-(1-methyl-1*H-*pyrazole-4-yl)pyrazolo[1,5-a]pyridine-3-carbonitrile (Ex. 17, 44 mg, yield: 26%), as a white solid. Analytical data: ¹H-NMR (400 MHz, CDCl₃):δ = 8.62 (d, *J* = 1.6 Hz, 1 H), 8.34 (d, *J* = 2.4 Hz, 1 H), 8.26 (s, 1 H), 7.95-8.01 (m, 2 H), 7.79 (s, 1 H), 7.57-7.73 (m, 5 H), 7.38 (d, *J* = 1.6 Hz, 1 H), 6.77 (d, *J* = 8.8 Hz, 1 H), 4.23 (d, *J* = 4.0 Hz, 2 H), 4.00 (s, 3 H), 3.22 (m, 1 H), 3.11 (s, 3 H), 3.01-3.11 (m, 2 H), 1.97-2.00 (m, 1 H), 1.72-1.84 (m, 3 H). Mass spectrum (ESI) *m*/*z:* 537.1 (M+H)⁺.

### Example 18: 4-(6-(4-(((R)-S-methyl-S-phenyl-oxo-λ⁴-sulfinyl)amino)piperidin-1-yl)pyridin-3-yl)-6-(1-methyl-1H-pyrazole-4-yl)pyrazolo[1,5-a]pyridine-3-carbonitrile (Ex. 18):

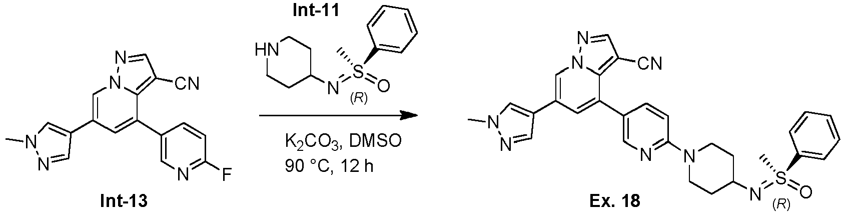

A mixture of *N*-((*R*)-methyl-oxo-phenyl-λ⁴-sulfinyl)-4-piperidinamine (Int-11, 225 mg, 0.942 mmol), 4-(6-fluoropyridin-3-yl)-6-(1-methyl-1*H*-pyrazole-4-yl)pyrazolo[1,5-a]pyridine-3-carbonitrile (Int-13, 100 mg, 0.314 mmol) and K₂CO₃ (217 mg, 1.57 mmol) in DMSO (2 mL) was stirred at 90°C for 12 h, and LC-MS showed that the reaction was completed. After cooling to room temperature,the solid product was collected by filtration and purified by preparative HPLC (column: Phenomenex Synergi C18 150^{∗}25^{∗}10 um; mobile phase: [water (0.225% formic acid)-acetonitrile]; B%: 12% to 42%, 10 minutes) to obtain 4-(6-(4-(((*R*)-*S*-methyl-*S*-phenyl-oxo-λ⁴-sulfinyl)amino)piperidin-1-yl)pyridin-3-yl)-6-(1-methyl-1*H*-pyrazole-4-yl)pyrazolo[1,5-a]pyridine-3-carbonitrile (Ex. 18, 27 mg, yield: 16%), as a white solid. Analytical data: ¹H-NMR (400 MHz, CDCl₃):δ = 8.62 (d, *J* = 1.6 Hz, 1 H), 8.33 (d, *J* = 2.4 Hz, 1 H), 8.25 (s, 1 H), 7.96-7.98 (m, 2 H), 7.79 (s, 1 H), 7.57-7.73 (m, 5 H), 7.38 (d, *J* = 1.6 Hz, 1 H), 6.77 (d, *J* = 8.8 Hz, 1 H), 4.23 (td, *J* = 4.8, 9.2 Hz, 2 H), 4.00 (s, 3 H), 3.21 (m, 1 H), 3.11 (s, 3 H), 2.95-3.08 (m, 2 H), 1.92-2.07 (m, 1 H), 1.72-1.86 (m, 3 H). Mass spectrum(ESI) *m*/*z*:537.1 (M+H)⁺.

### Example 19: tert-butyl (4-(5-(3-cyano-6-(1-methyl-1H-pyrazole-4-yl)pyrazolo[1,5-a]pyrazine-4-yl)pyridin-2-yl)-1-oxothiomorpholine-1-imino)carboxylate (Ex. 19):

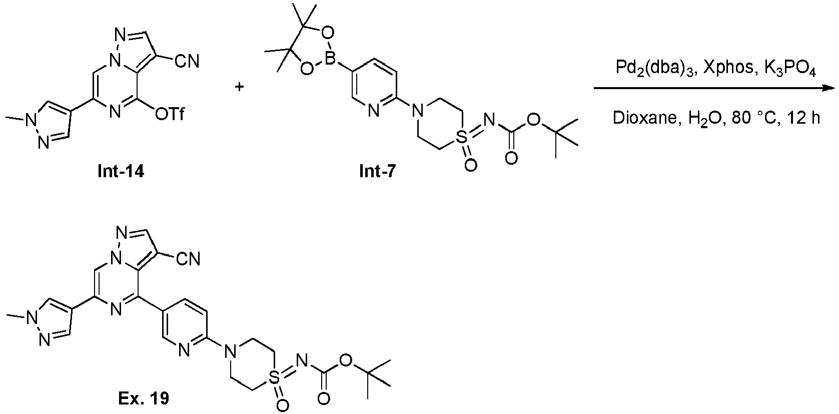

Under nitrogen gas, Pd₂(dba)₃ (24.6 mg, 0.0269 mmol) and XPhos (25.6 mg, 0.0537 mmol) were added to a mixture of 3-cyano-6-(1-methyl-1*H*-pyrazole-4-yl)pyrazolo[1,5-a]pyrazine-4-yl trifluoromethanesulfonate (Int-14, 0.20 g, 0.537 mmol) and tert-butyl (1-oxo-4-(5-(4,4,5,5-tetramethyl-1,3,2-dioxaborolane-2-yl)pyridin-2-yl)thiomorpholine-1-imino)carboxylate (Int-7, 258 mg, 0.591 mmol) in aqueous K₃PO₄ solution (2.0 M, 0.806 mL, 1.61 mmol) and dioxane (5 mL). The resulting mixture was stirred at 80°C for 12 h, and LC-MS showed that the reaction was completed. After cooling to room temperature, the mixture was filtered, and the filtrate was concentrated under reduced pressure to obtain a residue, which was purified by silica gel column chromatography (eluent: DCM: ethyl acetate = 15:1 to 5:1, and then DCM: MeOH = 50:1) to obtain tert-butyl (4-(5-(3-cyano-6-(1-methyl-1H-pyrazole-4-yl)pyrazolo[1,5-a]pyrazine-4-yl)pyridin-2-yl)-1-oxothiomorpholine-1-imino)carboxylate (Ex. 19, 110 mg, 0.206 mmol, yield: 38%), as a yellow solid. Analytical data: ¹H-NMR (400 MHz, CDCl₃): δ = 8.83 (d, *J* = 2.8 Hz, 1 H), 8.58 (s, 1 H), 8.36 (s, 1 H), 8.16 (s, 1 H), 7.87-8.01 (m, 2 H), 6.94 (d, *J* = 8.8 Hz, 1 H), 4.47 (dd, *J* = 4.0, 14.4 Hz, 2 H), 4.32-4.34 (m, 2 H), 4.01 (s, 3 H), 3.68-3.71 (m, 2 H), 3.34-3.39 (m, 2 H), 1.51 (s, 9 H). Mass spectrum (ESI) *m*/*z:* 434.2 (M+H-100)⁺.

### Example 20: 4-(6-(1-imino-1-oxothiomorpholinyl)pyridin-3-yl)-6-(1-methyl-1H-pyrazole-4-yl)pyrazolo[1,5-a]pyrazinee-3-carbonitrile (Ex. 20):

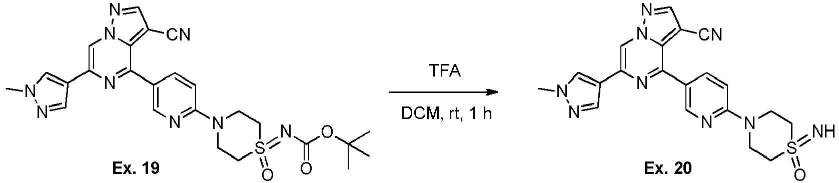

Trifluoroacetic acid (1 mL, 13.5 mmol) was added to a solution of tert-butyl (4-(5-(3-cyano-6-(1-methyl-1H-pyrazole-4-yl)pyrazolo[1,5-a]pyrazine-4-yl)pyridin-2-yl)-1-oxothiomorpholine-1-imino)carboxylate (Ex. 19, 100 mg, 0.187 mmol) in DCM (3 mL). The resulting mixture was stirred at room temperature for 1 h, and TLC showed that the reaction was completed. The reaction mixture was concentrated under reduced pressure to obtain a residue, which was purified by preparative HPLC (column: Phenomenex Synergi C18 150^{∗}25^{∗}10 um; mobile phase: [water (0.225% formic acid)-acetonitrile]; B%: 17% to 41%, 8 minutes) to obtain 4-(6-(1-imino-1-oxothiomorpholinyl)pyridin-3-yl)-6-(1-methyl-1H-pyrazole-4-yl)pyrazolo[1,5-a]pyrazine-3-carbonitrile (Ex. 20, 35 mg, yield: 43%), as a white solid. Analytical data: ¹H-NMR (400 MHz, DMSO-*d*₆):δ = 9.33 (s, 1 H), 8.83 (s, 1 H), 8.71 (d, *J* = 2.4 Hz, 1 H), 8.38 (s, 1 H), 8.11-8.14 (m, 2 H), 7.21 (d, *J* = 8.8 Hz, 1 H), 4.34 (dd, *J* = 1.6, 14.8 Hz, 2 H), 3.81-3.99 (m, 6 H), 2.96-3.11 (m, 4 H). Mass spectrum (ESI) *m*/*z:* 434.1 (M+H)⁺.

### Example 21: 4-(6-(1-(((6-methoxypyridin-3-yl)methyl)imino)-1-oxothiomorpholinyl)pyridin-3-yl)-6-(1-methyl-1H-pyrazole-4-yl)pyrazolo[1,5-a]pyrazine-3-carbonitrile (Ex. 21):

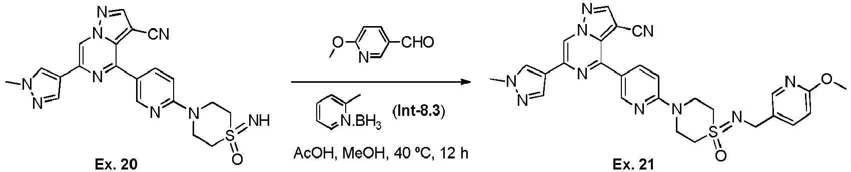

2-Methylpyridin-boronic acid complex (Int-8.3, 180 mg, 1.68 mmol) was added to a solution of 4-(6-(1-imino-1-oxothiomorpholinyl)pyridin-3-yl)-6-(1-methyl-1*H*-pyrazole-4-yl)pyrazolo[1,5-a]pyrazine-3-carbonitrile (Ex. 20, 0.50 g, 1.15 mmol), 6-methoxynicotinaldehyde (614 mg, 4.48 mmol) and acetic acid (0.299 mL, 5.23 mmol) in MeOH (10 mL). The resulting mixture was stirred at 40°C for 12 h, and LC-MS showed that the reaction was completed. The reaction mixture was filtered, and the filtrate was concentrated under reduced pressure to obtain a residue, which was purified by preparative HPLC (column: Phenomenex luna C18 150^{∗}40 mm^{∗}15 um; mobile phase: [water (0.225% formic acid)-acetonitrile]; B%: 18% to 48%, 9 minutes) to obtain 4-(6-(1-(((6-methoxypyridin-3-yl)methyl)imino)-1-oxothiomorpholinyl)pyridin-3-yl)-6-(1-methyl-1*H*-pyrazole-4-yl)pyrazolo[1,5-a]pyrazine-3-carbonitrile (Ex. 21, 27 mg, yield: 4.3%), as a yellow solid. Analytical data: ¹H-NMR (400 MHz, CDCl₃):δ = 8.81 (d, *J* = 2.4 Hz, 1 H), 8.57 (s, 1 H), 8.36 (s, 1 H), 8.08-8.21 (m, 2 H), 7.99 (d, *J* = 8.8 Hz, 2 H), 7.68 (dd, *J* = 2.8, 8.8 Hz, 1 H), 6.89 (d, *J* = 8.8 Hz, 1 H), 6.75 (d, *J* = 8.4 Hz, 1 H), 4.31-4.34 (m, 2 H), 4.29 (s, 2 H), 4.00-4.16 (m, 2 H), 4.01 (s, 3 H), 3.94 (s, 3 H), 3.10-3.16 (m, 4 H). Mass spectrum (ESI) *m*/*z:* 555.2 (M+H)⁺.

### Example 22: 4-(6-(1-((6-methoxypyridin-3-yl)imino)-1-oxothiomorpholinyl)pyridin-3-yl)-6-(1-methyl-1H-pyrazole-4-yl)pyrazolo[1,5-a]pyrazine-3-carbonitrile (Ex. 22):

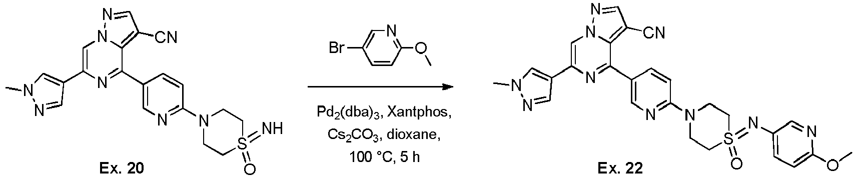

Under nitrogen gas, Xantphos (52.8 mg, 0.091 mmol) and Pd₂(dba)₃ (41.8 mg, 0.0457 mmol) were added to a suspension of 4-(6-(1-imino-1-oxothiomorpholinyl)pyridin-3-yl)-6-(1-methyl-1*H*-pyrazole-4-yl)pyrazolo[1,5-a]pyrazine-3-carbonitrile (Ex. 20, 0.50 g, 0.913 mmol), 5-bromo-2-methoxypyridine (172 mg, 0.913 mmol) and CS₂CO₃ (892 mg, 2.74 mmol) in dioxane (5 mL). The resulting mixture was stirred at 100°C for 5 h, and LC-MS showed that the reaction was completed. The reaction mixture was filtered, and the filtrate was concentrated under reduced pressure to obtain a residue, which was purified by preparative HPLC (column: Phenomenex luna C18 150^{∗}40 mm^{∗} 15 um; mobile phase: [water (0.225% formic acid)-acetonitrile]; B%: 22% to 52%, 9 minutes) to obtain 4-(6-(1-((6-methoxypyridin-3-yl)imino)-1-oxothiomorpholinyl)pyridin-3-yl)-6-(1-methyl- 1*H*-pyrazole-4-yl)pyrazolo[1,5-a]pyrazine-3-carbonitrile (Ex. 22, 52.5 mg, yield: 10%), as a yellow solid. Analytical data: ¹H-NMR (400 MHz, CDCl₃): δ = 8.83 (d, *J* = 2.4 Hz, 1 H), 8.58 (s, 1 H), 8.37 (s, 1 H), 8.15 (dd, *J* = 2.4, 8.8 Hz, 1 H), 7.93-8.07 (m, 3 H), 7.43 (dd, *J* = 2.8, 8.8 Hz, 1 H), 6.93 (d, *J* = 8.8 Hz, 1 H), 6.68 (d, *J* = 8.8 Hz, 1 H), 4.52 (d, *J* = 16.0 Hz, 2 H), 4.08 (dd, *J* = 9.6, 12.8 Hz, 2 H), 4.01 (s, 3 H), 3.91 (s, 3 H), 3.38 (dd, *J* = 2.4, 12.0 Hz, 2 H), 3.15-3.27 (m, 2 H). Mass spectrum (ESI) *m*/*z:* 541.2 (M+H)⁺.

### Example 23: 4-(6-(4-((dimethyloxo-λ⁴-sulfinyl)amino)piperidin-1-yl)pyridin-3-yl)-6-(1-methyl-1H-pyrazole-4-yl)pyrazolo[1,5-a]pyrazine-3-carbonitrile (Ex. 23):

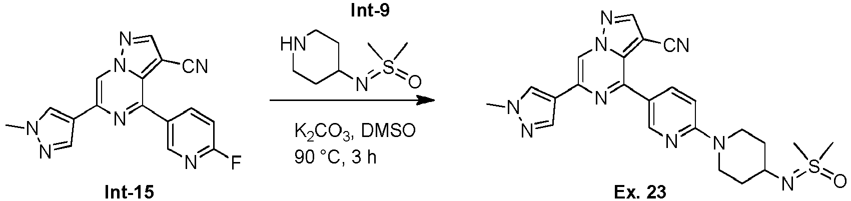

A mixture of *N*-(dimethyloxo-λ⁴-sulfinyl)-4-piperidinamine (Int-9, 51.6 mg, 0.292 mmol), 4-(6-fluoropyridin-3-yl)-6-(1-methyl-1H-pyrazole-4-yl)pyrazolo[1,5-a]pyrazine-3-carbonitrile (Int-15, 100 mg, 0.266 mmol) and K₂CO₃ (73.5 mg, 0.532 mmol) in DMSO (2 mL) was stirred at 90°C for 3 h, and LC-MS showed that the reaction was completed. The solid product was collected by filtration, and purified by preparative HPLC (column: Shim-pack C18 150^{∗}25^{∗}10 um; mobile phase: [water (0.225% formic acid)-acetonitrile]; B%: 11% to 31%, 10 minutes) to obtain 4-(6-(4-((dimethyloxo-λ⁴-sulfinyl)amino)piperidin-1-yl)pyridin-3-yl)-6-(1-methyl-1H-pyrazole-4-yl)pyrazolo[1,5-a]pyrazine-3-carbonitrile (Ex. 23, 27 mg, yield: 21%), as a yellow solid. Analytical data: ¹H-NMR (400 MHz, DMSO-*d*₆):δ = 9.28 (s, 1 H), 8.82 (s, 1 H), 8.64 (d, *J=* 2.3 Hz, 1 H), 8.37 (s, 1 H), 8.13 (s, 1 H), 8.02 (dd, *J* = 2.5, 8.9 Hz, 1 H), 6.98 (d, *J* = 8.8 Hz, 1 H), 4.13-4.22 (m, 2 H), 3.90 (s, 3 H), 3.31 (s, 3 H), 3.15 (s, 6 H), 1.65-1.82 (m, 2 H), 1.38-1.54 (m, 2 H). Mass spectrum (ESI) *m*/*z:* 476.2 (M+H)⁺.

### Example 24: 4-(6-(4-(((S)-S-methyl-S-phenyl-oxo-λ⁴-sulfinyl)amino)piperidin-1-yl)pyridin-3-yl)-6-(1-methyl-1H-pyrazole-4-yl)pyrazolo[1,5-a]pyrazine-3-carbonitrile (Ex. 24):

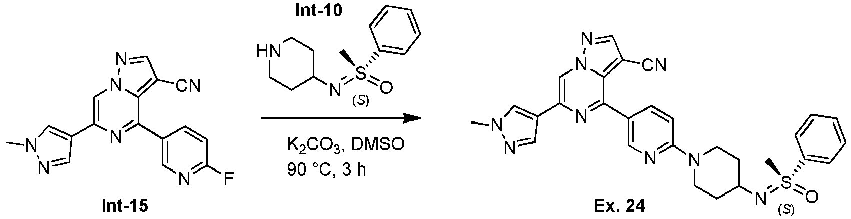

Compound Ex.24 was prepared by the method as described in Example 23. Analytical data: ¹H-NMR (400 MHz, DMSO-*d*₆):δ = 9.28 (s, 1 H), 8.82 (s, 1 H), 8.64 (d, *J* = 2.4 Hz, 1 H), 8.37 (s, 1 H), 8.13 (s, 1 H), 8.01 (dd, *J* = 2.8, 8.8 Hz, 1 H), 7.91-7.93 (m, 2 H), 7.65-7.70 (m, 3 H), 6.98 (d, *J* = 8.8 Hz, 1 H), 4.16 (m, 2 H), 3.90 (s, 3 H), 3.31 (s, 3 H), 3.15 (s, 3 H), 3.10-3.20 (m, 2 H), 1.79-1.83 (m, 1 H), 1.64-1.68 (m, 1 H), 1.41-1.54 (m, 2 H). Mass spectrum (ESI) *m*/*z:* 538.1 (M+H)⁺.

### Example 25: 4-(6-(4-(((R)-S-methyl-S-phenyl-oxo-λ⁴-sulfinyl)amino)piperidin-1-yl)pyridin-3-yl)-6-(1-methyl-1H-pyrazole-4-yl)pyrazolo[1,5-a]pyrazine-3-carbonitrile (Ex. 25):

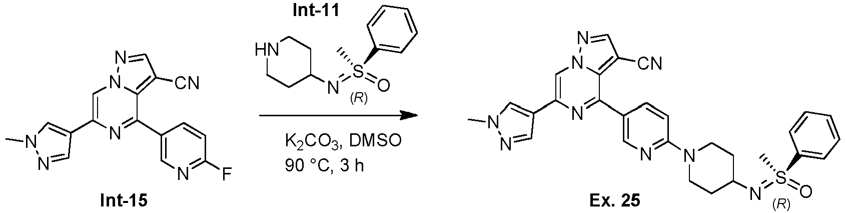

Compound Ex.25 was prepared by the method as described in Example 23. Analytical data: ¹H-NMR (400 MHz, CDCl₃):δ = 8.78 (d, *J* = 2.4 Hz, 1 H), 8.51 (s, 1 H), 8.33 (s, 1 H), 7.96-8.01 (m, 5 H), 7.61-7.66 (m, 3 H), 6.77 (d, *J* = 8.8 Hz, 1 H), 4.26-4.32 (m, 2 H), 3.99 (s, 3 H), 3.24 (m, 1 H), 3.08-3.15 (m, 2 H), 3.13 (s, 3 H), 1.97 (m, 1 H), 1.61-1.78 (m, 3 H). Mass spectrum (ESI) *m*/*z:* 538.0 (M+H)⁺.

### Example 26: Determination of biochemical IC₅₀ values of RET kinase and its mutants and fusions:

The determination of biochemical inhibitory activity of the compounds prepared in the Examples of the present invention on wild-type RET kinase, RET(V804M) mutant and RET-CCDC6 fusions was entrusted to American Reaction Biology Corporation (One Great Valley Parkway, Suite 2, Malvern, PA 19355, USA). For detailed test methods, the following references were referred to: T. Anastassiadis, S. W. Deacon, K. Devarajan, H. Ma, J. R. Peterson. Comprehensive assay of kinase catalytic activity reveals features of kinase inhibitor selectivity. Nat. Biotech. 2011, 29(11), 1039-1045.

### I. General conditions for determination:

The compounds of the present invention were dissolved in dimethyl sulfoxide (DMSO) to prepare 10 mM stock solutions, and ten different doses by 3-fold serial dilutions from 10 mM were prepared. The ATP concentration used in this determination was 1 µM.

### II. Reagents used:

Base reaction buffer: 20 mM 4-(2-hydroxyethyl)-1-piperazineethanesulfonic acid buffer (Hepes, pH 7.5), 10 mM magnesium chloride, 1 mM ethylene glycol-bis(β-aminoethylether)-N,N,N',N'-tetraacetic acid (EGTA), 0.02% BrijTM-35 (purchased from ThermoFisher Scientific), 0.02 mg/mL bovine serum albumin (BSA), 0.1 mM sodium orthovanadate (Na₃VO₄), 2 mM dithiothreitol (DTT), 1% dimethyl sulfoxide (DMSO).

### III. Reaction steps:

a) adding the corresponding substrate to a freshly prepared base reaction buffer;
b) adding necessary cofactors;
c) adding kinase and shake gently;
d) adding the compounds in DMSO using Acoustic technology (Echo550; nanoliter range) and incubating at room temperature for 20 minutes;
e) adding 33P-ATP (specific activity 10 µCi/µL) to initiate the reaction;
f) incubating the kinase reaction at room temperature for 2 hours;
g) spotting the resultant of reaction on P81 ion exchange paper (purchased from Whatman);
h) detecting kinase activity by filter binding assay.

### IV. Acquiring and processing data:

The filter was washed extensively with 0.75% phosphoric acid to remove unbound phosphate, and after subtracting the background derived from the control reaction containing the inactive enzyme, the kinase activity was expressed as: the remaining kinase activity of the compound test group accounted for a percent of blank test control group (DMSO). IC₅₀ values were generated using Prism (GraphPad software).

### V. Measurement results:

The biochemical IC₅₀ values of the compounds prepared in the Examples of the present invention against wild-type RET kinases, RET(V804M) mutants and RET-CCDC6 fusions were listed in Table 1:

**Table 1**

| Example | Biochemical IC₅₀ (nM) | | |
|---|---|---|---|
| | RET | RET (V804M) | RET -CCDC6 |
| Ex. 2 | 4.23 | 21.2 | 2.81 |
| Ex. 3 | 1.66 | 3.78 | 0.993 |
| Ex. 4 | 6.74 | 55.7 | 4.59 |
| Ex. 5 | 3.62 | 17.9 | 2.09 |
| Ex. 7 | 18.1 | 165 | 12.6 |
| Ex. 8 | 9.45 | 63.6 | 5.89 |
| Ex. 9 | 14.2 | 138 | 8.14 |
| Ex. 10 | 6.18 | 36.0 | 4.59 |
| Ex. 12 | 0.849 | 5.55 | 0.523 |
| Ex. 13 | 0.89 | 5.10 | 0.416 |
| Ex. 14 | 0.657 | 5.21 | 0.336 |
| Ex. 15 | 0.759 | 1.72 | 0.518 |
| Ex. 16 | 1.16 | 5.55 | 0.619 |
| Ex. 17 | 1.49 | 10.6 | 0.776 |
| Ex. 18 | 0.907 | 4.72 | 0.501 |
| Ex. 20 | 1.16 | 7.91 | 0.677 |
| Ex. 21 | 1.02 | 7.83 | 0.528 |
| Ex. 22 | 0.673 | 3.24 | 0.410 |
| Ex. 23 | 4.09 | 25.0 | 2.16 |
| Ex. 24 | 4.06 | 26.3 | 1.93 |
| Ex. 25 | 2.02 | 10.6 | 1.15 |

It can be seen from the above data that the compounds in the present invention have strong inhibitory activity on wild-type RET kinase, representative RET (V804M) of mutant RET kinase and representative RET-CCDC6 of fusion RET kinase, in which IC₅₀ value is less than 100 nM, and individual results were less than 1 nM, reaching the picomolar scale.

It should be noted that there are many mutants of RET kinase, and multiple fusion proteins can also be formed. These mutants and fusion proteins include, but not limited to, RET(A883F), RET(E762Q), RET(G691S), RET(L790F), RET(M918T), RET(R749T), RET(R813Q), RET(R912P), RET(S891A), RET(S904A), RET(S904F), RET(V804M), RET(V778I), RET(V804E), RET(V804L), RET-KIF5B, RET(G810R)-KIF5B, RET(V804L)-KIF5B, RET(V804M)-KIF5B, RET(Y791F), RET(Y806H), RET-BCR, RET-KIF5B(Kex15Rex14), RET-CCDC6, RET-NCOA4(PTC3), RET-PRKAR1A(PTC2). Example 26 determined the inhibitory activity of some compounds of the present invention on the representative RET (V804M) of the mutant RET kinase and the representative RET-CCDC6 of the fusion RET kinase. These results are not used to limit the protection scope of the present invention. The compounds in the present invention that have inhibitory activity against other RET mutants or fusions other than these two types also belong to the protection scope of the present invention.

### Example 27: Determination of IC₅₀ values for Phospho-RET and Phospho-ERK in tumor cells:

The determination of inhibitory activity of the compounds prepared in the examples of the present invention on Phospho-RET and Phospho-ERK in tumor cells was entrusted to the American Reaction Biology Corporation (Address: One Great Valley Parkway, Suite 2, Malvern, PA 19355, USA).

### I. General conditions for determination:

The compounds of the present invention were dissolved in dimethyl sulfoxide (DMSO) to prepare 10 mM stock solutions, and ten different doses by 3-fold serial dilutions from 10 mM were prepared. An ELISA assay format was used in the test.

### II. Reagents and cell culture:

PathScan^{®} Phospho-Ret (panTyr) and p44/42 MAPK (Thr202/Tyr204) (ERK) Sandwich ELISA kits were purchased from Cell Signaling Technology. The LC-2/ad lung cancer cell line was purchased from Sigma-Aldrich. The LC-2/ad cells were cultured in RPMI-1640 : F-12K (1:1). The medium was supplemented with 10% fetal bovine serum (FBS), 100 µg/mL penicillin and 100 µg/mL streptomycin, and the cultures were maintained at 37°C in a humidified atmosphere of 5% CO₂ and 95% air.

### III. Reaction steps:

a) 1.5×10⁴ LC-2/ad cells were seeded in the wells of a 96-well tissue culture plate overnight;
b) the cells were treated with DMSO or the test compound in DMSO (the initial concentration was 1 µM, and ten doses by 3-fold serial dilutions) for one hour;
c) the cells were washed once with 200 µL ice-cold phosphate buffered saline (PBS) and washed with 100 µL/well of cell lysis buffer (1% NP-40, 20 mM Tris/pH = 8.0, 137 mM NaCl, 10% glycerol, 2 mM EDTA, protease/phosphatase inhibitor mixture). The plate was shaken for 1 minute, then placed on a rocking platform at 4°C for 30 minutes;
d) 80 µL/well of lysate was added to the appropriate wells of each ELISA plate and the plate was incubated overnight at 4°C on a rocking platform;
e) the contents of the plate were discarded into a container and the wells were washed for four times with 200 µL/well of IX ELISA wash buffer;
f) 100 µL of reconstituted Phospho-Ret(panTyr) or Phospho-ERK(Thr202/Tyr204; p44/42MAPK) detection antibody was added to the appropriate wells and the plate was incubated at 37°C for 1 hour;
g) the washing procedure was repeated and 100 µL of reconstituted HRP-linked secondary antibody was added to each well of two plates and the plates were incubated at 37°C for 30 minutes;
h) the washing procedure was repeated, 100 µL of TMB substrate was added to each well, and the plates were incubated at 37°C for 45 minutes;
i) 100 µL of STOP solution was added to each well and the plate was gently shaken for a few seconds.

### IV. Acquiring and processing data:

Absorbance was read at 460 nm using an Envision 2104 Multilabel Reader (purchased from PerkinElmer, Santa Clara, CA, USA); IC₅₀ curves were plotted and IC₅₀ values were calculated based on a sigmoidal dose-response equation using GraphPad Prism 4 software.

### V. Measurement results:

The IC₅₀ values of the compounds prepared in the examples of the present invention to Phospho-RET and Phospho-ERK in LC-2/ad lung cancer tumor cells were listed in Table 2:

**Table 2**

| Example | Cell IC₅₀(nM) LC-2/ad lung cancer cells | |
|---|---|---|
| | Phospho-RET | Phospho-ERK |
| LOXO292^{∗} | 6.08 | >1000 |
| Ex. 12 | 11.5 | 246 |
| Ex. 13 | 16.3 | 6.55 |
| Ex. 14 | 11.5 | 82.5 |
| Ex. 15 | 0.51 | 321 |
| Ex. 16 | 66.4 | 7.19 |
| Ex. 18 | 7.10 | 6.83 |
| Ex. 20 | 91.4 | 7.77 |
| Ex. 21 | 21.3 | 6.42 |
| Ex. 25 | 84.7 | 9.27 |

LOXO292^{∗} is a RET inhibitor in clinical trials and is used here as a positive control.

From the data listed in Table 2, it can be seen that some of the compounds synthesized in the Examples of the present invention have a strong inhibitory effect on Phospho-RET and Phospho-ERK in tumors cells, in which IC₅₀ value is less than 100 nM, and individual results were less than 1 nM, reaching the picomolar scale. Interestingly, compared with the positive control LOXO292, the compounds in the present invention have much stronger inhibitory activity on Phospho-ERK, wherein the IC₅₀ value of the former is >1000 nM, while IC₅₀ values of the compounds of the present invention are mostly lower than single digit nM. This also shows that the compounds in the present invention have obvious advantages over LOXO292 in inhibiting the key tumor-driven target ERK in tumor cells.

Although the present invention has been described in detail with general descriptions, specific embodiments and tests above, some modifications or improvements can be made on the basis of the present invention, which will be obvious to a person skilled in the art. Therefore, these modifications or improvements made without departing from the spirit of the present invention belong to the scope of protection of the present invention.

### Industrial applicability

The present invention provides a heterocyclic sulfoximine compound represented by formula (I), or a racemate, an enantiomer, a pharmaceutically acceptable salt or a solvate thereof. At the same time, also disclosed are an intermediate compound for synthesizing the compound, a preparation method therefor, and a pharmaceutical composition comprising the compound and use thereof. The compound is RET, RET mutant, or RET fusion protein inhibitors, capable of treating diseases caused by abnormal activity of RET, RET mutant, or RET fusion proteins, for example, tumors and the like.

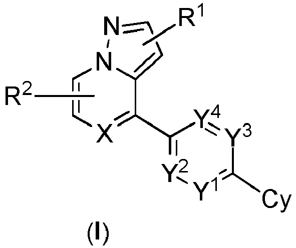

## Claims

1. A heterocyclic sulfoximine compound, or a racemate, an enantiomer, a pharmaceutically acceptable salt or a solvate thereof, a molecular structure of the compound is represented by formula (I): 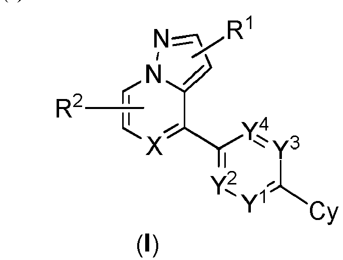 in the formula:
Cy is (Ia) or (Ib): 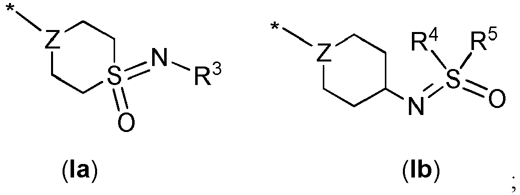
^{∗} represents an attachment point between Cy and the rest of the molecular structure represented by formula (I);
X is N or C-R;
Y¹, Y², Y³ and Y⁴ are the same or different, and each independently represent N or C-R';
Z is N or C-R";
R, R' or R" are the same or different, and each independently selected from hydrogen, deuterium, halogen, CN, OR^{a}, NR^{b}R^{c}, S(=O)wR^{d}, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R, R' or R" are optionally substituted by the same or different deuterium, halogen, CN, NO₂, OH, OCH₃, OCF₃ or CF₃;
R¹ and R² are the same or different, and each independently selected from one or two of the same or different hydrogen, deuterium, halogen, CN, NO₂, OR^{a}, NR^{b}R^{c}, S(=O)wR^{d}, CO₂R^{e}, CONR^{b}R^{c}, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl, wherein one or more hydrogens in the C₁₋₁₂alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl are optionally substituted by the same or different G¹;
R³ is selected from hydrogen, deuterium, S(=O)_{w}R^{d}, S(=O)_{w}NR^{f}R^{g}, C(=O)R^{h}, C(=O)NRⁱR^{j}, C(=O)OR^{k}, C₁₋₁₂alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R³ are optionally substituted by the same or different G²;
R⁴ and R⁵ are the same or different, and each independently selected from hydrogen, deuterium, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R⁴ and R⁵ represent two same or different C₁₋₁₂ alkyl groups, the two same or different C₁₋₁₂ alkyl groups are connected to each other and form a heteroalicycle together with the S atom to which the two same or different C₁₋₁₂ alkyl groups are commonly connected, the heteroalicycle optionally contains one or more additional heteroatoms selected from O, N or S(=O)_{w}; one or more hydrogens in R₄ and R₅ are optionally substituted by the same or different G³;
R^{a}, R^{b}, R^{c}, R^{d}, R^{e}, R^{f}, R^{g}, R^{h}, Rⁱ, R^{j} or R^{k} is each independently selected from hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R^{a}, R^{b}, R^{c}, R^{d}, R^{e}, R^{f}, R^{g}, R^{h}, Rⁱ, R^{j}, or R^{k} are optionally substituted by the same or different G⁴;
R^{b} and R^{c} are the same or different, when R^{b} and R^{c} represent two same or different C₁₋₁₂ alkyl groups connected to the same nitrogen atom, the two same or different C₁₋₁₂ alkyl groups are optionally connected with each other, and form a heteroalicycle together with the nitrogen atom, the heteroalicycle optionally contains one or more additional heteroatoms selected from O, N or S(=O)w;
R^{f} and R^{g} are the same or different, when R^{f} and R^{g} represent two same or different C₁₋₁₂ alkyl groups connected to the same nitrogen atom, the two same or different C₁₋₁₂ alkyl groups are optionally connected with each other, and form a heteroalicycle together with the nitrogen atom, the heteroalicycle optionally contains one or more additional heteroatoms selected from O, N or S(=O)_{w};
Rⁱ and R^{j} are the same or different, when Rⁱ and R^{j} represent two same or different C₁₋₁₂ alkyl groups connected to the same nitrogen atom, the two same or different C₁₋₁₂ alkyl groups are optionally connected with each other, and form a heteroalicycle together with the nitrogen atom, the heteroalicycle optionally contains one or more additional heteroatoms selected from O, N or S(=O)w;
G¹, G², G³ and G⁴ are the same or different, and each independently selected from one or more same or different deuterium, halogen, CN, NO₂, OH, OCF₃, CF₃, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, C₅₋₁₂ heteroaryl, R⁶O-, R⁷R⁸N-, R⁶S(=O)_{W}-, R⁷R⁸NS(=O)_{W}-, R⁶C(=O)-, R⁷R⁸NC(=O)-, R⁶OC(=O)-, R⁶C(=O)O-, R⁷R⁸NC(=O)O-, R⁶C(=O)NR⁹-, R⁷R⁸NC(=O)NR⁹-, R⁶OC(=O)NR⁹-, R⁶S(=O)_{W}NR⁹-, R⁷R⁸NS(=O)_{W}NR⁹-, R⁷R⁸NC(=NR¹⁰)NR⁹-, R⁷R⁸NC(=CHNO₂)NR⁹-, R⁷R^{S}NC(=N-CN)NR⁹-, R⁷R⁸NC(=NR¹⁰)-, R⁶S(=O)(=NR¹⁰)NR⁹-, or R⁷R⁸NS(=O)(=NR¹⁰)-, wherein one or more hydrogens in the C₁₋₁₂alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl are optionally substituted by the same or different deuterium, halogen, CN, NO₂, OH, OCF₃, CF₃, C₁₋₁₂alkyl, C₃₋₁₂ cycloalkyl, C₃₋₁₂ heteroalicyclyl, C₂₋₁₂ alkenyl, C₂₋₁₂ alkynyl, C₆₋₁₂ aryl, C₅₋₁₂ heteroaryl, R⁶O-, R⁷R⁸N-, R⁶S(=O)_{W}-, R⁷R⁸NS(=O)_{W}-, R⁶C(=O)-, R⁷R⁸NC(=O)-, R⁶OC(=O)-, R⁶C(=O)O-, R⁷R⁸NC(=O)O-, R⁶C(=O)NR⁹-, R⁷R^{S}NC(=O)NR⁹-, R⁶OC(=O)NR⁹-, R⁶S(=O)_{W}NR⁹-, R⁷R⁸NS(=O)_{W}NR⁹-, R⁷R⁸NC(=NR¹⁰)NR⁹-, R⁷R⁸NC(=CHNO₂)NR⁹-, R⁷R⁸NC(=N-CN)NR⁹-, R⁷R⁸NC(=NR¹⁰)-, R⁶S(=O)(=NR¹⁰)NR⁹- or R⁷R^{S}NS(=O)(=NR¹⁰)-;
R⁶, R⁷, R⁸, R⁹ and R¹⁰ are the same or different, and each independently selected from hydrogen, deuterium, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R⁷ and R⁸ are two same or different C₁₋₁₂ alkyl groups connected to the same nitrogen atom, the two same or different C₁₋₁₂ alkyl groups are connected to each other, and form a heteroalicycle together with the nitrogen atom, the heteroalicycle optionally comprises one or more additional heteroatoms selected from O, N or S(=O)w; and one or more hydrogens in R⁶, R⁷, R⁸, R⁹ and R¹⁰ are further optionally substituted by the same or different deuterium, halogen, OH, CN, NO₂, OCH₃, OCF₃, C₁₋₁₂ alkyl or C₃₋₁₂ cycloalkyl; and
w is 0, 1 or 2.

2. The heterocyclic sulfoximine compound, or a racemate, an enantiomer, a pharmaceutically acceptable salt or a solvate thereof according to claim 1, wherein the structural formula of the compound is (IIa) or (IIb): 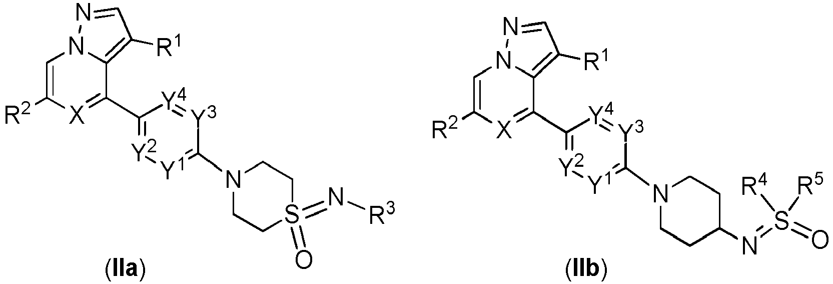 wherein, X, Y¹, Y², Y³, Y⁴, R¹, R², R³, R⁴, and R⁵ have the same definitions as described in claim 1.

3. The heterocyclic sulfoximine compound, or a racemate, an enantiomer, a pharmaceutically acceptable salt or a solvate thereof according to claim 1 or 2, wherein, the structural formula of the compound is (IIIa), (IIIb), (IIIc) or (IIId): 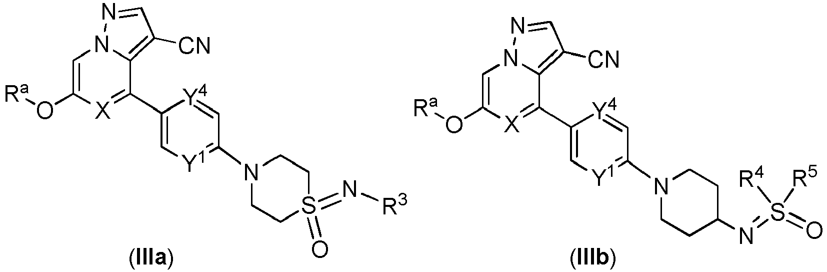 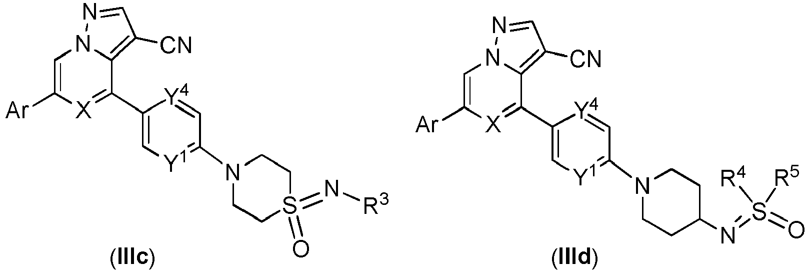 wherein,
X is N or C-R;
Y¹ and Y⁴ are the same or different N or C-R'; R^{a} is selected from hydrogen, C₁₋₁₂alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl;
one or more hydrogens in R^{a} are optionally substituted by the same or different G¹;
R and R' are each independently selected from hydrogen, deuterium, halogen, CN, C₁₋₁₂ alkyl, OH or C₁₋₁₂ alkyl-O-, one or more hydrogens in the C₁₋₁₂ alkyl groups are optionally substituted by the same or different deuterium, halogen, CN, NO₂, OH, OCH₃, OCF₃ or CF₃;
Ar represents C₆₋₁₂ aryl or C₅₋₁₂ heteroaryl; one or more hydrogens in Ar are optionally substituted by the same or different G¹; and
R³, R⁴, R⁵ and G¹ have the same definitions as described in claim 1.

4. The heterocyclic sulfoximine compound, or a racemate, an enantiomer, a pharmaceutically acceptable salt or a solvate thereof according to any one of claims 1 to 3, wherein, the structural formula of the compound is (IVa), (IVb), (IVc), (IVd), (IVe), (IVf), (IVg) or (IVh): 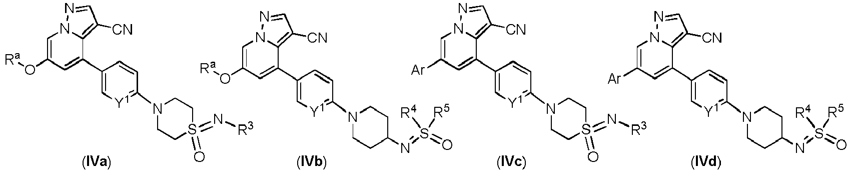 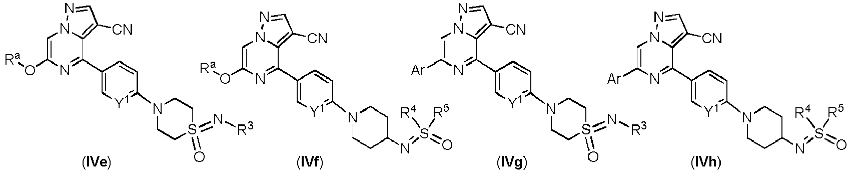 wherein,
Y¹ represents N or CH;
R^{a} is selected from hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R^{a} are optionally substituted by the same or different G¹;
Ar represents C₆₋₁₂ aryl or C₅₋₁₂ heteroaryl; one or more hydrogens in Ar are optionally substituted by the same or different G¹;
R³ represents hydrogen, deuterium, C₁₋₁₂ alkyl, C₂₋₁₂ alkenyl, C₂₋₁₂ alkynyl, C₃₋₁₂ cycloalkyl, C₃₋₁₂ heteroalicyclyl, C₆₋₁₂ aryl, C₅₋₁₂ heteroaryl, S(=O)_{w}R^{d}, S(=O)_{w}NR^{f}R^{g}, C(=O)R^{h}, C(=O)NRⁱR^{j} or C(=O)OR^{k}; one or more hydrogens in R³ are optionally substituted by the same or different G²;
R^{d}, R^{f}, R^{g}, R^{h}, Rⁱ, R^{j}, or R^{k} is each independently selected from hydrogen, deuterium, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R^{d}, R^{f}, R^{g}, R^{h}, Rⁱ, R^{j} or R^{k} are further optionally substituted by the same or different deuterium, halogen, CN, NO₂, OH, OCH₃, OCF₃, C₁₋₁₂alkyl or C₃₋₁₂cycloalkyl;
R^{f} and R^{g} are the same or different, when R^{f} and R^{g} represent two same or different C₁₋₁₂ alkyl groups connected to the same nitrogen atom, the two same or different C₁₋₁₂ alkyl groups are optionally connected with each other, and form a heteroalicycle together with the nitrogen atom, the heteroalicycle optionally comprises one or more additional heteroatoms selected from O, N or S(=O)w;
Rⁱ and R^{j} are the same or different, when Rⁱ and R^{j} represent two same or different C₁₋₁₂ alkyl groups connected to the same nitrogen atom, the two same or different C₁₋₁₂ alkyl groups are optionally connected with each other, and form a heteroalicycle together with the nitrogen atom, the heteroalicycle optionally comprises one or more additional heteroatoms selected from O, N or S(=O)w;
R⁴ and R⁵ are the same or different, and each independently selected from hydrogen, deuterium, C₁₋₁₂alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R⁴ and R⁵ each represent a same or different C₁₋₁₂ alkyl groups, the two C₁₋₁₂ alkyl groups are connected to each other, and form a heteroalicycle together with the sulfur atom to which the two C₁₋₁₂ alkyl groups are commonly connected, the heteroalicycle optionally comprises one or more additional heteroatoms selected from O, N or S(=O)_{w}; one or more hydrogens in R⁴ and R⁵ are optionally substituted by the same or different G³; and
w, G¹, G² and G³ have the same definitions as described in claim 1.

5. The heterocyclic sulfoximine compound, or a racemate, an enantiomer, a pharmaceutically acceptable salt or a solvate thereof according to claim 4, wherein,
R^{a} is selected from hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; one or more hydrogens in R^{a} are optionally substituted by the same or different deuterium, halogen, CN, NO₂, OH, CH₃, OCH₃, OCF₃, CF₃;
preferably, R^{a} is selected from hydrogen, C₁₋₁₂ alkyl, C₃₋₁₂ cycloalkyl, C₃₋₁₂ heteroalicyclyl, C₂₋₁₂ alkenyl or C₂₋₁₂ alkynyl;
more preferably, R^{a} is selected from hydrogen or C₁₋₁₂ alkyl.

6. The heterocyclic sulfoximine compound, or a racemate, an enantiomer, a pharmaceutically acceptable salt or a solvate thereof according to claim 4, wherein,
Ar represents C₆₋₁₂ aryl or C₅₋₁₂ heteroaryl; one or more hydrogens in Ar are optionally substituted by the same or different hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl;
preferably, Ar represents C₅₋₁₂ heteroaryl; one or more hydrogens in Ar are optionally substituted by the same or different hydrogen, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl;
more preferably, one or more hydrogens in Ar are optionally substituted by the same or different hydrogen or C₁₋₁₂ alkyl.

7. The heterocyclic sulfoximine compound, or a racemate, an enantiomer, a pharmaceutically acceptable salt or a solvate thereof according to claim 4, wherein,
R³ represents hydrogen, deuterium, C₁₋₁₂ alkyl, C₃₋₁₂ cycloalkyl, C₃₋₁₂ heteroalicyclyl, C₅₋₁₂ heteroaryl or -C(=O)O-C₁₋₁₂ alkyl;
one or more hydrogens in R³ are optionally substituted by the same or different deuterium, halogen, CN, NO₂, OH, OCF₃, CF₃, C₁₋₁₂alkyl-O, C₃₋₁₂ cycloalkyl-O, C₃₋₁₂ heteroalicyclyl-O, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, C₅₋₁₂ heteroaryl or -C(=O)O-C₁₋₁₂ alkyl, wherein one or more hydrogens in the C₁₋₁₂ alkyl, C₂₋₁₂ alkenyl, C₂₋₁₂ alkynyl, C₃₋₁₂ cycloalkyl, C₃₋₁₂ heteroalicyclyl, C₆₋₁₂ aryl, C₅₋₁₂ heteroaryl or -C(=O)O-C₁₋₁₂ alkyl are further optionally substituted by the same or different deuterium, halogen, CN, NO₂, OH, OCF₃, CF₃, C₁₋₁₂ alkyl, C₁₋₁₂ alkyl-O, C₃₋₁₂ cycloalkyl-O or C₃₋₁₂ heteroalicyclyl-O;
preferably, R³ represents hydrogen, deuterium, C₁₋₁₂ alkyl, C₃₋₁₂ cycloalkyl, C₃₋₁₂ heteroalicyclyl, C₅₋₁₂ heteroaryl or -C(=O)O-C₁₋₁₂ alkyl; and
one or more hydrogens in R³ are optionally substituted by the same or different deuterium, C₁₋₁₂ alkyl-O, C₃₋₁₂ cycloalkyl-O, C₃₋₁₂ heteroalicyclyl-O, C₁₋₁₂ alkyl, C₃₋₁₂ cycloalkyl, C₆₋₁₂ aryl, C₅₋₁₂ heteroaryl or -C(=O)O-C₁₋₁₂ alkyl, wherein one or more hydrogens in the C₁₋₁₂ alkyl, C₃₋₁₂ cycloalkyl, C₆₋₁₂ aryl, C₅₋₁₂ heteroaryl, or -C(=O)O-C₁₋₁₂ alkyl are further optionally substituted by the same or different deuterium, C₁₋₁₂ alkyl-O, C₃₋₁₂ cycloalkyl-O or C₃₋₁₂ heteroalicyclyl-O.

8. The heterocyclic sulfoximine compound, or a racemate, an enantiomer, a pharmaceutically acceptable salt or a solvate thereof according to claim 4, wherein,
R⁴ and R⁵ are the same or different, and each independently selected from C₁₋₁₂ alkyl or C₆₋₁₂ aryl;
one or more hydrogens in R⁴ and R⁵ are optionally substituted by the same or different deuterium, halogen, CN, NO₂, OH, OCF₃, CF₃, C₁₋₁₂ alkyl-O, C₃₋₁₂ cycloalkyl-O, C₃₋₁₂ heteroalicyclyl-O, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl, wherein one or more hydrogens in the C₁₋₁₂ alkyl, C₂₋₁₂ alkenyl, C₂₋₁₂ alkynyl, C₃₋₁₂ cycloalkyl, C₃₋₁₂ heteroalicyclyl, C₆₋₁₂ aryl, or C₅₋₁₂ heteroaryl are further optionally substituted by the same or different deuterium, halogen, CN, NO₂, OH, OCF₃, CF₃, C₁₋₁₂ alkyl, C₁₋₁₂ alkyl-O, C₃₋₁₂ cycloalkyl-O or C₃₋₁₂ heteroalicyclyl-O;
preferably, R⁴ and R⁵ are the same or different, and each independently selected from C₁₋₆ alkyl or C₆ aryl; and
one or more hydrogens in R⁴ and R⁵ are optionally substituted by the same or different deuterium, halogen, CN, NO₂, OH, OCF₃, CF₃, C₁₋₁₂ alkyl-O, C₃₋₁₂ cycloalkyl-O, C₃₋₁₂ heteroalicyclyl-O, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl, wherein one or more hydrogens in the C₁₋₁₂ alkyl, C₂₋₁₂ alkenyl, C₂₋₁₂ alkynyl, C₃₋₁₂ cycloalkyl, C₃₋₁₂ heteroalicyclyl, C₆₋₁₂ aryl, or C₅₋₁₂ heteroaryl are further optionally substituted by the same or different deuterium, halogen, CN, NO₂, OH, OCF₃, CF₃, C₁₋₁₂ alkyl, C₁₋₁₂ alkyl-O, C₃₋₁₂ cycloalkyl-O or C₃₋₁₂ heteroalicyclyl-O.

9. The heterocyclic sulfoximine compound, or a racemate, an enantiomer, a pharmaceutically acceptable salt or a solvate thereof according to any one of claims 1 to 8, wherein, the structural formula of the compound is any one of the following:

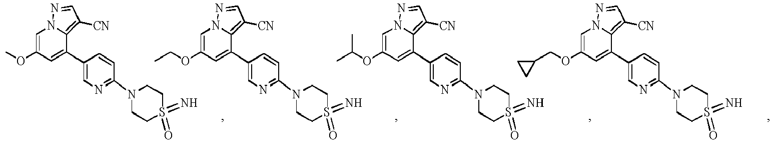

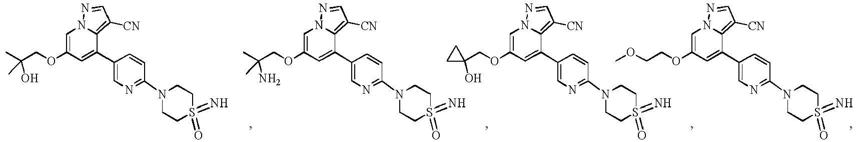

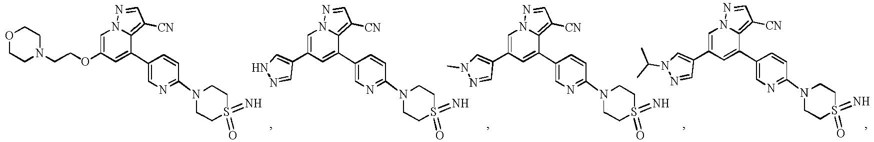

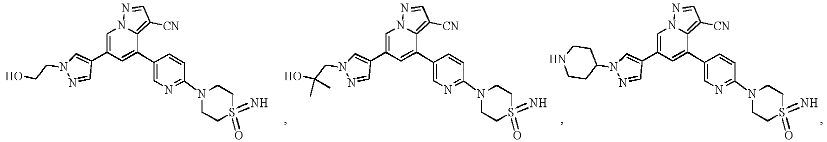

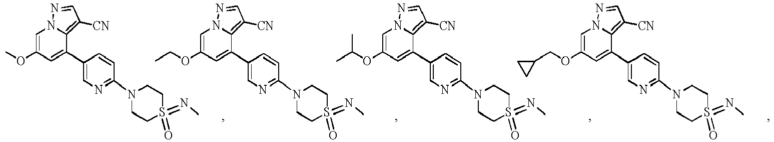

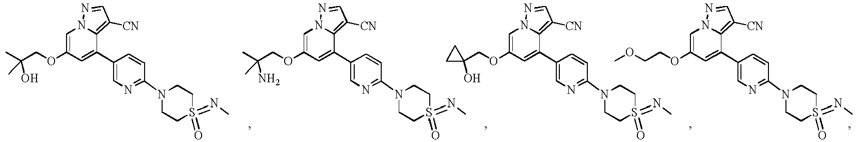

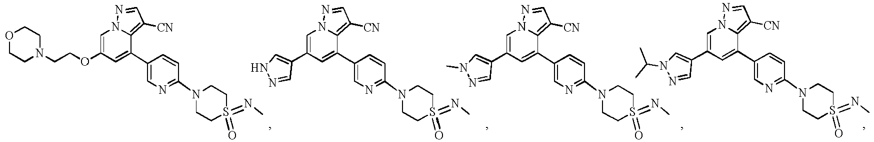

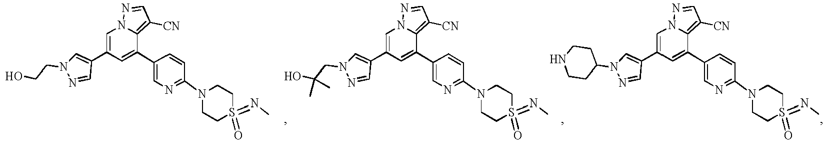

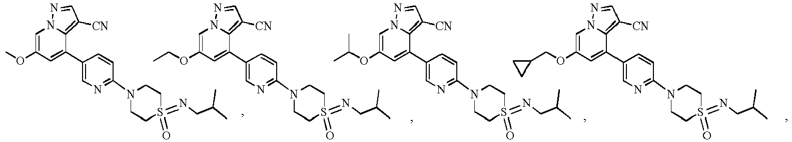

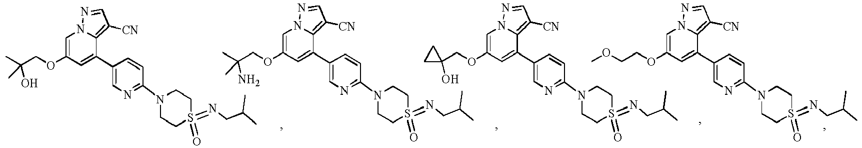

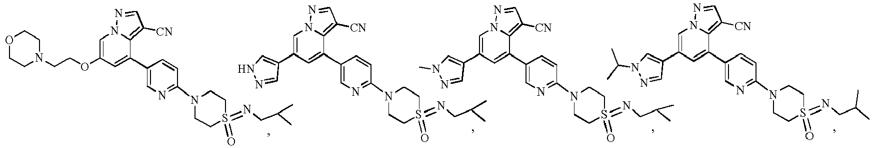

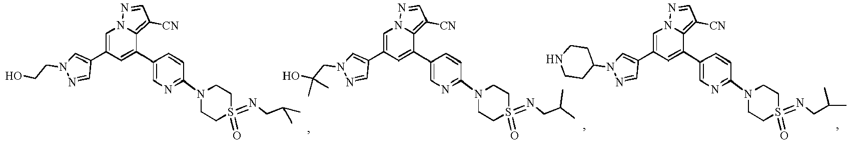

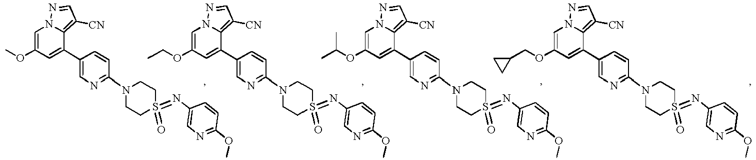

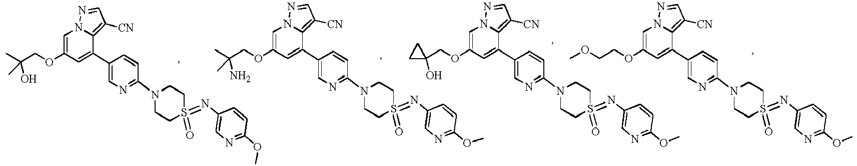

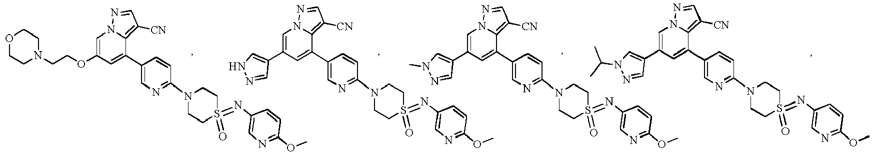

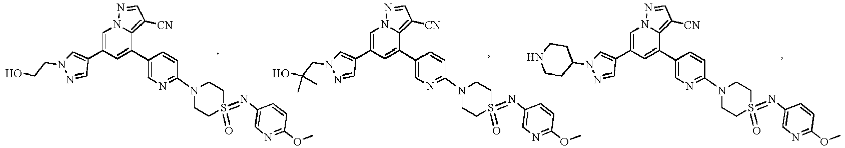

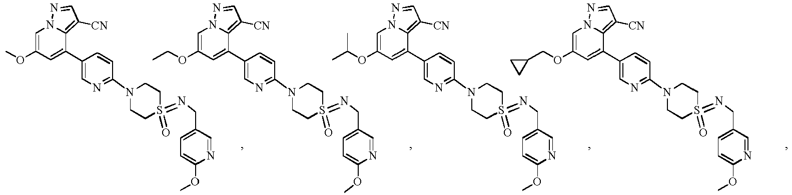

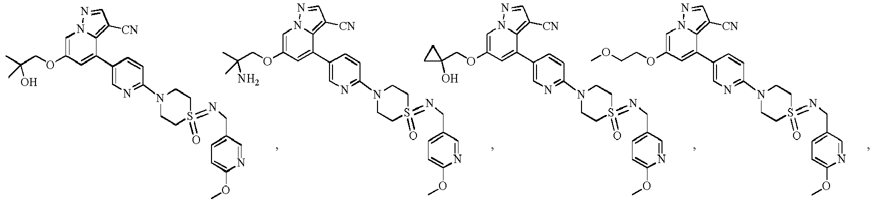

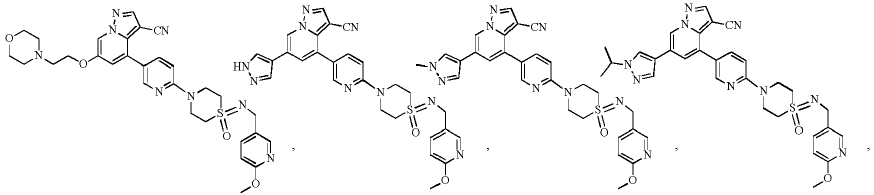

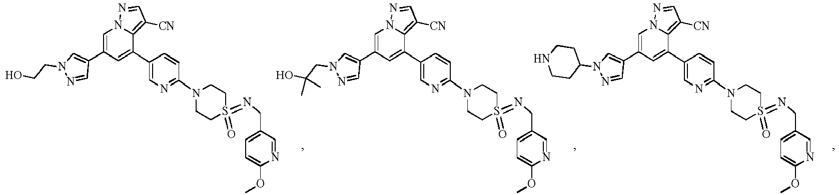

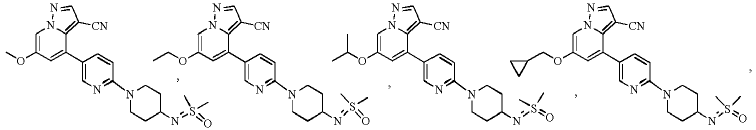

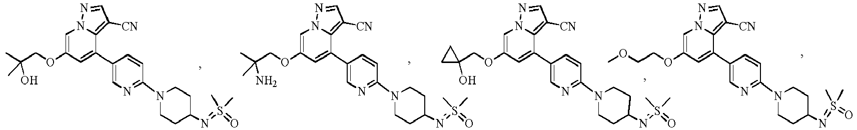

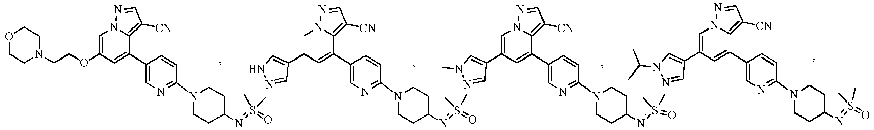

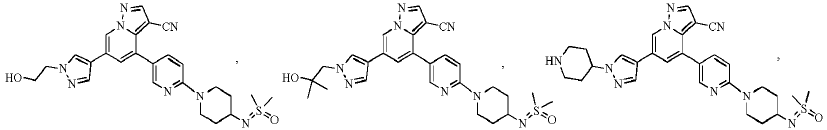

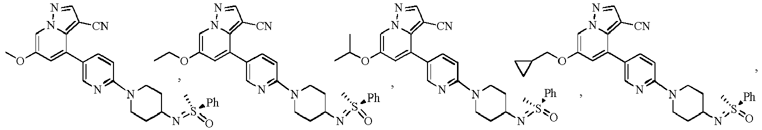

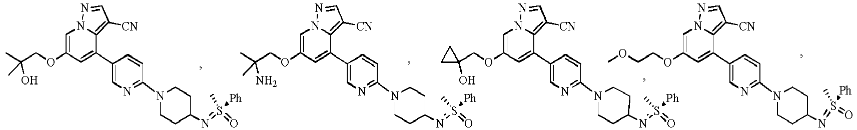

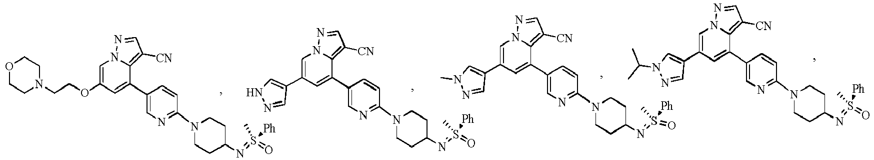

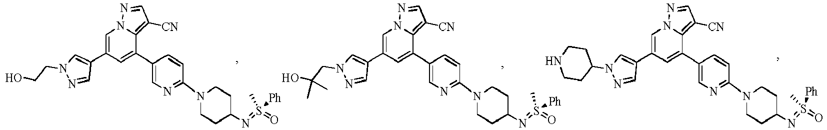

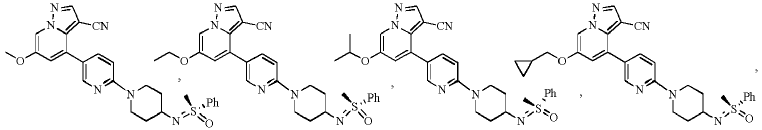

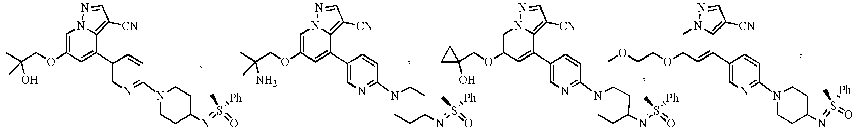

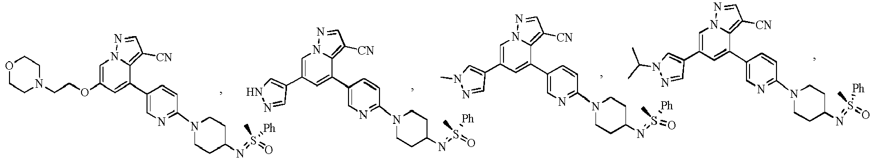

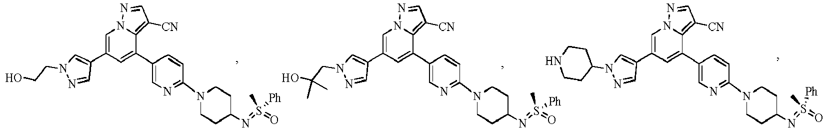

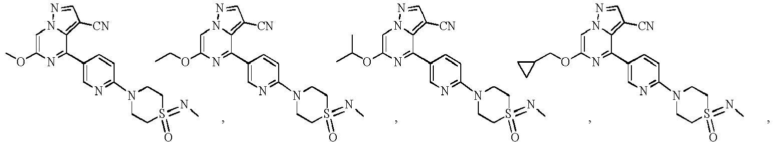

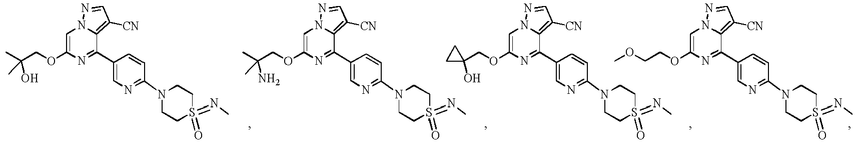

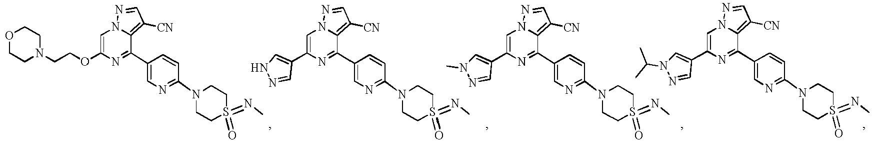

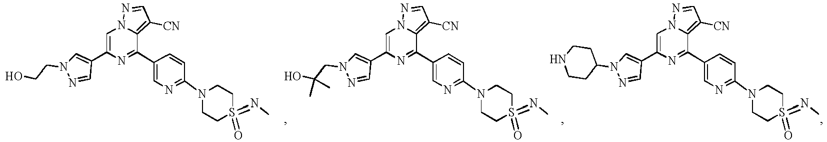

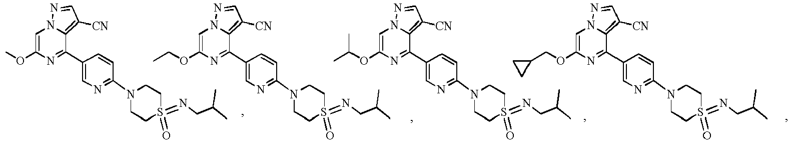

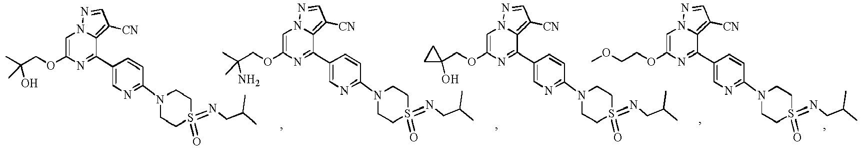

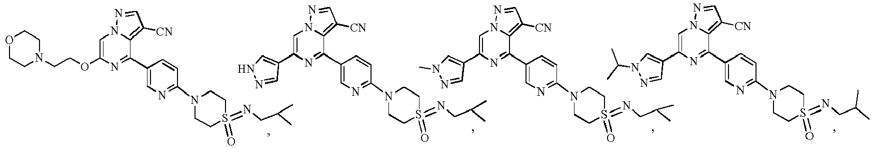

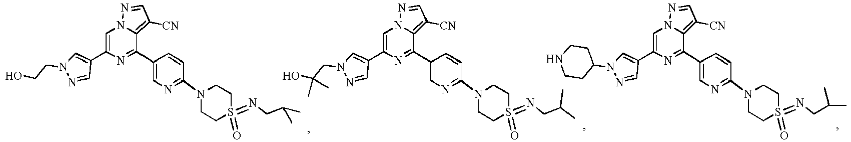

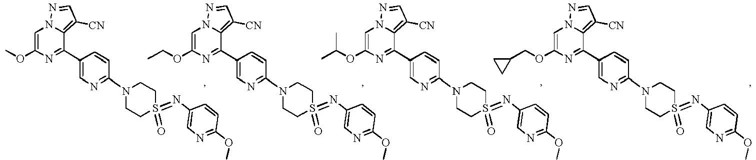

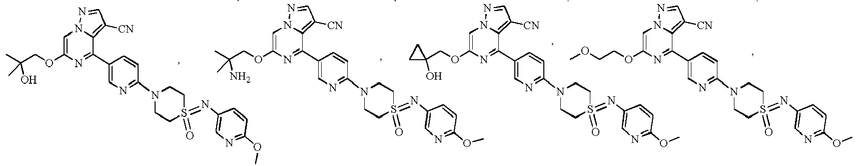

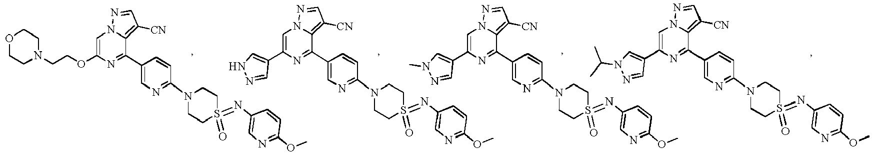

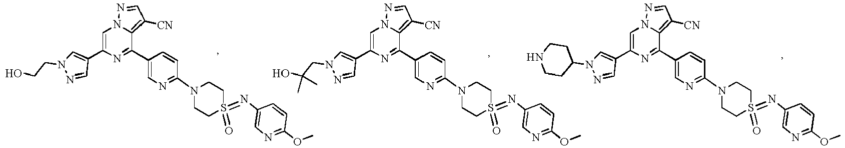

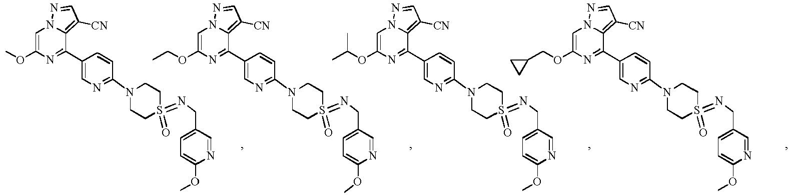

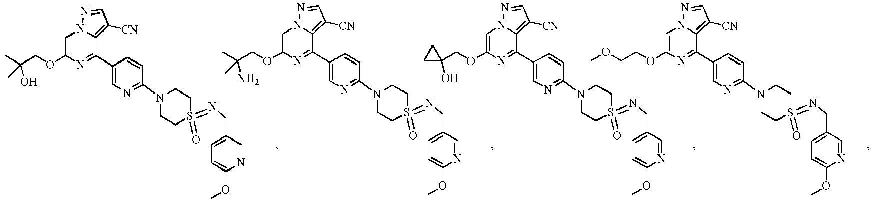

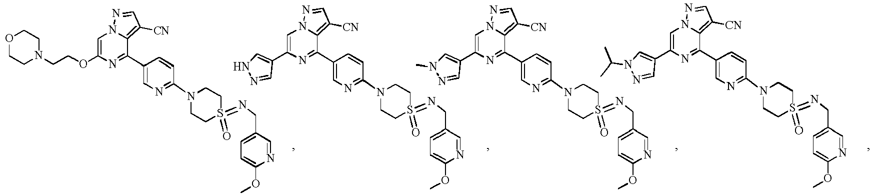

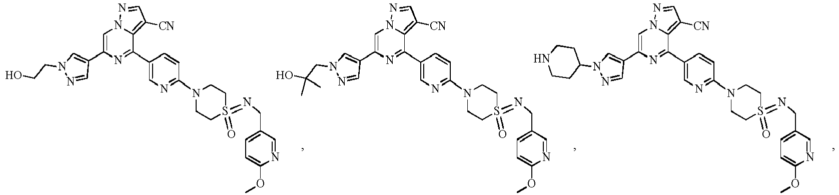

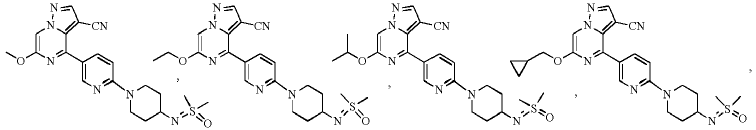

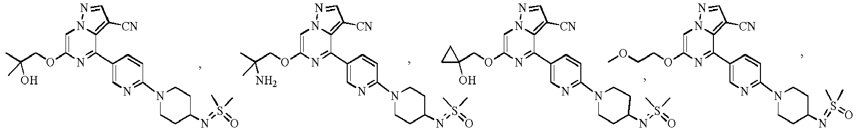

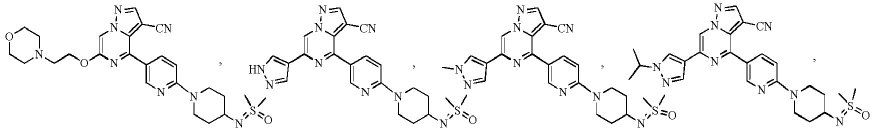

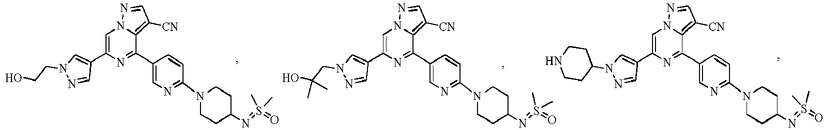

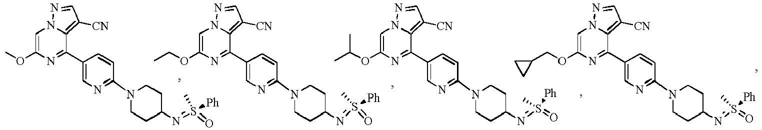

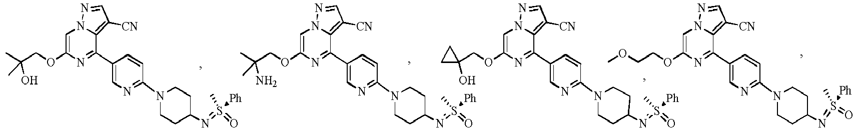

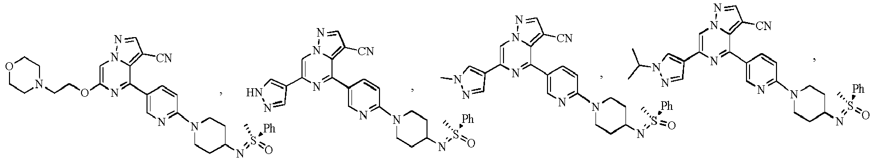

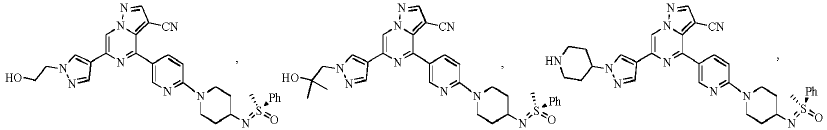

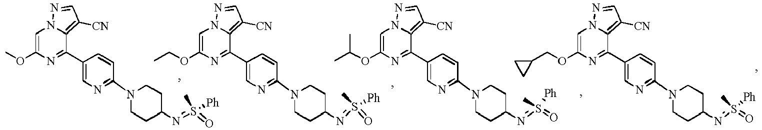

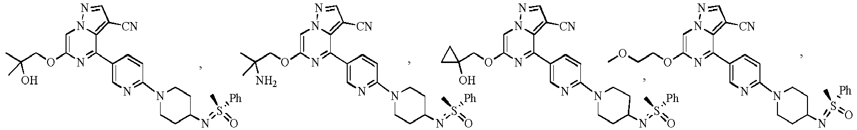

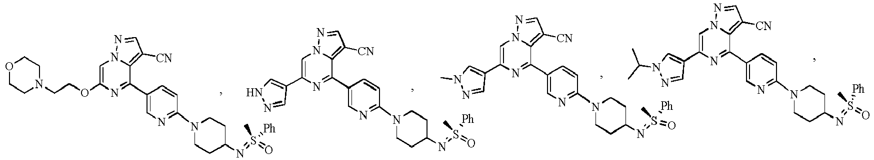

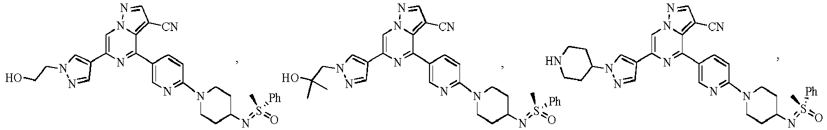

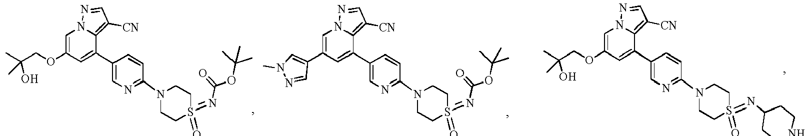

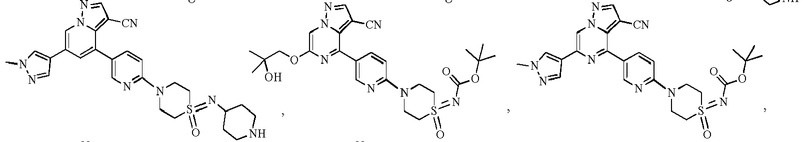

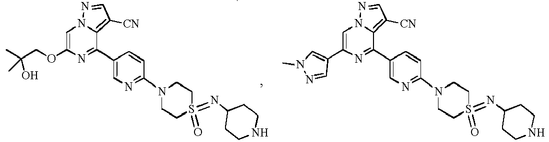

10. A pharmaceutical composition comprising at least one heterocyclic sulfoximine compound, or a racemate, an enantiomer, a pharmaceutically acceptable salt or a solvate thereof according to any one of claims 1 to 9.

11. The pharmaceutical composition according to claim 10, further comprising at least one pharmaceutically acceptable carrier or diluent.

12. The pharmaceutical composition according to claim 10 or 11, wherein, the preparation forms of the pharmaceutical composition comprise: oral preparation, injection, anal plug, nostril inhalation, eye drops or skin patch.

13. Use of the heterocyclic sulfoximine compound, or a racemate, an enantiomer, a pharmaceutically acceptable salt or a solvate thereof according to any one of claims 1 to 9 or the pharmaceutical composition according to any one of claims 10 to 12 in the treatment of diseases caused by abnormal activity of RET, RET mutant, or RET fusion proteins.

14. The use according to claim 13, wherein, the disease is tumor, the tumor is selected from solid tumor and liquid tumor, preferably, the tumor is selected from one or any combination of lung cancer, bone cancer, pancreatic cancer, skin cancer, head and neck cancer, skin or intraocular melanoma, uterine cancer, ovarian cancer, rectal cancer, colorectal cancer, anal area cancer, stomach cancer, colon cancer, breast cancer, fallopian tube cancer, endometrial cancer, cervical cancer, vaginal cancer, vulva cancer, Hodgkin's disease, esophageal cancer, small bowel cancer, endocrine system cancer, thyroid cancer, parathyroid cancer, soft tissue sarcoma, urethral cancer, penile cancer, prostate cancer, chronic or acute leukemia, bladder cancer, kidney or ureter cancer, kidney cancer, adrenal cancer, renal cell carcinoma, renal pelvis cancer, brain glioma, brain stem glioma, neuroendocrine glioma, glioma, central nervous system (CNS) neoplasms, spinal axis tumor, pituitary adenoma, gastrointestinal stromal tumor, colorectal cancer, non-small cell lung cancer, small cell lung cancer, mastocytosis, glioma, sarcoma, and lymphoma.

15. An intermediate compound for synthesizing the heterocyclic sulfoximine compound according to any one of claims 1 to 9, wherein the structural formula of the intermediate compound is (Va), (Vb), (Vc), (Vd) or (Ve): 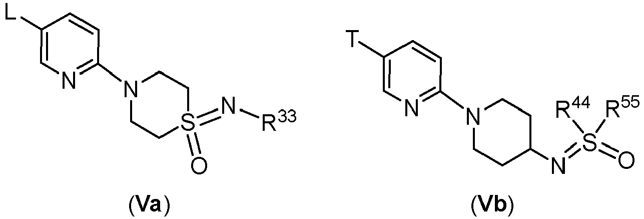 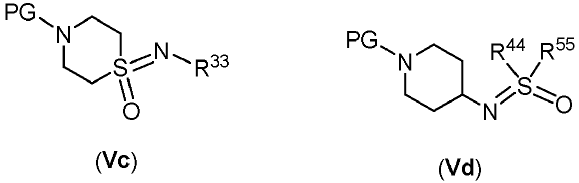 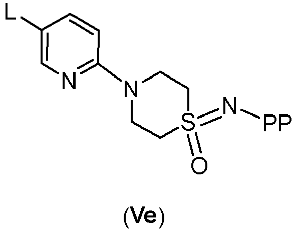 wherein:
L and T are independently selected from chlorine, bromine, iodine, F₃C-SO₃, a boronic acid group, a boronic ester group, or a boronic acid salt group;
PG is selected from hydrogen, (tert-butoxy)C(=O)-, (benzyloxy)C(=O)-, (p-methylbenzyloxy)C(=O)- or benzyl;
PP is an N protecting group;
R³³ is selected from hydrogen, (tert-butoxy)C(=O)-, (benzyloxy)C(=O)-, (p-methylbenzyloxy)C(=O)-, benzyl, trifluoroacetyl, acetyl, S(=O)_{w}R^{d}, S(=O)_{w}NR^{f}R⁹, C(=O)R^{h}, C(=O)NRⁱR^{j}, C(=O)OR^{k}, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl, wherein one or more hydrogens in the C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl are optionally substituted by the same or different G²;
R⁴⁴ and R⁵⁵ are the same or different, and each independently selected from C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R⁴⁴ and R⁵⁵ represent two same or different C₁₋₁₂ alkyl groups, the two same or different C₁₋₁₂ alkyl groups are connected to each other, and form a heteroalicycle together with the sulfur atom to which the two C₁₋₁₂ alkyl groups are commonly connected, the heteroalicycle optionally comprises one or more additional heteroatoms selected from O, N or S(=O)_{w}; one or more hydrogens in R⁴⁴ and R⁵⁵ are optionally substituted by the same or different G³;
in the structural formula (Vd), when PG is (benzyloxy)C(=O)-, R⁴⁴ and R⁵⁵ are not methyl at the same time;
G², G³, R^{d}, R^{f}, R^{g}, R^{h}, Rⁱ, R^{j}, R^{k} and w have the same definitions as described in claim 1;
preferably, the boronic acid group, boronic ester group or boronic acid salt group is selected from (HO)₂B, (CH₃O)₂B, (CH₃CH₂O)₂B, (isopropyl-O)₂B, 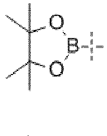 , BF₃K or BF₃Na; and
PP is selected from Boc, CBZ, trifluoroacetyl, acetyl, Bn or PMB.

16. A synthetic method for synthesizing the intermediate compound according to claim 15, comprising Steps 1-4 or Steps 1-3 and 5-7, 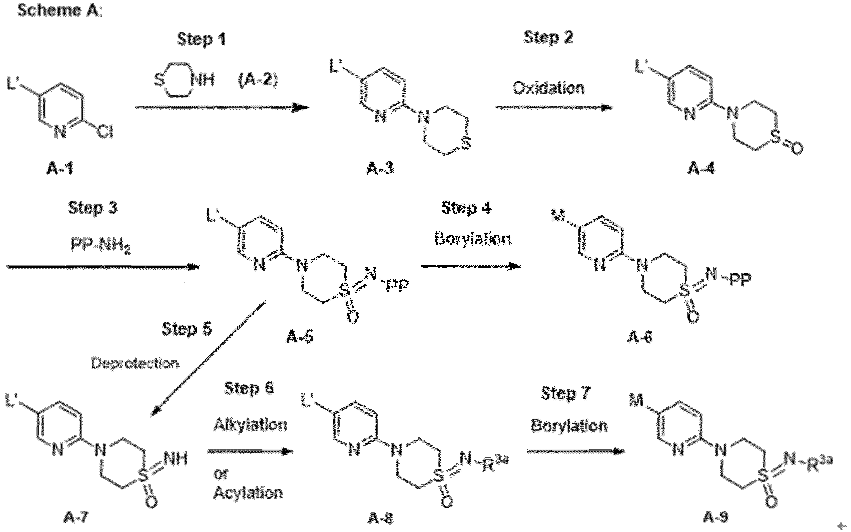 wherein:
L' is selected from chlorine, bromine, iodine, or F₃C-SO₃;
M is selected from a boronic acid group, a boronic ester group or a boronic acid salt group;
PP has the same definition as described in claim 15;
R^{3a} is selected from S(=O)_{w}R^{d}, S(=O)_{w}NR^{f}R^{g}, C(=O)R^{h}, C(=O)NRⁱRⁱ, C(=O)OR^{k}, C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl, wherein one or more hydrogens in the C₁₋₁₂ alkyl, C₃₋₁₂ cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl are optionally substituted by the same or different G²;
G², R^{d}, R^{f}, R^{g}, R^{h}, Rⁱ, R^{j}, R^{k} and w have the same definitions as described in claim 1;
Step 1: reacting a compound of formula A-1 with a compound of formula A-2 to obtain a compound of formula A-3;
Step 2: reacting the compound of formula A-3 with an oxidant to obtain a compound of formula A-4;
Step 3: reacting the compound of formula A-4 with an amino compound PP-NH₂ with a protective group under a rhodium catalyst to obtain a compound of formula A-5;
Step 4: subjecting the compound of formula A-5 to a boronation reaction to obtain an intermediate compound represented by formula A-6; or
Step 5: removing the protective group PP on the N atom in the compound of formula A-5 to obtain a compound of formula A-7;
Step 6: subjecting the compound of formula A-7 to an alkylation reaction or an acylation reaction to obtain a compound A-8;
Step 7: subjecting the compound of formula A-8 to a boronation reaction to obtain an intermediate compound represented by formula A-9; preferably,
M is selected from (HO)₂B, (CH₃O)₂B, (CH₃CH₂O)₂B, (isopropyl-O)₂B, 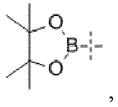 BF₃K or BF₃Na.

17. A method for preparing the heterocyclic sulfoximine compound according to any one of claims 1 to 9, comprising Step 1 or Steps 2-4, 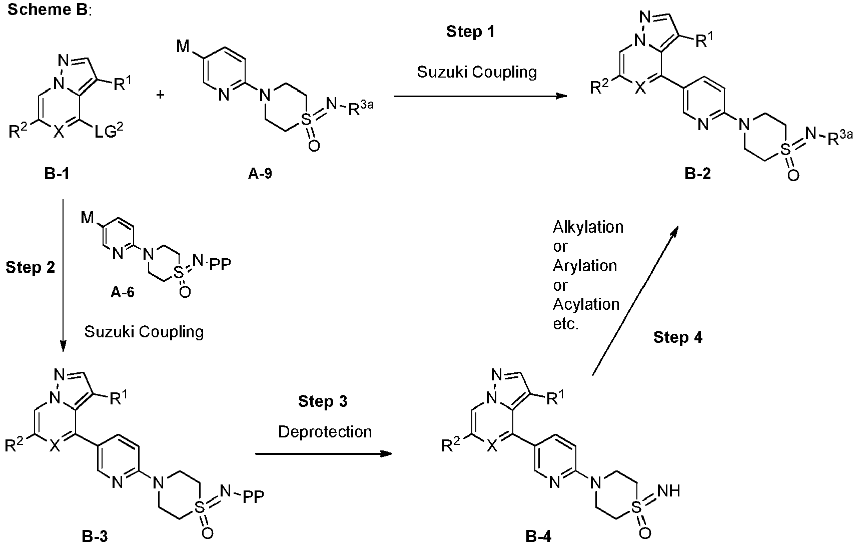 wherein:
R¹, R² and X have the same definitions as described in claim 1;
LG² represents chlorine, bromine, iodine, or F₃C-SO₃;
PP has the same definition as described in claim 15;
M and R^{3a} have the same definitions as described in claim 16;
Step 1: subjecting a compound of formula B-1 to a Suzuki reaction with a compound of formula A-9 to obtain a heterocyclic sulfoximine compound represented by formula B-2; or
Step 2: subjecting a compound of formula B-1 to a Suzuki reaction with a compound of formula A-6 to obtain a compound of formula B-3;
Step 3: removing a protecting group PP in the compound of formula B-3 to obtain a compound of formula B-4;
Step 4: subjecting the compound of formula B-4 to a reaction to obtain a heterocyclic sulfoximine compound represented by formula B-2;
preferably, in Step 4, the compound of formula B-4 is subjected to an alkylation, an acylation or an arylation reaction to introduce a R^{3a} substituent to obtain a heterocyclic sulfoximine compound represented by the formula B-2.

18. A synthetic method for synthesizing the intermediate compound according to claim 15, comprising Steps 1-2, 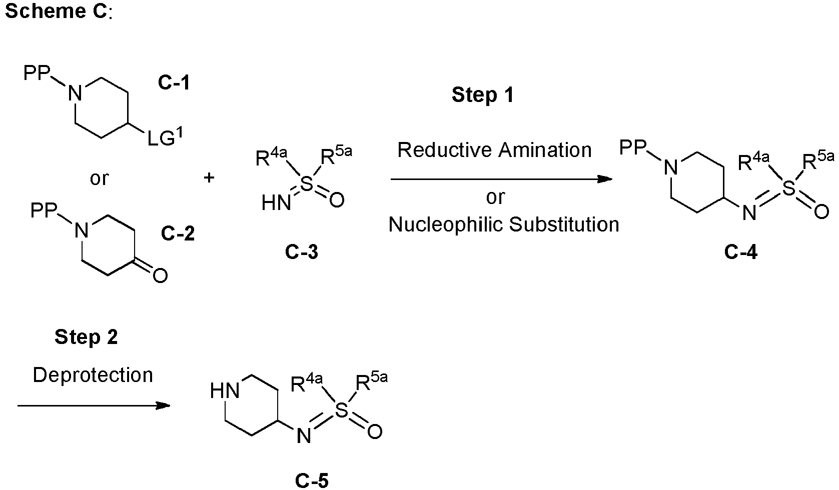 wherein:
LG¹ is selected from fluorine, chlorine, bromine, iodine, H₃C-SO₃, F₃C-SO₃, C₆H₄SO₃, p-CH₃C₆H₄-SO₃ or *p*-O₂NC₆H₄-SO₃;
R^{4a} and R^{5a} are the same or different, each independently selected from hydrogen, deuterium, C₁₋₁₂alkyl, C₃₋₁₂cycloalkyl, C₃₋₁₂ heteroalicyclyl, C₂₋₁₂ alkenyl, C₂₋₁₂ alkynyl, C₆₋₁₂ aryl, or C₅₋₁₂ heteroaryl; when R^{4a} and R^{5a} represent two same or different C₁₋₁₂ alkyl groups, the two same or different C₁₋₁₂ alkyl groups are connected to each other, and form a heteroalicycle together with the sulfur atom to which the two C₁₋₁₂ alkyl groups are commonly connected, the heteroalicycle optionally comprises one or more additional heteroatoms selected from O, N or S(=O)_{w}; one or more hydrogens in R^{4a} and R^{5a} are further optionally substituted by the same or different G³;
G³ and w have the same definitions as described in claim 1;
PP has the same definition as described in claim 15;
Step 1: subjecting a compound of formula C-1 to nucleophilic substitution with a compound of formula C-3, or subjecting a compound of formula C-2 to reductive amination with a compound of formula C-3, to obtain a compound of formula C-4;
Step 2: removing a protecting group PP in the compound of formula C-4 to obtain an intermediate compound represented by formula C-5.

19. A method for preparing the heterocyclic sulfoximine compound according to any one of claims 1 to 9, comprising Steps 1-2 or Steps 1, and 3-4, 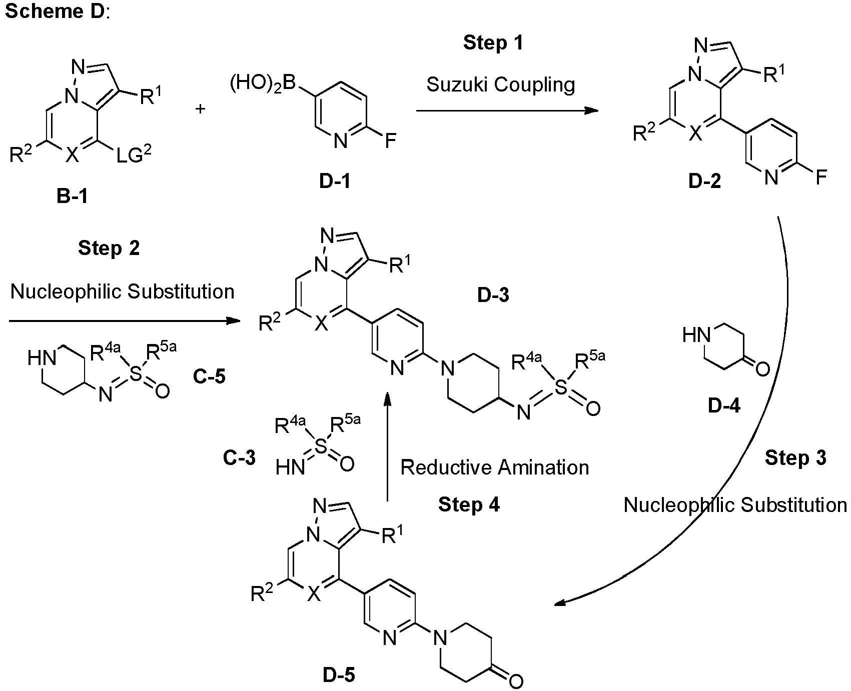 wherein:
R¹, R², X have the same definitions as described in claim 1, LG² has the same definition as described in claim 17, R^{4a} and R^{5a} have the same definitions as described in claim 18;
Step 1: subjecting a compound of formula B-1 to Suzuki reaction with a compound of formula D-1 to obtain a compound of formula D-2;
Step 2: subjecting the compound of formula D-2 and a compound of formula C-5 to a nucleophilic substitution in the presence of a base to generate a heterocyclic sulfoximine compound represented by formula D-3; or
Step 3: subjecting the compound of formula D-2 and a compound of formula D-4 to a nucleophilic substitution in the presence of a base to generate a compound of formula D-5;
Step 4: subjecting the compound of formula D-5 to reductive amination with a compound of formula C-3 to obtain a heterocyclic sulfoximine compound represented by formula D-3.
